# EUROPEAN PATENT SPECIFICATION

(11) **EP 1 558 741 B1**
(45) Date of publication and mention of the grant of the patent: **20.02.2008**
(21) Application number: 03734584.0
(22) Date of filing: 16.06.2003
(51) Int. Cl.: C12N 15/74

(54) **RECOMBINATION OF NUCLEIC ACID LIBRARY MEMBERS**
REKOMBINATION VON NUKLEINSÄUREBIBLIOTHEKSMITGLIEDERN
RECOMBINAISON D'ELEMENTS DE BIBLIOTHEQUE D'ACIDES NUCLEIQUES

(30) Priority: 14.06.2002 US 389032 P
(43) Date of publication of application: 03.08.2005
(73) Proprietor: Dyax Corporation, Cambridge, MA 02139 (US)
(72) Inventor: HUFTON, Simon, E., NL-6247 AM Gronsveld (NL)
(74) Representative: Wichmann, Hendrik
(86) International application number: PCT/US2003/018817
(87) International publication number: WO 2003/106639

(56) References cited:
- WO-A-02/00729
- WO-A-02/46400
- US-A- 5 354 847
- US-A- 5 888 732
- BODER ERIC T ET AL: "Directed evolution of antibody fragments with monovalent femtomolar antigen-binding affinity" PROCEEDINGS OF THE NATIONAL ACADEMY OF SCIENCES OF USA, NATIONAL ACADEMY OF SCIENCE, WASHINGTON, DC, US, vol. 97, no. 20, 26 September 2000 (2000-09-26), pages 10701-10705, XP002185398 ISSN: 0027-8424
- VAN DEN BEUCKEN ET AL: "Affinity maturation of Fab antibody fragments by fluorescent-activated cell sorting of yeast-displayed libraries" FEBS LETTERS, vol. 546, 2003, pages 288-294,
- SWERS ET AL: "Shuffled antibody libraries created by in vivo homologous recombination and yeast surface display" NUCLEIC ACID RESEARCH, vol. 32, no. 3, 2004, page E36,

## Description

### BACKGROUND

The invention relates to methods of varying a nucleic acid sequence by recombination. Nucleic acid recombination can be generally classified as homologous or site-specific.

Homologous recombination generates a new nucleic acid strand that incorporates sequence from parental strands such that the 5' end of one parental strand is joined to the 3' end of another parental strand. Typically, but not necessarily, homologous recombination proceeds through an intermediate, termed a Holliday junction in which a four-way junction of substrate nucleic acids is resolved to form a recombined strand. An essential feature of homologous recombination is that recombination is largely independent of the underlying sequence, provided that the substrate nucleic acids are homologous.

Site-specific recombination, in contrast, results in the exchange of nucleic acids at a specific site. The enzymes that mediate site-specific recombination, site-specific recombinases, are dedicated to recognize such sites and recombining sequences at the sites. In many natural cases, site-specific recombination results in the integration and/or excision of a sequence. For example, the bacteriophage lambda genome encodes enzymes that use site-specific recombination to specifically integrate or excise the lambda genome into and out of a bacteria. In another example, the yeast FLP-FRT system uses a recombinase to resolve replication intermediates.

Both types of recombination have been used in a variety of applications. For example, homologous recombination has been used to insert sequences into a heterologous context, e.g., as in gene targeting. Meiotic recombination has been used to generate new sequence combinations, e.g., as in the extensive breeding of strains of agricultural crops and animals. Site-specific recombination has been used in vitro for cloning nucleic acids (see, e.g., U.S. 5,888,732).

### SUMMARY

In one aspect, the invention features a method that includes: providing a first plurality of yeast cells, each cell of the first plurality expressing an antibody protein associated with a surface of the cell and each cell comprising antibody coding nucleic acid sequences that encode the antibody protein, wherein the antibody protein comprises a light chain variable immunoglobulin domain and a heavy chain variable immunoglobulin domain, and wherein each cell of the first plurality expresses a unique antibody protein; selecting, from the first plurality, a subset that comprises one or more yeast cells that interact with a target; introducing one or more Ig segment-coding nucleic acids into one or more cells of the subset, wherein each of the one or more Ig segment-coding nucleic acids comprises a sequence encoding a segment of an immunoglobulin variable domain or a complement thereof; and maintaining the cell or cells of the subset under conditions that allow the introduced Ig segment-coding nucleic acids to recombine with antibody-coding nucleic acid sequences of the cells of the subset, thereby producing one or a plurality of cells that can express an antibody protein having an altered immunoglobulin variable domain. Clones and other cells can be present in addition to the cells of the first plurality.

In one aspect, the invention features a method that includes:
providing yeast cells that each include a heterologous nucleic acid including a promoter and a coding region, wherein the coding region includes nucleic acid homologous to a immunoglobulin variable domain encoding nucleic acid and the promoter is operably linked to the coding region;
introducing one or more Ig segment-coding nucleic acids into the yeast cells, wherein each of the one or more Ig segment-coding nucleic acids includes a sequence encoding a segment of an immunoglobulin variable domain or a complement thereof; and
maintaining the yeast cells under conditions that allow the introduced Ig segment-coding nucleic acids to recombine with the coding region, thereby producing a recombined nucleic acid in which the coding region encodes a polypeptide that includes an immunoglobulin variable domain, the variable domain being encoded, at least in part, by a sequence introduced by an Ig segment-coding nucleic acid. The method can be used, e.g., to alter the sequence of an antibody protein.

In one embodiment, the method further includes expressing the recombined nucleic acid such that the polypeptide that includes the immunoglobulin variable domain is displayed on a cell surface and is accessible to a probe.

In one embodiment, the coding region is a non-functional coding region prior to recombination. In one embodiment, the coding region includes a non-immunoglobulin sequence, e.g., a stop codon, an inserted coding sequence for a non-immunoglobulin protein, e.g., a marker gene, e.g., a counter selectable marker gene. For example, in the absence of recombination the non-immunoglobulin sequence can prevent production of a polypeptide that includes an immunoglobulin variable domain. In one embodiment, the coding region includes a frameshift, inversions, or other alteration that prevents production of a polypeptide that includes an immunoglobulin variable domain.

In one embodiment, the method further includes fusing (e.g., mating) the yeast cells that include the recombined nucleic acid to other yeast cells that include a nucleic acid that encodes an immunoglobulin variable domain, compatible to the immunoglobulin variable domain encoded by the recombined nucleic acid so that the fused cells include a nucleic acid that encodes a polypeptide that includes an Ig LC and a polypeptide that includes an Ig HC.

In one embodiment, providing the yeast cells includes introducing the one or more Ig segment-coding nucleic acids into the yeast cells, which include the heterologous nucleic acid. In one embodiment, providing the yeast cells includes concurrent introducing, into the cells, the one or more Ig segment-coding nucleic acids and one or more nucleic acids homologous to regions of a immunoglobulin variable domain of the heterologous nucleic acid into the yeast cells. For example, the yeast cells can be co-transformed with the one or more Ig segment-coding nucleic acids and linearized nucleic acids that include a break in a immunoglobulin variable domain coding sequence, e.g., a CDR coding sequence.

The method can further include selecting one or more of the recombined nucleic acids for a criterion and repeating the method by providing further yeast cells, wherein the coding region of the further yeast cells is prepared from the one or more selected recombined nucleic acids.

In one embodiment, the Ig segment-coding nucleic acids each include a sequence encoding a CDR of an immunoglobulin variable domain or a complement thereof, e.g., each contains a sequence encoding a single CDR of an immunoglobulin variable domain or a complement thereof. In one embodiment, the one or more of the Ig segment-coding nucleic acids are obtained from human nucleic acids.

In one embodiment, the one or more of the Ig segment-coding nucleic acids include a plurality of nucleic acids that encode different variants of the same CDR. For example, the yeast cells are contacted with nucleic acids that include different variants of CDR1 alone.

In one embodiment, the one or more of the Ig segment-coding nucleic acids include a plurality of nucleic acids that encode different variants for a plurality of different CDRs. For example, the yeast cells are contacted with nucleic acids that include different variants of LC CDR1 and different variants of HC CDR2.

In one embodiment, the one or more of the Ig segment-coding nucleic acids include nucleic acids encoding different variants of CDR1, CDR2 and CDR3 of a immunoglobulin light or heavy chain variable domain.

In one embodiment, each Ig segment-coding nucleic acid is less than 300, 250, 200, 150, or 100 nucleotides in length.

In one embodiment, introducing includes contacting at least 10³, 10⁴, 10⁵, 10⁶, 10⁸, 10⁹, or 10¹⁰ different Ig segment-coding nucleic acids to one or more of the yeast cells. In one embodiment, the one or more of the Ig segment-coding nucleic acids are single stranded. The method can further include, prior to the introducing, inserting a counter-selectable marker into the nucleic acid members of the cells of the subset, and, after the introducing, selecting cells in which the counter-selectable marker is replaced by an Ig segment coding nucleic acid. The method can further include, prior to the introducing, inserting a mutation into the coding region, wherein the mutation prevents expression of a functional immunoglobulin variable domain.

In one embodiment, the mutation includes a stop codon, a marker gene, a frameshift, or a site of site-specific endonuclease.

In one embodiment, the polypeptide expressed from the recombined nucleic acid includes a sequence which enables an interaction with the cell such that recovery of the polypeptide results in recovery of the cell containing nucleic acid encoding the polypeptide. For example, the sequence that enables interaction with the cell encodes an anchor domain that includes a transmembrane domain or a domain that becomes GPI-linked to the yeast cell surface.

In one embodiment, the nucleic acid member can be integrated into a chromosome of the yeast cell (e.g., an endogenous chromosome or an artificial chromosome), e.g., as described herein or can be present on a plasmid.

The method can include other features described herein.

In another aspect, the invention features a method that includes: providing a plurality of yeast cells, each cell of the plurality including antibody coding nucleic acid sequences that encode a unique antibody protein, wherein the antibody protein includes a light chain variable immunoglobulin domain and a heavy chain variable immunoglobulin domain; introducing one or more Ig segment-coding nucleic acids into one or more cells from the plurality, wherein each of the one or more Ig segment-coding nucleic acids includes a sequence encoding a segment of an immunoglobulin variable domain or a complement thereof; and maintaining the cell or cells from the plurality under conditions that allow the introduced Ig segment-coding nucleic acids to recombine with antibody-coding nucleic acid sequences of the cells of the subset, thereby producing one or a plurality of cells that can express an antibody protein having an altered immunoglobulin variable domain. The method can be used, e.g., to alter the sequence of an antibody protein.

The plurality of the Ig segment-coding nucleic acids can be introduced into cells of the plurality separately (e.g., at different moments), in parallel, e.g., in separate reaction mixtures, or in the same reaction mixture.

In another aspect, the invention features a method that includes:
providing a plurality of yeast cells, the plurality including cells that each include antibody coding sequences that can encode an antibody protein that includes a light chain variable immunoglobulin domain and a heavy chain variable immunoglobulin domain or that can recombine with Ig-segment encoding nucleic acids to form functional coding sequences that encode the antibody protein;
performing one or more cycles of:
   (i) introducing nucleic acids that each include a segment encoding a CDR of an immunoglobulin variable domain or complement thereof into cells that include at least a part of the antibody coding sequences from cells of the subset;
   (ii) maintaining the cells that contact the nucleic acid in (i) under conditions that allow the introduced nucleic acids to recombine with antibody coding sequences of the cells, thereby producing modified cells that include altered antibody coding sequences that have one or more altered immunoglobulin variable domains;
   (iii) expressing the altered antibody coding sequences in the modified cells;
   (iv) contacting the modified cells to the target; and
   (iv) selecting a further subset of cells from the modified cells, the further subset including one or more yeast cells that interact with the target.

For example, at least two, three, four, or five cycles are performed. In one embodiment, the step (iii) of contacting includes contacting the cells to the target under different conditions during different cycles. In one embodiment, the step (iv) of selecting includes requiring improved binding to the target relative to a previous selecting step. For example, higher stringency washes can be used in later cycles, or increased concentration of a competitor can be used.

In one embodiment, prior to the step (i) of introducing the nucleic acid, a marker sequence is inserted into the antibody-coding sequences.

In one embodiment, the method further includes recovering an antibody coding sequence from a cell of the further subset from one or more of the cycles. In one embodiment, the method further includes sequencing at least a CDR-coding region of an antibody coding sequence in cell of the further subset from one or more of the cycles. In one embodiment, the nucleic acids are introduced into cells of the subset.

In one embodiment, the method further includes r, in one or more of the cycles, sporulating cells of the subset prior to (i), and mating cells into which nucleic acids have been introduced after (ii).

In one embodiment, the providing of a plurality of yeast cells includes amplifying an original cell such that yeast cells of the plurality are substantially identical. In one embodiment, the providing of a plurality of yeast cells includes amplifying a plurality of original cells wherein the original cells include antibody coding sequences for different antibodies that interact with the same target. For example, the method can be used to affinity mature a single antibody, or a plurality of antibodies, the antibody or antibodies being from any source.

In one embodiment, the providing of a plurality of yeast cells includes selecting one or more nucleic acids from a phage display library and reformatting one or more nucleic acids from a phage display system into a yeast expression system.

The method can be used to select a cell that displays an antibody protein. The method can include other features described herein.

In another aspect, the invention features a method of varying subunits of an antibody protein displayed on a yeast cell. The method includes:
providing a first haploid yeast cell that includes a first nucleic acid member encoding a first subunit of an antibody protein, the first subunit including a immunoglobulin variable domain;
introducing one or a plurality of nucleic acids that each include a segment encoding a CDR of the immunoglobulin variable domain or a complement thereof into the first haploid cell or clones thereof such that the introduced nucleic acid recombines with the first nucleic acid member in the first haploid cell or clones thereof, thereby producing one or more modified first haploid cells; and
mating the one or more modified first haploid cells to one or more second haploid cells to provide one or more diploid cells, wherein a second haploid yeast cell includes a second nucleic acid member encoding a second subunit of the antibody protein, the second subunit including a immunoglobulin variable domain complementary to the immunoglobulin variable domain of the first subunit, and each diploid cell can express, on its cell surface, an antibody protein including the first and second subunit.

In one embodiment, the method further includes, prior to the introducing, inserting a non-immunoglobulin sequence into the first nucleic acid member, thereby disrupting the coding of the first subunit.

In one embodiment, the non-immunoglobulin coding sequence includes a counter-selectable marker. The method can include other features described herein.

In another aspect, the method includes:
providing a first haploid yeast cell that includes a first nucleic acid member that can encode a polypeptide including a first subunit of an antibody protein, the first subunit including a immunoglobulin variable domain, wherein the region of the nucleic acid member that encodes the immunoglobulin variable domain is disrupted;
introducing one or a plurality of nucleic acids that each include a segment encoding a CDR of the immunoglobulin variable domain or a complement thereof into the first haploid cell or clones thereof such that the introduced nucleic acid recombines with the first nucleic acid member in the first haploid cell or clones thereof, thereby producing one or more modified, first haploid cells; and
mating the one or more modified, first haploid cells to one or more second haploid cells to provide one or more diploid cells, wherein a second haploid yeast cell includes a second nucleic acid member encoding a polypeptide including a second subunit of the antibody protein, the second subunit including a immunoglobulin variable domain complementary to the immunoglobulin variable domain of the first subunit, and each diploid cell can express, on its cell surface, an antibody protein including the first and second subunit. The method can further include, e.g., prior to the mating, amplifying one or more of the modified, first haploid cells by one or more rounds of cell division. The method can include other features described herein.

In another aspect, the invention features a method that includes:providing a first haploid yeast cell that includes a first nucleic acid member encoding a polypeptide including a first subunit of an antibody protein, the first subunit including a immunoglobulin light chain variable domain and a second haploid yeast cell that includes a second nucleic acid member encoding a polypeptide including a second subunit of the antibody protein, the second subunit including a immunoglobulin heavy chain variable domain; introducing one or a plurality of nucleic acids that each include a segment encoding a CDR of an immunoglobulin variable light chain domain or a complement thereof into the first haploid cell or clones thereof such that the introduced nucleic acid recombines with the first nucleic acid member in the first haploid cell or clones thereof, thereby producing one or more modified first haploid cells; introducing one or a plurality of nucleic acids that each include a segment encoding a CDR of an immunoglobulin variable heavy chain domain or a complement thereof into the second haploid cell or clones thereof such that the introduced nucleic acid recombines with the second nucleic acid member in the second haploid cell or clones thereof, thereby producing one or more modified second haploid cells; and mating the one or more modified first haploid cells to the one or more modified second haploid cells to provide one or more diploid cells that each can express, on a cell surface, an antibody protein with a varied immunoglobulin light chain variable domain and a varied immunoglobulin heavy chain variable domain.

In one embodiment, the steps of providing the first and second haploid cell includes:
providing a diploid yeast cell that includes (i) a first nucleic acid member encoding a first subunit of an antibody protein, the first subunit including a immunoglobulin light chain variable domain and (ii) a second nucleic acid member, encoding a second subunit of the antibody protein, the second subunit including a immunoglobulin heavy chain variable domain; and sporulating the diploid yeast cell to provide the first haploid cell that contains the first nucleic acid member, but not the second nucleic acid member and the second haploid cell that contains the second nucleic acid member, but not the first nucleic acid member.

In one embodiment, the method further includes, prior to the mating, amplifying the one or more modified first or second haploid cells by one or more rounds of cell division.

In one embodiment, the method further includes, prior to the mating, amplifying the one or more modified first and second haploid cells by one or more rounds of cell division.

In one embodiment, the method further includes sporulating one or more of the diploid cells formed by the mating.

In one embodiment, the method further includes selecting a subset of the one or more of the diploid cells by contacting the one or more diploid cells, or clones thereof, to a target. For example, the subset includes one or more of the cells that interact with the target or one or more of the cells that do not interact with the target. Cells of the subset can be sporulated.

The method can include other features described herein.

In another aspect, the invention features a method that includes:
providing a plurality of diverse nucleic acids that include a protein coding sequence or a complement thereof;
providing a plurality of eukaryotic cells, each cell of the plurality including a heterologous nucleic acid that includes acceptor sequences that can recombine with homologous sequences present in one or more of the diverse nucleic acids, wherein recombination of the acceptor sequences and a diverse nucleic acid produces a recombined nucleic acid that encodes a polypeptide that includes a segment encoded by the diverse nucleic acid or complement thereof;
introducing nucleic acids from the plurality of diverse nucleic acids into cells of the plurality of cells;
recombining one or more of the introduced nucleic acids with the heterologous nucleic acid in each cell, thereby producing recombined nucleic acids that each encodes a polypeptide that includes a segment that is encoded by one or more of the introduced nucleic acids or a complement thereof;
expressing the recombined nucleic acids in the cells such that the polypeptides encoded by the recombined nucleic acids are associated with a surface of the cells;
contacting the cells that express the recombined nucleic acids to a target; and
selecting one or more cells as a function of their interaction with the target. The method can be used to select a cell that displays a binding protein.

In one embodiment, the heterologous nucleic acid includes a nonfunctional immunoglobulin domain coding sequence that provides the acceptor sequences for recombination. For example, the nonfunctional immunoglobulin domain coding sequence includes a mutation (e.g., a stop codon or frame shift) prior to the 3' end of the immunoglobulin domain coding sequence. In one embodiment, the mutation is in a region encoding a CDR.

In one embodiment, the nonfunctional immunoglobulin domain coding sequence includes a marker gene in a region encoding a CDR and sequences encoding FR regions of the immunoglobulin domain are intact. For example, the marker gene includes a counter-selectable marker.

In one embodiment, the recombined nucleic acids encoded an antibody light chain, and the expressing further includes expressing a nucleic acid encoding an antibody heavy chain that includes a VH domain and CH1 domain and an anchor domain such that the antibody heavy and light chain associate and the anchor domain anchors the antibody heavy chain to the cell surface.

In one embodiment, the heterologous nucleic acid includes a sequence which enables an interaction with the cell such that recovery of the polypeptide results in recovery of the cell containing nucleic acid encoding the polypeptide. For example, the sequence encodes an anchor domain, e.g., a transmembrane domain or a domain that becomes GPI-linked to the yeast cell surface.

In one embodiment, the eukaryotic cells are yeast cells, e.g., S. *cerevisiae* cells.

In one embodiment, the target is a protein. In another embodiment, the target is a cell, e.g., a mammalian cell, e.g., a cancer cell or a differentiated cell.

In one embodiment, the heterologous nucleic acid does not encode a functional protein prior to the recombining. In one embodiment, prior to the recombining, a counter-selectable marker is located between the acceptor sequences of the heterologous nucleic acid, and the recombining removes the counter-selectable marker.

In one embodiment, the recombining is enhanced by cleaving the heterologous nucleic acid in each cell. For example, the cleaving creates a double-stranded break in the heterologous nucleic acid. In one embodiment, prior to the recombining, a site recognized by a site-specific endonuclease that can be expressed in yeast cells is located between the acceptor sequences of the heterologous nucleic acid, and the recombining is enhanced by providing the site-specific endonuclease in each of the cells. For example, the site-specific endonuclease is HO endonuclease and the providing includes transcribing a gene encoding the HO endonuclease. Typically the endonuclease is chosen so that lethality does not result if the endonuclease is expressed.

In one embodiment, the recombined nucleic acids include a sequence encoding an immunoglobulin variable domain.

The method can include other features described herein.

In another aspect, the invention features a method of altering the sequence of an antibody protein. The method includes: providing a first plurality of yeast cells (e.g., a yeast cell display library or replicates of an original cell), each cell of the first plurality expressing an antibody protein associated with a surface of the cell and each cell including antibody coding nucleic acid sequences that encode the antibody protein, wherein the antibody protein includes a light chain variable immunoglobulin domain and a heavy chain variable immunoglobulin domain, and wherein each cell of the first plurality can express a unique antibody protein; optionally selecting, from the first plurality, a subset that includes one or more yeast cells that interact with a target; introducing one or more Ig segment-coding nucleic acids into one or more cells of the subset or of the first plurality (e.g., where replicates of an original cell are used), wherein each of the one or more Ig segment-coding nucleic acids includes a sequence encoding a segment of an immunoglobulin variable domain or a complement thereof; and maintaining the cell or cells of the subset or of the first plurality (e.g., where replicates of an original cell are used) under conditions that allow the introduced Ig segment-coding nucleic acids to recombine with antibody-coding nucleic acid sequences of the cells of the subset, thereby producing one or a plurality of cells that can express an antibody protein having an altered immunoglobulin variable domain. Clones and other cells can be present in addition to the cells of the first plurality.

In one embodiment, the Ig segment-coding nucleic acids each include a sequence encoding a CDR of an immunoglobulin variable domain or a complement thereof. For example, they can include a single CDR, e.g., the do not span a complete FR domain, e.g., the include a single CDR and parts of flanking FR regions. In another embodiment, the Ig segment-coding nucleic acids each include a sequence encoding a FR of an immunoglobulin variable domain or a complement thereof.

In one embodiment, the one or more of the Ig segment-coding nucleic acids are obtained from human nucleic acids.

The one or more of the Ig segment-coding nucleic acids can include a plurality of nucleic acids that encode different variants of the same CDR (e.g., different variants of CDR1) or different variants for a plurality of different CDRs (e.g., CDR1, CDR2 and CDR3 of a immunoglobulin light or heavy chain variable domain).

In one embodiment, each Ig segment-coding nucleic acid is less than 300, 250, 200, or 150 nucleotides in length. In one embodiment, the introducing includes contacting at least 10³, 10⁵, or 10⁷ different Ig segment-coding nucleic acids to the one or more cells of the subset. The one or more of the Ig segment-coding nucleic acids can be single stranded or double stranded. The plurality of the Ig segment-coding nucleic acids can be introduced into cells of the subset in parallel or in the same reaction mixture.

The method can further include inserting a non-immunoglobulin coding sequence, e.g., a counter-selectable marker into the nucleic acid members of the cells of the subset and, after the introducing, selecting cells in which the counter-selectable marker is replaced by an Ig segment coding nucleic acid.

The method can further include contacting cells from the pool of cells with the target and selecting a subset of cells that interact with the target.

In one embodiment, at least a part of the antibody protein (e.g., an immunoglobulin heavy chain that includes a VH and CH1 domain) is fused to an anchor domain, e.g., a transmembrane domain or GPI-linked polypeptide on the yeast cell surface. An immunoglobulin heavy chain can further include, e.g., a CH2 and CH3 domain. An immunoglobulin light chain can be associated with the yeast cell surface by covalent or non-covalent interaction with the immunoglobulin heavy chain. In another embodiment, the immunoglobulin light chain is fused to an anchor domain, e.g., a transmembrane domain or GPI-linked polypeptide on the yeast cell surface.

The nucleic acid member can be integrated into a chromosome of the yeast cell (e.g., an endogenous chromosome or an artificial chromosome) or can be present on a plasmid. For example, the LC and HC coding sequences are integrated into different yeast chromosomes (e.g., different homologous chromosomes or different non-homologous chromosomes).

For example, the antibody-coding nucleic acid sequences includes a LC coding sequence that encodes a polypeptide that includes a LC variable immunoglobulin domain and a HC-coding sequence that encodes a polypeptide that includes a HC variable immunoglobulin domain.

In one embodiment, wherein the yeast cell is a diploid cell, at least at the time of the selecting, and the LC coding sequence and the HC-coding sequence are integrated into loci on homologous chromosomes such that the LC coding sequence and the HC-coding sequence segregate into different spores when the diploid cell is sporulated. For example, the sequences can be integrated at a position that is linked to the MAT locus, e.g., within 20 kb of the MAT locus, e.g., within the MAT locus, e.g., such that at least the MATα gene that encodes MATα2 protein is not disrupted. it can be desirably e.g., after sporulation to perform in vivo recombination to modify the same CDR, another CDR, other sets of CDRs, and so forth. E.g., one can walk along a CDR by using oligonucleotides that mutagenize different sets of amino acids within a CDR, e.g., different sets of four amino acids within a CDR.

In one embodiment, the antibody protein is a Fab. The method can include other features described herein.

In another aspect, the invention features a method that includes: providing a first plurality of yeast cells, the first plurality including cells that each include a different antibody protein associated with the cell surface and antibody coding sequences that encode the antibody protein, wherein the antibody protein includes a light chain variable immunoglobulin domain and a heavy chain variable immunoglobulin domain; selecting a subset of cells from the first plurality, wherein the subset includes one or more yeast cells that interact with a target; performing one or more cycles of:
(i) introducing nucleic acids that each include a segment encoding a CDR of an immunoglobulin variable domain or complement thereof into cells that include at least a part of the antibody coding sequences from cells of the subset;
(ii) maintaining the cells that contact the nucleic acid in (i) under conditions that allow the introduced nucleic acids to recombine with antibody coding sequences of the cells, thereby producing modified cells that include altered antibody coding sequences that have one or more altered immunoglobulin variable domains;
(iii) expressing the altered antibody coding sequences in the modified cells;
(iv) contacting the modified cells to the target; and
(iv) selecting a further subset of cells from the modified cells, the further subset including one or more yeast cells that interact with the target. The method can be used to select a cell that displays an antibody protein. In one embodiment, at least two cycles are performed. In one embodiment, the step (iii) of contacting includes contacting the cells to the target under different conditions during different cycles. In one embodiment, the step (iv) of selecting includes requiring improved binding to the target relative to a previous selecting step. In one embodiment, prior to the step (i) of introducing the nucleic acid, a sequence is inserted into the antibody-coding sequences. The insert sequence can include one or more of: a stop codon, a marker sequence (e.g., a counter-selectable marker), or site for cleavage by a site specific endonuclease (e.g., an endonuclease which can be expressed without lethal consequences to a yeast cell, e.g., an endonuclease which does not irreparably cleave an endogenous gene in the yeast cell).

The method can further include one or more of: recovering an antibody coding sequence from a cell of the further subset from one or more of the cycles, sequencing at least a CDR-coding region of an antibody coding sequence in cell of the further subset from one or more of the cycles.

In one embodiment, the nucleic acids are introduced into cells of the subset.

The method can further, in one or more of the cycles, sporulating cells of the subset prior to (i), and mating cells into which nucleic acids have been introduced after (ii). The method can include other features described herein.

In another aspect, the invention features a method that includes: providing a first plurality of yeast cells, each cell of the first plurality expressing an antibody protein associated with a surface of the cell and each cell comprising nucleic acid sequences that encodes the antibody protein, wherein the antibody protein comprises a light chain variable immunoglobulin domain and a heavy chain variable immunoglobulin domain, and wherein each cell of the first plurality expresses a unique antibody protein; providing one or a plurality of immunoglobulin-coding nucleic acids that comprises a sequence encoding a segment (e.g., a CDR or FR) of an immunoglobulin variable domain or a complement thereof; selecting, from the first plurality, a subset that comprises one or more yeast cells that interact with a target; introducing one or more of the immunoglobulin-coding nucleic acids into one or more cells of the subset; and maintaining the cell or cells of the subset under conditions that allow the introduced diverse nucleic acids to recombine with antibody-coding nucleic acid sequences of the cells of the subset, thereby producing one or a plurality of cells that can express an antibody protein having an altered immunoglobulin variable domain. Clones and other cells can be present in addition to the cells of the first plurality. The method can be used to alter the sequence of an antibody protein. The method can include other features described herein.

In another aspect, the invention features a method of identifying a modified polypeptide. The method includes: (a) providing (1) a plurality of coding nucleic acids that each includes a segment encoding a polypeptide domain that becomes attached to a surface of a yeast cell, wherein the polypeptide domain is heterologous to the yeast cell, and (2) a plurality of diverse nucleic acids, (b) for each of the coding nucleic acids of the plurality, recombining one of the diverse nucleic acids and the coding nucleic acid in a yeast cell to form a modified nucleic acid that encodes a modified polypeptide domain, (c) expressing the modified nucleic acids in a yeast cell, wherein the modified polypeptide domains are attached to a surface of the yeast cell and each modified nucleic acid includes an immunoglobulin variable domain; (d) contacting the yeast cells to a target (e.g., a target compound or a target cell); and (e) recovering one or more cells that bind to the target thereby identifying one or more polypeptides encoded by the modified nucleic acids of the recovered cells. The method can further include modifying one or more of the modified nucleic acids from the recovered cells, e.g., by repeating the method.

In one embodiment, each diverse nucleic acid includes a segment that encodes a CDR or a complement thereof.

The method can further include reformatting the modified nucleic acids of the recovered cells for mammalian cell expression and expressing the reformatted nucleic acids in a mammalian cell. The method can also include evaluating a biological property of the polypeptides (e.g., a polypeptide that includes an immunoglobulin domain) expressed by the mammalian cell, e.g., a property that includes recruitment of a cytotoxic cell or a complement protein.

In one embodiment, the recombining can further include inserting a cassette (e.g., including a counter-selectable marker (e.g., *URA3* or kanR)) into a plasmid to form the acceptor nucleic acid, the cassette including a marker and then substituting the cassette with the donor nucleic acid. In one embodiment, inserting the cassette deletes a target-binding sequence.

In one embodiment, each modified polypeptide is covalently attached, e.g., fused to a cell-surface anchor domain, e.g., a domain that is membrane associated such as a transmembrane domain or a GPI-anchored domain. In one embodiment, the anchor domain includes: yeast Aga1p, Aga2p, or fragments thereof.

In one embodiment, the diverse donor nucleic acids include at least 10³, 10⁴, 10⁵, 10⁶, 10⁸, 10⁹, or 10¹⁰ different donor nucleic acids. For example, the diverse donor nucleic acids can include between 10³- 10¹², 10³- 10¹⁰ or 10⁴- 10⁹ different donor nucleic acids. In one embodiment, each diverse donor nucleic acid is less than 2000, 500, 200, 120, 80, 50, or 40 nucleotides in length. The diverse donor nucleic acids can be at least about 20, 40, 100, 500, 1000, or 2000 nucleotides in length. Each diverse donor nucleic acid can be at least 40%, 50%, 60%, 70%, 80%, 90%, 95%, or 98% identical to at least another diverse donor nucleic acid. For example, a diverse donor nucleic acid can have 1, 2, 3, or at least 4 mismatches with respect to another diverse donor nucleic acid.

In an embodiment, each of the diverse donor nucleic acids is of equal length as the others or is within 30, 20, 15, or 10% of the average length of the diverse donor nucleic acids. In one embodiment, the diverse donor nucleic acids of the plurality all have a length within 8, 6, 4, 3, 2, or 1 nucleotide of each other. Each of the diverse donor nucleic acids can include 3' and/or 5' terminal regions of at least 6 basepairs in length that are identical (or at least 70% identical) to corresponding terminal regions of each of the other diverse donor nucleic acids. In a preferred embodiment, the terminal regions are exactly complementary to a corresponding site on the template nucleic acid. In an embodiment, each of the diverse donor nucleic acids includes a sequence corresponding to (e.g., partially complementary to) a common region of the template (e.g., of at least 5 or 10 nucleotides). Each diverse donor nucleic acid can include a naturally occurring sequence or a synthetic sequence.

In an embodiment, each diverse donor nucleic acid encodes a CDR or fragment thereof, e.g., a fragment including at least 5 amino acids. In an embodiment, the diverse donor nucleic acids further include 3' and/or 5' terminal regions that anneal to a sequence that flanks a sequence encoding a CDR (or its complement), e.g., a sequence that encodes a framework region (or its complement), e.g., at least one, two, three, four, or five nucleotides thereof. The terminal regions are preferably less varied than the sequence between the terminal regions among the diverse donor nucleic acids. The CDR can be a heavy chain CDR (e.g., heavy chain CDR1, CDR2, and CDR3) or a light chain CDR (e.g., light chain CDR1, CDR2, and CDR3). In a preferred embodiment the diverse donor nucleic acids preferably do not include the entire sequence of the framework regions which flank the CDR, e.g., contain less than 2, 5, 8, 10, or 15 of the amino acids of each of the flanking framework regions.

In another aspect, the invention features a method that includes: (a) providing (1) an acceptor nucleic acid that includes first recombination sites and acceptor sequences, and (2) a donor nucleic acid that includes second recombination sites and a segment encoding a subject polypeptide or a complement thereof, (b) recombining the first and second recombination sites of the respective acceptor and donor nucleic acids in a cell (e.g., a yeast cell) to form a recombined nucleic acid that encodes the subject polypeptide and includes the acceptor sequences, and (c) expressing the recombined nucleic acid in a cell such that the subject polypeptide is detectable on a surface of the cell (e.g., the subject polypeptide is attached, covalently or non-covalently, to the surface of the cell). In on embodiment, the subject polypeptide includes an immunoglobulin domain. In one embodiment, the donor nucleic acid includes a sequence that encodes a CDR.

In another aspect, the invention features a method of expressing a modified polypeptide. The method includes: (a) providing (1) a coding nucleic acid that includes a segment encoding a heterologous polypeptide domain that can be detected on a cell surface, and (2) a modifying nucleic acid, (b) recombining the modifying nucleic acid and the coding nucleic acid in a cell to form a modified nucleic acid that encodes a modified polypeptide domain, and (c) expressing the modified nucleic acid in a cell such that the modified polypeptide domain is detectable on a surface of the cell. The polypeptide can be directly or indirectly attached to the cell surface. For example, the polypeptide can be fused to a transmembrane protein, attached by a covalent bond (e.g., a disulfide bond to a transmembrane protein), or attached by a non-covalent interaction with a transmembrane protein. The polypeptide can be similarly attached to another membrane (e.g., a non-transmembrane protein) or cell-associated protein.

The cell can be a microorganism, e.g., a yeast cell, e.g., *S. cerevisiae* or *S. pombe.*

The method can further include evaluating the modified nucleic acid for a property, e.g., binding to a target, e.g., a target compound or target cell. The evaluating can include contacting the cell to a target, and evaluating an interaction between the cell and the target, e.g., between the modified polypeptide domain and the target. The evaluating can further include assessing a quantitative measure of binding affinity.

In one embodiment, the cell is a yeast cell and the method further includes reformatting the modified nucleic acid for mammalian cell expression and expressing the reformatted nucleic acid in a mammalian cell. The method can also include evaluating a biological property of the modified polypeptide (e.g., a polypeptide that includes an immunoglobulin domain) expressed by the mammalian cell, e.g., a property that includes recruitment of a cytotoxic cell or a complement protein.

In another aspect, the invention features a method of expressing a modified polypeptide. The method includes: (a) providing (1) a plurality of coding nucleic acids that each includes a segment encoding a polypeptide domain that can be detected on a surface of a cell, wherein the polypeptide domain is heterologous to the cell, and (2) a plurality of diverse nucleic acids, (b) for each of the coding nucleic acids of the plurality, recombining one of the diverse nucleic acids and the coding nucleic acid in a cell to form a modified nucleic acid that encodes a modified polypeptide domain, and (c) expressing the modified nucleic acids in cells such that the modified polypeptide domains are detectable on a surface of the cell.

The cell can be a microorganism, e.g., a yeast cell, e.g., *S. cerevisiae* or *S. pombe.*

The method can further include evaluating the modified nucleic acids for a property, e.g., binding to a target compound. The evaluating can include contacting the cells to a target compound, and recovering cells that bind to the target compound, e.g., cells that display on their surface a modified polypeptide domain that binds to the target compound. The evaluating can further include assessing a quantitative measure of binding affinity.

The method can further include mating the cell after the recombining, e.g., to another cell in which a second donor nucleic acid and a second acceptor nucleic acid are recombined. If the cells are allowed to divide prior to the mating, numerous combinations of recombination products can be produced.

In one embodiment, each modified polypeptide is covalently attached, e.g., fused to a cell-surface anchor domain, e.g., a domain that is membrane associated such as a transmembrane domain or a GPI-anchored domain. In one embodiment, the anchor domain includes: yeast Aga1p, Aga2p, or fragments thereof.

In one embodiment, the cell is a yeast cell and the method further includes reformatting the modified nucleic acids for mammalian cell expression and expressing the reformatted nucleic acids in a mammalian cell. The method can also include evaluating a biological property of the polypeptides (e.g., a polypeptide that includes an immunoglobulin domain) expressed by the mammalian cell, e.g., a property that includes recruitment of a cytotoxic cell or a complement protein.

In one embodiment, the diverse donor nucleic acids include at least 10³, 10⁴, 10⁵, 10⁶, 10⁸, 10⁹, or 10¹⁰ different donor nucleic acids. In one embodiment, each diverse donor nucleic acid is less than 2000, 500, 200, 120, 80, 50, or 40 nucleotides in length. The diverse donor nucleic acids can be at least about 20, 40, 100, 500, 1000, or 2000 nucleotides in length. Each diverse donor nucleic acid can be at least 40%, 50%, 60%, 70%, 80%, 90%, 95%, or 98% identical to at least another diverse donor nucleic acid. For example, a diverse donor nucleic acid can have 1, 2, 3, or at least 4 mismatches with respect to another diverse donor nucleic acid.

In an embodiment, each of the diverse donor nucleic acids is of equal length as the others or are within 30, 20, 15, or 10% of the average length of the diverse donor nucleic acids. In one embodiment, the diverse donor nucleic acids of the plurality all have a length within 8, 6, 4, 3, 2, or 1 nucleotide of each other. Each of the diverse donor nucleic acids can include 3' and/or 5' terminal regions of at least 6 basepairs in length that are identical (or at least 70% identical) to corresponding terminal regions of each of the other diverse donor nucleic acids. In a preferred embodiment, the terminal regions are exactly complementary to a corresponding site on the template nucleic acid. In an embodiment, each of the diverse donor nucleic acids includes a sequence corresponding to (e.g., partially complementary to) a common region of the template (e.g., of at least 5 or 10 nucleotides). Each diverse donor nucleic acid can include a naturally occurring sequence or a synthetic sequence.

In an embodiment, each diverse donor nucleic acid encodes a CDR or fragment thereof, e.g., a fragment including at least 5 amino acids. In an embodiment, the diverse donor nucleic acids further include 3' and/or 5' terminal regions that anneal to a sequence that flanks a sequence encoding a CDR (or its complement), e.g., a sequence that encodes a framework region (or its complement), e.g., at least one, two, three, four, or five nucleotides thereof. The terminal regions are preferably less varied than the sequence between the terminal regions among the diverse donor nucleic acids. The CDR can be a heavy chain CDR (e.g., heavy chain CDR1, CDR2, and CDR3) or a light chain CDR (e.g., light chain CDR1, CDR2, and CDR3). In a preferred embodiment the diverse donor nucleic acids preferably do not include the entire sequence of the framework regions which flank the CDR, e.g., contain less than 2, 5, 8, 10, or 15 of the amino acids of each of the flanking framework regions.

The method can include other features described herein.

In another aspect, the invention features a method that includes: (a) providing (1) an acceptor nucleic acid that includes first recombination sites and acceptor sequences, and (2) a donor nucleic acid that includes second recombination sites and a segment encoding a subject polypeptide or a complement thereof, (b) recombining the first and second recombination sites of the respective acceptor and donor nucleic acids in a cell to form a recombined nucleic acid that encodes the subject polypeptide and includes the acceptor sequences, and(c) expressing the recombined nucleic acid in a cell such that the subject polypeptide is detectable on a surface of the cell.

The cell can be a microorganism, e.g., a eukaryotic microorganism such as a yeast cell, e.g., *S. cerevisiae* or *S. pombe,* or a prokaryotic microorganism, e.g., a bacterial cell, e.g., *E. coli.*

In one embodiment, the method further includes, prior to the recombining, introducing the donor nucleic acid into the cell, and/or introducing the acceptor nucleic acid into the cell. For example, donor nucleic acid is single stranded, and/or the acceptor nucleic acid is single stranded. In one embodiment, the acceptor nucleic acid is circular.

In one embodiment, the method further includes generating a break (e.g., a nick or a double-stranded break) in the acceptor nucleic acid. The break can be generated in vitro or in the cell. For example, the break is generated by induced expression of a cleavage enzyme (e.g., HO endonuclease). In another example, the break is generated by a cleavage-directing oligonucleotide.

The method can further include inserting a cassette into a plasmid to form the acceptor nucleic acid, the cassette including a marker. The recombining may substitute the cassette with the donor nucleic acid. In one embodiment, inserting the cassette deletes a target-binding sequence. In one embodiment, the acceptor nucleic acid includes a counter-selectable marker (e.g., URA3 or kanR) between the first recombination sites.

In one embodiment, the donor nucleic acid is single-stranded, e.g., an oligonucleotide or a single-stranded plasmid, or double-stranded, e.g., a double-stranded plasmid. The donor nucleic acid can include a sequence encoding a CDR or segment thereof. The donor nucleic acid can be a member of a pool of diverse nucleic acids. The recombined nucleic acid can encode a polypeptide that includes an immunoglobulin domain. In another embodiment, the recombined nucleic acid can encode an enzyme or fragment thereof. For example, the donor nucleic acid can encode an active site residue, e.g., a residue that is within 2 Angstroms of a bound substrate or cofactor.

In one embodiment, the first and/or second recombination sites include a site specific recombination site, e.g., a site that includes a yeast FLP recognition site or a lambda attB site. The method can further include inducing a site specific recombinase.

The method can include fusing a first cell that includes the donor nucleic acid and a second cell that includes the acceptor nucleic acid. The fusing can be yeast mating. The method can further include inducing a site specific recombinase or generating a break (e.g., a double-stranded break) in the donor or acceptor nucleic acid, e.g., using HO endonuclease.

In one embodiment, the acceptor sequences include a sequence encoding a first subunit of a multimeric protein and the subject sequence encodes at least a fragment of a second subunit of the multimeric protein. For example, the multimeric protein is an antibody or a T-cell receptor. In one embodiment, the cassette deletes a sequence encoding a CDR or a portion thereof.

The acceptor and/or donor nucleic acid can be bound by (e.g., coated with) a nucleic acid binding protein, e.g., a single-stranded binding protein, e.g., recA.

In one embodiment, the acceptor and donor nucleic acid are combined in vitro prior to the recombining. The mixture formed by the combining can be introduced into the cell.

The providing can include selecting a member of a display library for a given property, e.g., a cell display library, e.g., a yeast display library. The providing can further include isolating the acceptor nucleic acid from the selected member of a display library, and/or introducing the donor nucleic acid into the selected member. The providing of the acceptor nucleic acid can include PCR amplifying the acceptor nucleic acid, and/or isolating a tagged or untagged nucleic acid strand, or oligonucleotide-mediated cleavage.

The method can further include one or more of: contacting the cell to a ligand, evaluating binding of the cell to the ligand or isolating the cell based on its ability to bind the ligand; and mating the cell after the recombining, e.g., to another cell in which a second donor nucleic acid and a second acceptor nucleic acid are recombined.

In one embodiment, the subject polypeptide is fused to a cell-surface anchor domain, e.g., a domain that is membrane associated such as a transmembrane domain or a GPI-anchored domain. In one embodiment, the anchor domain includes: yeast Aga1p, Aga2p, or fragments thereof.

The method can further include ligating the first and second nucleic acid in vitro and introducing the ligated nucleic acid into a cell, wherein recombination in the cell circularizes the nucleic acid.

In one embodiment, the donor nucleic acid includes a first non-functional allele of a marker gene and the acceptor nucleic acid includes a second non-functional allele of the marker gene, wherein the first and second alleles can recombine to generate a functional allele of the marker gene.

The method can include other features described herein.

In another aspect, the invention features a method that includes: (a) providing a first plurality of yeast cells, the first plurality including cells that each include a different nucleic acid member and a heteroligomeric protein detectable on a cell surface, the nucleic acid member encoding the polypeptide; (b) contacting the first plurality of yeast cells to a first target; (c) selecting a subset that includes one or more yeast cells of the first plurality, the one or more yeast cells containing a nucleic acid member which expresses a heteroligomeric protein that binds the first target; and (d) recombining nucleic acid members of the cells of the subset with a diverse pool of donor nucleic acids. At least one reaction phase of the recombining occurs in the yeast cells, and yields a second plurality of yeast cells. The second plurality includes cells that each include a variant of a corresponding nucleic acid member of the cells of the subset. The donor nucleic acids encode for a segment of a functional domain and include regions of homology to regions of a sequence encoding the heteroligomeric protein of one of the selected cells.

The method can further include (e) contacting cells from the second plurality of yeast cells with a second target, and (f) selecting one or more yeast cells that contain a second library member that encodes a polypeptide that binds the second target. The second target can be the same as the first target.

The method can further include, prior to the recombining, inserting a marker gene into a region of each nucleic acid member of the cells of the subset, the region encoding a segment of the expressed polypeptide. In one embodiment, the inserting deletes a sequence that encodes a binding determinant.

Each nucleic acid member of the cells of the subset can encode a polypeptide that includes an immunoglobulin variable domain. In such cases, the binding determinant can include a CDR or a fragment thereof. For example, the first plurality may include at least 10³ 10⁴, 10⁵, 10⁶, 10⁷, or 10⁸ different cells. The subset can include at least 10, 10³, 10³, 10⁵, 10⁶, 10⁷ different cells. The second plurality can include at least 10, 10³, 10³, 10⁵, 10⁶, 10⁷ different cells.

The method can further include automatically processing the members of the selected subset for the recombining, e.g. robotically picking members of the selected subset and combining the members in a common container for the recombining or each into an individual container for the recombining. The method can further include automatically evaluating a members of the second plurality of yeast cells for a property conferred by expression of the corresponding variant nucleic acid. For example, the method can include electronically recording information obtained from the automatic evaluating.

The method can include other features described herein.

In another aspect, the method includes a method of varying the sequence of a polypeptide. The method includes: (a) providing a first plurality of yeast cells, the first plurality including cells that each include a different nucleic acid member that includes a segment encoding at least a portion of a polypeptide and a first marker gene; (b) providing a second plurality of yeast cells, the second plurality including cells that each include a different nucleic acid member that includes a segment encoding at least a portion of a polypeptide and, optionally, a second marker gene, wherein nucleic acid members of the first and second plurality include regions of homology such that a member of the first plurality can recombine with a member of the second plurality; (c) forming mated cells, each mated cell being the product of one parental cell that is a cell of the first plurality and an other parental cell that is a cell of the second plurality and each mated cell including the nucleic acid members from the parental cells; and (d) selecting mated cells in which the nucleic acid members from parental cells have recombined to form a recombined nucleic acid that excludes the first marker gene (and optionally the second marker gene) and includes nucleic acid sequence from the nucleic acid members from the parental cells.

In one embodiment, the recombined nucleic acid encodes a immunoglobulin variable domain. In addition, the parental nucleic acids can each encode portions of an immunoglobulin variable domain. The method can include other features described herein.

In another aspect, the invention features a method that includes: providing a host bacterial cell that includes a restriction activity and a host nucleic acid encoding first and second subunits of a heteroligomeric receptor; infecting the host bacterial cell with a phage particle that includes an unmethylated nucleic acid, cleavable by the restriction activity and encoding different first and second subunits of a heteroligomeric receptor, wherein the unmethylated nucleic acid is cleaved after infection; recombining the cleaved nucleic acid and the host nucleic acid at a site in a region encoding the first or second subunits of the heteroligomeric receptor; and isolating a phage display particle that displays a heteroligomer receptor encoded by the recombined nucleic acids. The unmethylated nucleic acid can further include a first non-functional allele of a marker gene, and the host nucleic acid includes a second non-functional allele of the marker gene, wherein the first and second non-functional alleles can recombine to generate a functional allele.
The method can further include selecting for an activity dependent on a functional allele of the marker gene. The method can include other features described herein.

In another aspect, the invention features a method that includes: providing a diploid yeast cell that includes (i) a first nucleic acid member encoding a first subunit of a heteroligomeric protein, the first subunit comprising a immunoglobulin light chain variable domain and (ii) a second nucleic acid member, encoding a second subunit of the heteroligomeric protein, the second subunit comprising a immunoglobulin heavy chain variable domain, and (iii) the heteroligomeric protein detectable on a surface of the cell; sporulating the diploid yeast cell to provide a first haploid cell comprising the first nucleic acid member, but not the second nucleic acid member and a second haploid cell comprising the second nucleic acid member, but not the first nucleic acid member; introducing one or a plurality of nucleic acids that each comprise a segment encoding a CDR of an immunoglobulin variable light chain domain or a complement thereof into the first haploid cell or clones thereof such that the introduced nucleic acid recombines with the first nucleic acid member in the first haploid cell or clones thereof, thereby producing one or more modified first haploid cells; introducing one or a plurality of nucleic acids that each comprise a segment encoding a CDR of an immunoglobulin variable heavy chain domain or a complement thereof into the second haploid cell or clones thereof such that the introduced nucleic acid recombines with the second nucleic acid member in the second haploid cell or clones thereof, thereby producing one or more modified second haploid cells; and fusing (e.g., mating) one or more modified first haploid cells to one or more modified second haploid cells to provide cells (e.g., diploid) that each can express, on a cell surface, a heteroligomeric protein with a varied immunoglobulin light chain variable domain and a varied immunoglobulin heavy chain variable domain. The method can include other features described herein. Further, any polyploid cell that can sporulate to produce spores that can be fused to another spore can be used. For example, tetraploid cells that produce diploid cells of opposite mating types can be used. In addition, mating type loci deletions can be used or generated to facilitate mating.

The methods described herein can be used in a continuous screening method, e.g., for yeast cell display. For example, a library of yeast cells is screened for cells displaying a protein that binds to the target. Nucleic acids encoding the displayed protein are modified by a method described herein, (e.g., recombination including introduction of diverse nucleic acids by transformation, mating, introduction of a counter-selectable marker, combinations thereof and so forth) to form another display library. This cycle can be repeated until a protein is identified that binds to the target, e.g., with at least predetermined affinity and/or specificity. The method can used to generate protein variants without purifying and/or cloning nucleic acid from each selected member of the display library.

In another aspect, the invention also features a yeast cell display library that includes members whose nucleic acids are modified by a method described herein, a yeast cell display member modified and isolated by a method described herein, and a immunoglobulin protein that includes an immunoglobulin modified and isolated by a method described herein.

In another aspect, the invention features an isolated nucleic acid that comprises a 5' recombination sequence, a marker, and a 3' recombination sequence. Each recombination sequence can be at least 10, 20, 30, or 50 nucleotides in length, e.g., between 10-50, or 20-50, or 30-100 nucleotides in length. Each recombination sequence can be homologous or identical to a different immunoglobulin framework region. For example, the 5' recombination sequence can be homologous to FR1 and the 3' recombination sequence can be homologous to FR2 and vice versa. The marker can be a selectable marker, a dominant marker, or a counter selectable marker (e.g., URA3). Typically, the marker is a marker that enables cells that have lost the marker to be selectively identified, e.g.,. by growth or other phenotype. The marker can be functional in a yeast cell; e.g., he marker can be a yeast gene.

In another aspect, the invention features a yeast cell that contains a heterologous nucleic acid that comprises a coding region for encoding an immunoglobulin variable domain, wherein the region includes sequences that encode one or more portions of an immunoglobulin variable domain and a non-immunoglobulin coding sequence, such that the coding region does not encode a functional immunoglobulin variable domain and such that recombination with one or more other nucleic acid fragments from a immunoglobulin variable domain coding sequence can restore the coding region so that it encodes a functional immunoglobulin variable domain. In one embodiment, the non-immunoglobulin coding sequence comprises a genetic marker, e.g., a selectable marker or a detectable marker. In one embodiment, the selectable marker is a counter-selectable marker. In one embodiment, the yeast cell further includes a second coding region for encoding a second immunoglobulin variable domain (e.g., the other chain of an antibody). The second coding region can also be disrupted, e.g., with a non-immunoglobulin coding sequence, e.g., a different genetic marker. At least one of the coding regions can be configured so that an antibody formed by expression of functional versions of the coding regions is expressed on the surface of the yeast cell, e.g., attached to the plasma membrane, e.g., by a transmembrane domain or a GPI anchor. The yeast cell can be a yeast cell treated with a nucleic acid described herein, e.g., above and can include other features described herein.

A "heterologous" sequence refers to a sequence which is introduced into a cell or into the context of a nucleic acid by artifice. A heterologous sequence may be a copy of an endogenous gene, but, for example, inserted into an exogenous plasmid or into a chromosomal site at a position other than its endogenous position.

The term "fusion" refers to a single polypeptide chain that includes the components that are fused. An exemplary fusion protein includes an immunoglobulin domain and a transmembrane domain or GPI-linked domain. The fused components need not be directly adjacent to each other. For example, one or more other sequences (e.g., a linker polypeptide or a functional domain) can be located between the fused elements.

The term "coding region" refers to a nucleotide sequence that encodes a polypeptide or that includes one or more mutations, which may prevent encoding of the polypeptide, but which can be cured, e.g., by recombination, particularly homologous recombination. Exemplary mutations include inclusion of a non-coding sequence, a separate coding sequence (e.g., a marker gene), a stop codon, a frame shift mutation, and so forth.

An "immunoglobulin domain" refers to a domain from the variable or constant domain of immunoglobulin molecules. Immunoglobulin domains typically contain two β-sheets formed of about seven β-strands, and a conserved disulphide bond (see, e.g., A. F. Williams and A. N. Barclay 1988 Ann. Rev Immunol. 6:381-405). Variable domains can typically be described as a light chain variable domain (VL) or a heavy chain variable domain (VH). The VH and VL regions can be further subdivided into regions of hypervariability, termed "complementarity determining regions" ("CDR"), interspersed with regions that are more conserved, termed "framework regions" (FR). The extent of the framework region and CDRs has been precisely defined (see, Kabat, E.A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). For the purposes of a definition herein, the Kabat reference is used to delineate CDRs. Each VH and VL is composed of three CDRs and four FRs, arranged from amino-terminus to carboxy-terminus in the following order: FR1, CDR1, FR2, CDR2, FR3, CDR3, FR4. Variable domains can include one or more human or humanized framework regions and/or one or more human complementarity determining regions (CDR).

The term "antibody" or "antibody protein" refers to a protein that includes at least a paired VH and VL domain or sufficiently large VH and VL fragments that the VH and VL fragments pair to form an antigen binding site. Accordingly, the term "antibody" encompasses, and is not limited to full-length antibodies (e.g., an IgG (e.g., an IgG1, IgG2, IgG3, IgG4), IgM, IgA (e.g., IgA1, IgA2), IgD, and IgE, but preferably an IgG), antigen-binding fragments that lack one or more constant immunoglobulin domains (*e.g.*, a Fab, F(ab')₂ or a single chain antibody (e.g., a scFv fragment)), and other structures that include at least a paired VH and VL domain. The antibody can include two heavy chain immunoglobulin polypeptides and two light chain immunoglobulin polypeptides; a single heavy chain immunoglobulin polypeptide and a single light chain immunoglobulin polypeptide; or can be a single chain antibody. The antibodies can, optionally, include a constant region chosen from a kappa, lambda, alpha, gamma, delta, epsilon or a mu constant region gene. An exemplary antibody includes a heavy and light chain constant region substantially from a human antibody, e.g., a human IgG1 constant region or a portion thereof.

The antibody is preferably monospecific, e.g., a monoclonal antibody, or antigen-binding fragment thereof. The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition.

In one embodiment, the antibody is a recombinant antibody. The term "recombinant" antibody, as used herein, includes all antibodies that are prepared, expressed, created or isolated by recombinant means, such as antibodies expressed using a recombinant expression vector transfected into a host cell, antibodies isolated from a recombinant, combinatorial antibody library, antibodies isolated from an animal (*e.g.*, a mouse, goat, or cow) that is transgenic for human immunoglobulin genes or antibodies prepared, expressed, created or isolated by any other means that involves splicing of human immunoglobulin gene sequences to other DNA sequences.

The term "displayed antibody" refers to an antibody that is present on a genetic package such that the displayed antibody is physically associated with the genetic package and the genetic package includes a nucleic acid that encodes at least the variable domains of the displayed antibody. Examples of a genetic package include cells (e.g., yeast cells) and phage particles.

An "antigen binding site" refers to the antigen binding region of a paired VH and VL. A particular antigen binding region may or may not have a known ligand that is bound by the antigen binding site.

The term "recombination" refers to the process of exchange of genetic information between nucleic acid strands. In one example, the process results in the formation of a new nucleic acid strand that includes a region from a donor strand and a region from an acceptor strand. In another example, the process results in the insertion of one strand, such as a donor nucleic acid sequence, into another strand, such as a target or recipient strand

As used herein, the term "homologous" refers to sequence that is that is sufficiently related to a reference sequence such that the two sequences recombine with each other in an appropriate host cell, particularly in a yeast cell. Number of differences tolerated between a nearly-identical sequence and its reference sequence is defined by ability of the two sequences to recombine with each other in the host cell. A homologous sequence can be at least 70, 75, 80, 85, 90, 91, 92, 95, 96, 97, 98, or 99% identical, nearly-identical, or identical to a reference sequence.

As used herein, the term "recombinase" refers to an agent, preferably a polypeptide which stimulates, e.g., catalyzes, the exchange of genetic information between nucleic acids.

The term "single-stranded DNA binding protein" refers to a polypeptide which binds, for example with an affinity of less than 10 µM, 100 nM, or 10 nM to single-stranded DNA of variable sequence. The *E. coli* SSB protein (single-stranded DNA binding protein), however, refers to a specific polypeptide (i.e. SWISSPROT:P02339).

The details of one or more embodiments of the invention are set forth in the description below. Other features, objects, and advantages of the invention will be apparent from the description and the claims.

### BRIEF DESCRIPTION OF THE DRAWINGS

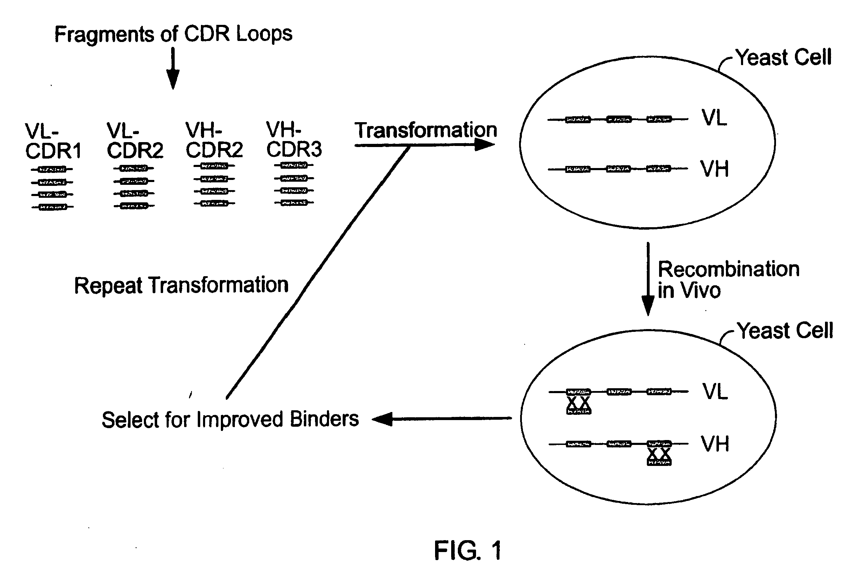

FIG. 1 depicts an exemplary method of recombining immunoglobulin proteins in a yeast cell.

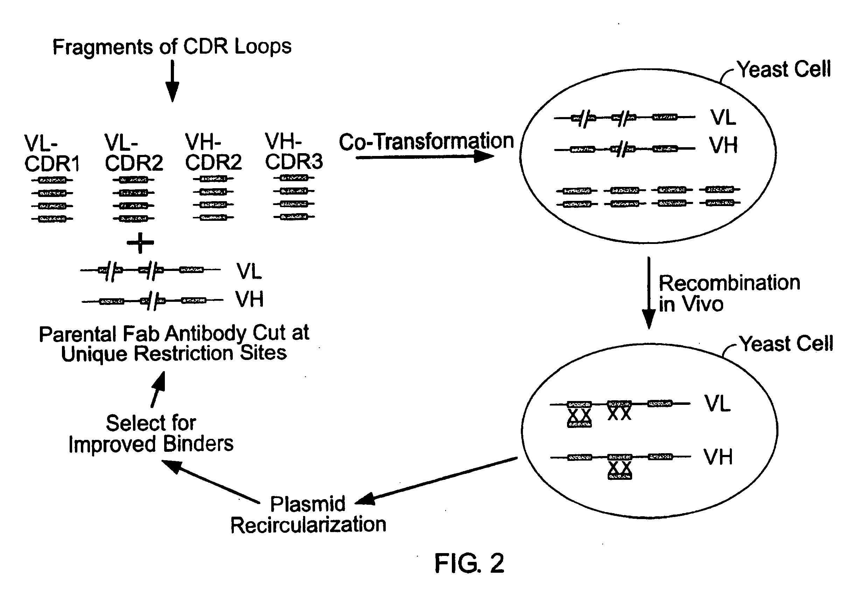

FIG. 2 depicts an exemplary cyclic method of recombining immunoglobulin proteins in a yeast cell.
FIGs. 3 and 4 depict exemplary methods of recombining immunoglobulin genes in yeast cells for yeast cell display. In FIG. 4, plasmids in haploid cells include sites **42** and **44** for site specific recombination **46**. Homologous recombination **48** occurs between two different *LEU2* alleles.
FIG. 5 depicts an exemplary method for assembling a immunoglobulin light chain expressing nucleic acid into an integrating yeast vector.
FIG. 6 depicts an exemplary method for assembling a immunoglobulin heavy chain expressing nucleic acid into an integrating yeast vector.
FIG. 7 depicts an exemplary method for varying immunoglobulin heavy and light chain coding sequences in separate haploid cells, mating cells containing the varied coding sequences to produce a diploid Fab repertoire, affinity selecting binding Fabs from the repertoire, sporulating cells that display binding Fabs, and optionally, performing additional rounds of affinity maturation.

### DETAILED DESCRIPTION

The invention provides, *inter alia,* a method of preparing a nucleic acid sequence that encodes a polypeptide that is displayed on a heterologous cell surface. In many implementations, a population of nucleic acid sequences is prepared, e.g., forming a nucleic acid library. The library can be screened, for example, using cell-based display and selection.

The method generally includes recombining a donor nucleic acid and an acceptor nucleic acid to form a recombined nucleic acid that encodes a polypeptide that is displayed. The recombination reaction is an in vivo reaction, meaning that at least the resolution of recombination intermediates (which in some implementations may be formed outside a cell) occurs within a cell. Both site-specific recombination and homologous recombination can be used.

A variety of cells, including yeast, bacterial, and mammalian cells, support homologous recombination with heterologous nucleic acids. Yeast cells, in particular, have been shown to be highly efficient at recombining nucleic acids with free ends with homologous counterparts. See, e.g., Orr-Weaver et al. (1981) Proc. Natl. Acad. Sci. USA 78:6354-8. In yeast, sequences with about 100 nucleotides of homology and even as little as 15 nucleotides of homology can be successfully used for homologous recombination. Oligonucleotides of around 80 nucleotides in length have been demonstrated to integrate at a homologous location and excise a counter selectable marker (Storici et al. (2001) Nature Biotechnol. 19:773).

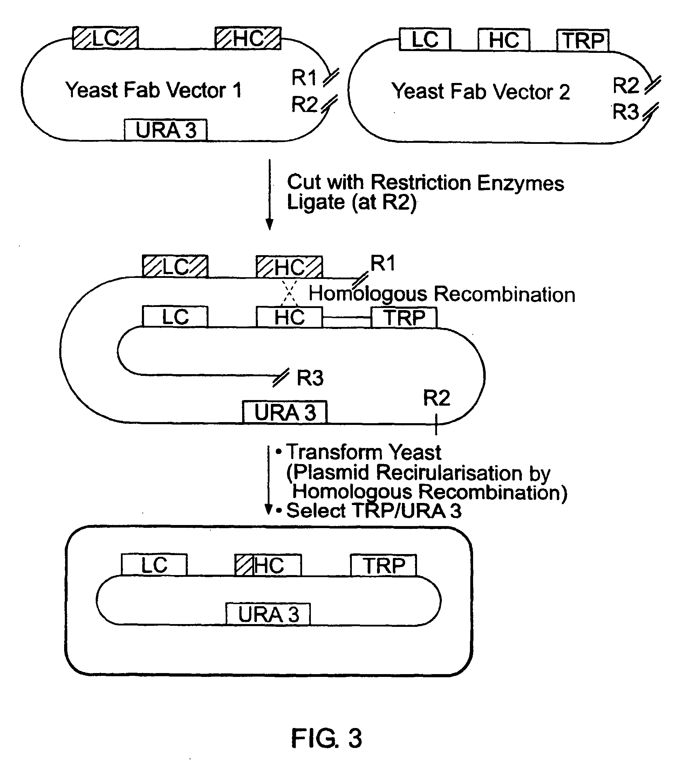

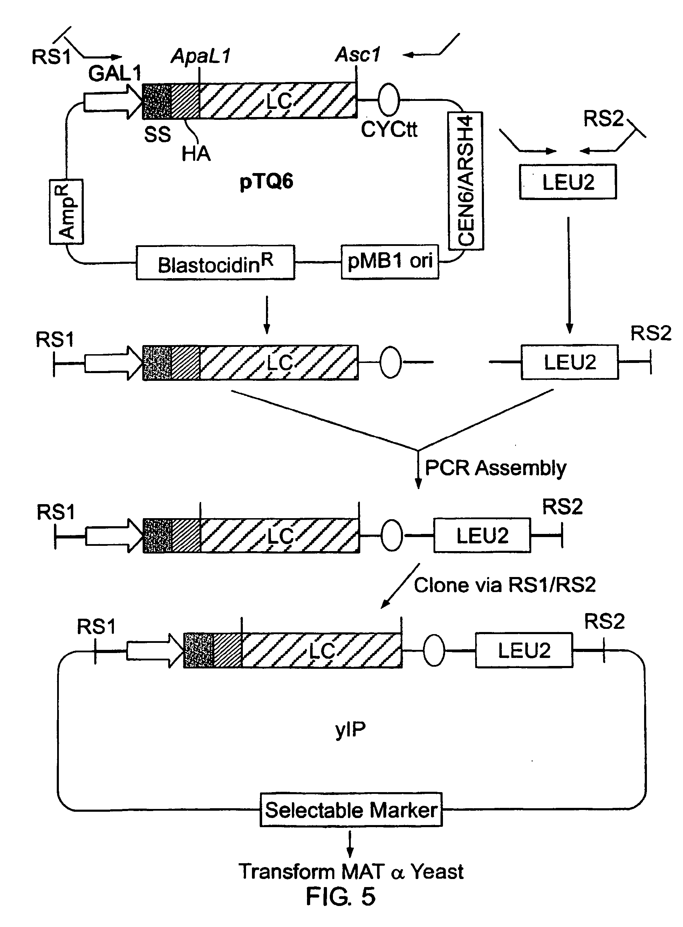

First, a variety of exemplary embodiments are set forth.
1. In a first example, the donor nucleic acid and the acceptor nucleic acid are combined in vitro and then introduced into a cell.
   A vector for yeast display includes a promoter and a sequence encoding a signal sequence, a first coding sequence encoding an N-terminal constant region of an immunoglobulin variable domain (e.g., VL) and a second coding sequence encoding the N-terminal region of an immunoglobulin constant domain (e.g., CL). A unique restriction enzyme site is positioned between the first and second coding sequences. At this stage, the vector does not encode a functional immunoglobulin chain as sequence encoding the CDR regions and intervening framework regions are omitted from the vector.
   In some implementations, the vector can also include a sequence encoding an anchor protein or fragment thereof. The anchor protein can be, for example, Aga1p or Aga2p.
   The vector nucleic acid is digested with the restriction enzyme and then combined with a pool of diverse nucleic acids that encode an immunoglobulin variable domain and at least the N-terminus of the constant domain. The nucleic acids of the pool are homologous to the ends of the linearized vector. The mixture of linearized vector nucleic acid and the diverse nucleic acids is electroporated into yeast cells. Homologous recombination within the cells results in circularization of the vector and introduction of one diverse nucleic acid from the pool into each copy of the vector. After recombination, the sequence encoding the immunoglobulin variable domain is completed. The vector, at this stage, encodes a polypeptide with a signal sequence and a complete variable domain and constant domain (e.g., VL-CL).
2. In a second example, a cell display library is screened to identify candidate ligands. The candidate ligands are diversified by recombination to form a secondary cell display library.
   A yeast display library that displays Fab fragments is screened for binding to a target molecule. Yeast cells that encode Fabs that bind the target are selected. Library plasmids that encode the Fabs are isolated from the yeast cells. The plasmids are digested with restriction enzymes that cleave uniquely on either side of a region that includes one or more CDRs of one of the immunoglobulin chains. The larger fragment (i.e., backbone fragment) of the plasmids is purified.
   The linearized plasmid is combined with a pool of diverse nucleic acids that encode the one or more CDRs that were excised. The diverse nucleic acids can be double stranded or single stranded. One requirement is that the diverse nucleic acids overlap in sequence to the terminal regions of the linearized plasmid. The overlap can be at least 15, 20, or 50 nucleotides at each terminus. The mixture is transformed into yeast cells where recombination ensues and introduces the excised CDR-coding sequence into the context of antigen binding sites selected in the first screen.
   The transformed yeast cells can be used as a cell display library. For example, the cells can be re-screened for binding to the target molecule. This strategy enables polypeptides identified in an initial screen to be rediversified in specific regions, such as one or more CDRs. Further, as described below, the diverse nucleic acids which are incorporated by recombination can be from a variety of sources, for example: the same population of molecules (thereby shuffling combinations of the specific region), synthetic sequences, and sequences from natural sources.
3. In a third example as with the second example, candidate ligands from a cell display library are diversified by recombination to form a secondary cell display library. The selected ligands are diversified without isolation of library nucleic acid from the selected cells.
   A yeast display library that displays Fab fragments is screened for binding to a target molecule. Yeast cells that encode Fabs that bind the target are selected. Each cell is transformed with a nucleic acid cassette that includes the *URA3* marker. The ends of the cassette are homologous to sequences in one of the Fab chains. The two ends can represent non-adjacent or adjacent sequences.
   If the cassette termini correspond to non-adjacent sequences, transformation and selection of Ura+ cells results in a population of cells in which part of the sequence encoding the immunoglobulin is replaced by the *URA3* marker. The *URA3* marker can be used to delete one or more CDRs. If the cassette termini correspond to adjacent sequences, transformation and selection of Ura+ cells results in a population of cells in which part of the sequence encoding the immunoglobulin is disrupted by the *URA3* marker. The disruption can be position, e.g., within a CDR.
   The cells are transformed with a diverse pool of nucleic acids (single-stranded or double-stranded) that encodes at least the region of the immunoglobulin gene sequence that is deleted or disrupted by the URA3 marker and sufficient sequence adjacent to that region to allow for homologous recombination with the immunoglobulin-encoding sequence. After transformations, cells are grown on 5-fluoroorotic acid (5-FOA) to select for ura- cells. Such cells arise when a nucleic acid of the diverse pool has recombined with the Fab encoding sequence and has replaced the cassette. As a result, the immunoglobulin-encoding sequences (e.g., donor sequences) from the diverse pool are combined with other immunoglobulin sequence (e.g., acceptor sequences) isolated from the display library.
   The method can be used to vary one chain of the Fab while retaining sequences for the other chain. Further, if the disruption or deletion by the *URA3* marker is localized to one or two CDRS, rather than all three CDRs within one immunoglobulin chain, then new CDR combinations within a chain can be generated.
4. In a fourth example, two libraries, each encoding a different Fab chain, are constructed in yeast cells of different mating types. The libraries are combined by mating and recombination to form a library of Fab loci, i.e., an artificial gene locus that includes a sequence encoding a light chain and a sequence encoding a heavy chain.
   Prior to recombination, a library of light chains is constructed using cells of one mating type. The light chain library (either plasmid-borne, or chromosomal) includes a polypeptide coding region that encodes a light chain and a site specific recombination site. A marker gene can be disposed such that it is separated from the coding region by the recombination site. Likewise, prior to recombination, a heavy chain library is constructed in cells of the opposite mating type. The heavy chain library includes a polypeptide coding region that encodes a heavy chain and a site specific recombination site. A different marker gene can, again, be disposed such that it is separated from the coding region by the recombination site.
   Cells of the two libraries are mated together. A gene encoding a recombinase specific for the site specific recombination site is induced. The recombinase activates strand exchange between the heavy chain and the light chain library sequences in the diploid cells produced by mating. The result of the recombination is a Fab locus. In the case of chromosomal libraries, for instance, the heavy and light chains are recombined onto the same chromosome.
   The construction of a single locus that includes both heavy and light chain coding sequences is particularly useful for recovering selected Fabs in bulk. If the coding sequences were on separate chains, after nucleic acid purification from a population of cells expressing different Fabs, the sequences encoding the heavy and light chains of a single Fab would sort randomly with those encoding other Fabs. In contrast, a single nucleic acid fragment that encodes both chains would retain the linkage even when many different Fab coding sequences are manipulated in the same reaction mixture.
5. In a fifth example, related to the second example, a cell display library is screened to identify candidate ligands. The candidate ligands are diversified by recombination to form a secondary cell display library.
   A yeast display library that displays Fab fragments is screened for binding to a target molecule. Yeast cells that encode Fabs that bind the target are selected. Single-stranded nucleic acids encoding one or both of the immunoglobulin chains of the Fab are isolated. Oligonucleotides are annealed to sequences encoding a framework region (or complement thereof) on either side of one of the CDRs. These oligonucleotides form double-stranded regions which are cleaved by restriction enzymes. The result is a linear single stranded nucleic acid from which a CDR encoding sequence has been excised.
   This cleaved single-stranded nucleic acid is combined with a pool of diverse nucleic acids that encode the CDR encoding sequence (or complement thereof). If the nucleic acids of the pool are single-stranded, the sense of the nucleic acids of the pool is opposite that of the cleaved single-stranded nucleic acid. The mixture is transformed into yeast cells where recombination ensues and introduces the CDR into the context of antigen binding sites selected in the first screen.
6. In a sixth example, the cell display library is a mammalian cell display library.
   A first mammalian cell display library is constructed from a first Fab expression cassette (e.g., episomal or integrated) that includes a functional selectable marker (e.g., hygromycin) and a non-functional selectable marker (e.g., mutated Blastocidin resistance gene). The Fab light chain gene and the Fab heavy chain gene are separated by a site specific recombination site (e.g., Cre/lox). The encoded Fab fragment is display on the mammalian cell surface as one of the chains, typically the heavy chain, is attached to a eukaryotic transmembrane domain (e.g., a fusion to the PDGFR transmembrane domain). A second Fab antibody display library is similarly constructed from a second Fab expression cassette (e.g., episomal or integrated) that includes a second functional selectable marker (e.g., neomycin) and a non-functional selectable marker (e.g., mutated blastocidin resistance gene). The non-functional selectable marker of the second cassette is a different allele of the non-functional selectable marker of the first cassette. Recombination between the two alleles can regenerate a functional allele. The two non-functional alleles can be engineered. As with the first cassette, the light chain and heavy chain genes are separated by a site specific recombination sequence (e.g., Cre/lox).
   The two cassettes of Fab antibodies of the two different mammalian cell display libraries are brought together by cell fusion (e.g., using PEG or Sendai virus). The two cassettes are able to contact each other such that site specific recombination can occur allowing strand exchange and generation of a recombination intermediate that can be resolved by homologous recombination between the two defective alleles of the non-functional selectable marker (e.g., the two different blastocidin resistance alleles). Recombination generates a functional blastocidin resistance marker gene. The selection for Hygromycin and Blastocidin or neomyocin and Blastocidin generates pools of cells that include sequences encoding new combinations of heavy and light chain genes, not present in the two original libraries.
7. A seventh example features a phage display repertoire that is diversified by in vivo recombination. A first pool of Fab antibodies is cloned into a phagemid expression vector fused to the p3 phage anchor protein but separated from a first, non-functional allele of an first antibiotic resistance marker (Tet gene carrying a first mutation) by a unique EcoR1 restriction sites. The phagemid vector also carries a functional selectable second antibiotic resistance marker (AmpR). The phagemid Fab display library is packaged using helper phage in a restriction negative and modification negative host cell (R-M-) to produce a library phage that include nucleic acid that is not methylated by the restriction-modification system of choice.
   These phage are infected into F+ *E. coli* cells which are restriction positive and modification positive (R+ M+). In particular, these cells also harbor nucleic acid from a second pool of constructs encoding Fab antibodies. The second pool of Fab-encoding constructs are cloned in a second expression plasmid carrying a third functional antibiotic resistance marker (e.g., Chloramphenicol resistance) and separated from a second, non-functional allele of the first antibiotic resistance marker (e.g., tet resistance gene carrying a second mutation different to the first but which can complement each other to produce a functionally active Tet gene).
   The *E. coli* strain is restriction and modification plus so unmethylated DNA is cleaved at *Eco*RI restriction sites so the first said unprotected phagemid Fab pool is cleaved at the appropriate unique EcoRI restriction site producing a linear vector. The second said pool of Fab antibodies contained within second said expression plasmid is protected and remains uncleaved. Homologous recombination (or site specific recombination) ensues at sites of homology between Fab antibody genes (i.e., the light chain and heavy chain genes) and between the two defective antibiotic resistance genes (e.g., two tet genes with different complementary mutations) which recombine to produce a functional resistance marker (e.g., tetR). This recombined phagemid vector can then be rescued by helper phage and used to produce viral particles that display recombined Fab genes.
8. In an eight example, a yeast cell display library is screened to identify candidate ligands. The candidate ligands are diversified by recombination to form a secondary display library as follows.
   Referring also to FIG. 3, a first yeast display library is configured on a yeast plasmid that includes a gene encoding a Fab light chain, a gene encoding a Fab heavy chain, and an auxotrophic marker gene (e.g., *URA3*). Two unique non-compatible restriction sites, R1 and R2, are positioned between the Fab heavy chain gene and the auxotropic marker.
   A second yeast display library is configured on a yeast plasmid that includes a gene encoding a Fab light chain, a gene encoding a Fab heavy chain, and an auxotrophic marker gene, different from the corresponding marker in the first library. For example, the auxotrophic marker of the second library can be *TRP1* when the corresponding marker of the first library is *URA3.* Two unique non-compatible restriction sites, R2' and R3, are positioned between the auxotrophic marker and the Fab light chain gene. The R2' site is compatible with the R2 restriction site of the first yeast display library. For example, R2' and R2 can be cleaved by the same restriction endonuclease. In another example, they are cleaved by different restriction endonucleases, but nevertheless have compatible cohesive overhangs.
   The first and second yeast display libraries are screened to identify Fab's that have at least a minimal binding activity against a target molecule. A pool of plasmids encoding the identified Fab's is isolated from each library. The first pool is digested with restriction endonucleases that cleave at R1 and R2. The second pool is digested with restriction endonucleases that cleave at R2' and R3. Appropriate fragments (as seen in FIG. 5) from each pool are ligated together using the compatible ends formed by R2 and R2'. The ligation products are linear DNAs that includes genes encoding two Fab antibodies and two different auxotrophic markers (e.g., *URA3* and *TRP1*)*.*
   These linear DNA molecules are transformed into yeast cells. Within the cells, the DNA molecules are circularized by intra-molecular homologous recombination. Recombination can occur at any homologous sites, e.g., within the two light chain genes and the two heavy chain genes. Depending on the implementation, recombination can also occur in the intervening region between the heavy and light chain genes. However, this region can be engineered to be heterologous to reduce recombination in the region. In another implementation, the heavy and light chain genes lack CH domains (e.g., CH1 and CL), e.g., they are arranged as scFv's. The omission of CH domains favors recombination within the variable domains where diversity is found. After transformation, the cells can be grown under conditions selective for both the auxotrophic markers, e.g., on media lacking uracil and tryptophan.
9. In a ninth example, a cell display library is diversified by V gene shuffling using a combination of site-specific recombination and homologous recombination. The diversified library is formed by mating cells from two different pools, inducing site-specific recombination, and selecting for gene shuffling events.

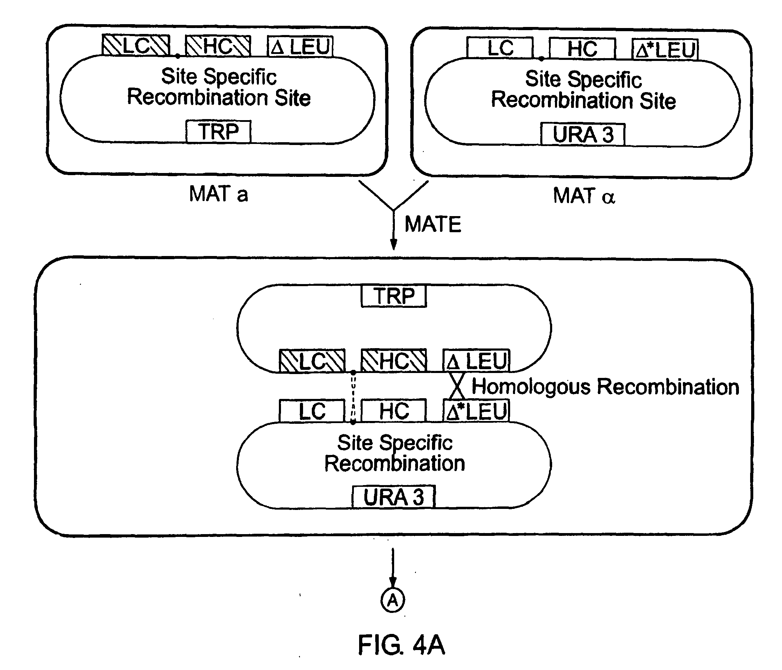

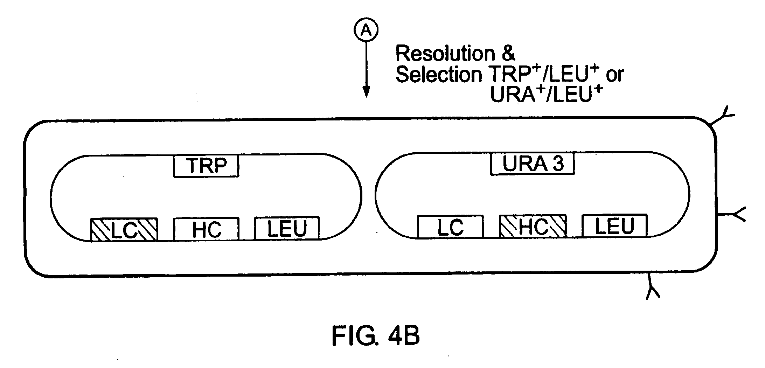

Referring also to FIG. 4, the yeast cells of the first pool (top left, FIG. 4) include expression plasmids that have a gene encoding a Fab light chain, a gene encoding a Fab heavy chain, a first allele of non-functional, first auxotrophic marker (e.g., *leu2-701*), and a functional, second auxotrophic marker (e.g., *TRP1*)*.* A site-specific recombination site (e.g., a lox site) is positioned between the light chain gene and the heavy chain gene. *leu2-701* is a non-functional allele of *LEU2* that includes a stop codon within the 5' region (e.g., within the first 20 codons) of the *LEU2* coding region. Such an allele can be created by genetic engineering. The yeast cells of the first pool have a first mating type.

The yeast cells of the second pool (top right, FIG. 4) include expression plasmids that have a gene encoding a Fab light chain, a gene encoding a Fab heavy chain, a second non-functional allele of the first auxotrophic marker (e.g., *leu2-702*), and a functional, third auxotrophic marker (e.g., *URA3*). The functional third marker typically differs from the functional second marker of the first pool. A site-specific recombination site (e.g., a lox site) is positioned between the light chain gene and the heavy chain gene. *leu2-702* is a non-functional allele of *LEU2* that that includes a stop codon within the 3' region of the *LEU2* coding region. Such an allele can also be created by genetic engineering. The yeast cells of the second pool have a second mating type.

Cells of the two pools are combined so that cells of the two pools mate, e.g., in multiple combinations. During or after mating, an appropriate site specific recombinase is expressed. For example, the gene encoding the recombinase can be regulated by a pheromone inducible promoter such as *FUS1,* or a diploid specific promoter. The recombinase induces site-specific recombination, which can form a large plasmid that includes sequences from a plasmid of the first pool and a plasmid of the second pool. Selection for a functional allele of the first auxotrophic marker requires homologous recombination between the first and second alleles of the marker. After mating, and optionally growth under non-selective conditions, the mated cells can be selected on media to require the first and second markers (e.g., Trp+ Leu+ cells) or to require the first and third markers (e.g., Ura+ Leu+ cells). The resulting cells form a diverse library that encode Fab antibodies which have undergone V gene shuffling such that new combinations of light chain and heavy chain genes are present relative to the combinations in the starting pools.

### Site Specific Recombination

Site-specific recombinases have both endonuclease and ligase properties. They recognize specific sequences of bases in DNA and exchange the DNA segments flanking those segments. Numerous site-specific recombination systems from various organisms have been described. Examples include the integrase/att system from bacteriophage lambda (Landy, A. (1993) Current Opinions in Genetics and Devel. 3:699-707), the Cre/loxP system from bacteriophage P1 (Hoess and Abremski (1990) In Nucleic Acids and Molecular Biology, vol. 4. Eds.: Eckstein and Lilley, Berlin-Heidelberg: Springer-Verlag; pp. 90-109), and the FLP/FRT system from the Saccharomyces cerevisiae 2µ circle plasmid (Broach et al. Cell 29:227-234 (1982)). As exemplified herein, site-specific recombinases can be used to generate nucleic acid combinations of nucleic acid sequences that reside on the same strand from parental material that resides on different strands.

Transposases can also be used to transfer nucleic acid segments between strands. Transposases are typically encoded by genes with mobile genetic elements, known as transposons. Integration of transposons can be random or highly specific. Representatives such as Tn7, which are highly site-specific, have been applied to the in vivo movement of DNA segments between replicons (Lucklow et al., J. Virol. 67:4566-4579 (1993)). Artificial transposons can be used to mobilize a nucleic acid sequence from one strand to another. For example, a library of protein coding sequences can be generated within a transposon.

### Counter-Selectable markers

Counter-selectable markers are markers whose absence allows cell growth or survival, typically under a specialized condition. Counter-selectable markers can be used to select for recombination events that excise the markers. These markers can be used in many cell types, include yeast and mammalian cells.

For example, *URA3* and *CAN1* are counter-selectable markers that are functional in *S. cerevisiae. CAN1* confers sensitivity to canavanine. Loss of the *CAN1* marker is selected by growing cells in canavanine. URA3 is particularly useful as both its presence and absence can be selected for, provided the host cell is otherwise *ura3*-. The presence of *URA3* can be selected by growth on minimal media lacking uracil while its absence can be selected by growth on media that includes 5-FOA.

For mammalian cells, the thymidine kinase gene can be used as a counter-selectable marker. Loss of the *tk* gene can be selected by growth in media that includes Gancyclovir. Many other counter-selectable markers are known for a variety of systems.

### Cell-Based Display Libraries

In still another format, the library is a cell-display library.

Proteins are displayed on the surface of a cell, e.g., a eukaryotic or prokaryotic cell. Exemplary prokaryotic cells include *E. coli* cells, *B. subtilis* cells, spores (see, e.g., Lu et al. (1995) Biotechnology 13:366). Exemplary eukaryotic cells include yeast (e.g., *Saccharomyces cerevisiae, Schizosaccharomyces pombe, Hanseula,* or *Pichia pastoris*). One implementation of yeast surface display is described in Boder and Wittrup (1997) Nat. Biotechnol. 15:553-557.

WO 03/029,456 describes, among other things, a yeast display system that can be used to display immunoglobulin proteins such as Fab fragments, and the use of mating to generate combinations of heavy and light chains.

In one embodiment, nucleic acid encoding immunoglobulin variable domains are cloned into a vector for yeast display. The cloning joins the nucleic acid encoding at least one of the variable domains with nucleic acid encoding fragments of a yeast cell surface protein, e.g., Flo1, a-agglutinin, α-agglutinin, or fragments derived thereof e.g. Aga2p, Aga1p. A domain of these proteins can anchor the polypeptide encoded by the diversified nucleic acid sequence by a GPI-anchor (e.g. a-agglutinin, α-agglutinin, or fragments derived thereof e.g. Aga2p, Aga1p), by a transmembrane domain (e.g., Flo1). The vector can be configured to express two polypeptide chains on the cell surface such that one of the chains is linked to the yeast cell surface protein. For example, the two chains can be immunoglobulin chains.

A number of methods are available to introduce nucleic acids into yeast cells, including the LiAc/PEG method, electroporation, and the spheroplast method.

Examples of cells for mammalian cell display include: COS cells, CV-1 cells, OVCAR cells, lymphocytes, lymphocytic cell lines, e.g., NSO myeloma cells and SP2 cells, and Chinese Hamster Ovary (CHO cells).

Further, as seen above, some implementations are also appropriate for other display methods, e.g., phage display.

### Yeast Mating

An exemplary implementation of the method utilizes the mating of *S*. *cerevisiae* cells. The first cell has a first mating type, e.g., MATa; the second cell has a second mating type different from the first, e.g., MATα. The two cells are contacted with one another, and yeast mating produces a single cell (e.g., MAT**a**/α) with a nucleus formed by the fusion of the respective genomes of both the first and second cells. The fusion event brings the display library nucleic acid of the first cell and a nucleic acid of the second cell into the same nucleus and provides them with an opportunity to recombine. Recombination between two similar loci (e.g., two artificial Fab loci) can result in new Fab encoding sequences that includes CDRs from both parental loci. For example, homologous recombination crossover may occur in a framework region between CDR2 and CDR3 resulting in a recombined sequence with a sequence encoding the CDR2 from one parent locus and CDR3 from the other parent.

In another example, site-specific recombination is induced. See, e.g., example 4 above. A variation of this example is one in which site-specific recombination is induced between two complete Fab loci.

The nucleic acid of the second cell can also be a display library nucleic acid, e.g., a nucleic acid selected concurrently with the first nucleic acid, or in a separate screen. The nucleic acid can be a non-display library nucleic acid, e.g., a linear nucleic acid such as a PCR amplification product or a synthetic oligonucleotide.

The method can including providing multiple first cells, all of the same first mating type where each first cell displays a polypeptide (encoded by a first nucleic acid) with a first property; and providing multiple second cells, all of the same second mating type where each second cell has a nucleic acid with a second property and that is homologous in at least some regions to the first nucleic acids. The cells are fused, e.g., in multiple pairwise combinations. Recombination is induced or enabled. Then displayed, varied polypeptides are selected for a property, e.g., the first or second property, or a new property. This method is used to "breed" proteins.

### Recombination Stimulating Agents

A variety of agents can be used to stimulate recombination. For embodiments that occur entirely within cells, such agents can be provided, e.g., by inducing gene expression, typically using a heterologous expression cassette. For embodiments that initiate outside of cells, the agents can be, e.g., contacted to the nucleic acids prior to their introduction into cells, introduced concurrently with the nucleic acids, or expressed from within the cell.

For example, donor nucleic acid for homologous recombination can be treated in vitro with agents that stimulate recombination. The nucleic acid can be coated with the agent. The agent can be a polypeptide such as a recombinase or a single-stranded DNA binding protein.

The recombinase can be a recA-like protein which polymerizes as a filament on an incoming ssDNA and induces strand exchange by displacing one of the annealed strands from a duplex with the incoming strand. Such proteins can include eukaryotic proteins, such as Rad51, Rad52, Rad54, DMC1, and mei3; and prokaryotic proteins such as *E. coli:* recA (e.g., recA-803 and wildtype recA), *E. coli* SSB (single-stranded binding protein), and T4 bacteriophage gene 32. Further, many organisms have recA-like strand-transfer proteins (e.g., Fugisawa et al. (1985) Nucl. Acids Res. 13:7473; Hsieh et al. (1986) Cell 44: 885; Hsieh et al. (1989) J. Biol. Chem. 264: 5089; Fishel et al. (1988) Proc. Natl. Acad. Sci. USA 85: 3683; Cassuto et al. (1987) Mol. Gen. Genet. 208: 10; Ganea et al. (1987) Mol. Cell Biol. 7: 3124; Moore et al. (1990) J. Biol. Chem. 19:11108; Keene et al. (1984) Nucl. Acids Res. 12: 3057; Kimiec (1984) Cold Spring Harbor Symp. 48:675; Kimeic (1986) Cell 44: 545; Kolodner et al. (1987) Proc. Natl. Acad. Sci. USA 84:5560; Sugino et al. (1985) Proc. Natl. Acad. Sci. USA 85: 3683; Halbrook et al. (1989) J. Biol. Chem. 264: 21403; Eisen et al. (1988) Proc. Natl. Acad. Sci. USA 85: 7481; McCarthy et al. (1988) Proc. Natl. Acad. Sci. USA 85: 5854; Lowenhaupt et al. (1989) J. Biol. Chem. 264: 20568). Additional examples include: sep1 (Kolodner et al. (1987) Proc. Natl. Acad. Sci. U.S.A. 84:5560; Tishkoff et al. Molec. Cell. Biol. 11:2593), RuvC (Dunderdale et al. (1991) Nature 354:506), DST2, KEM1, XRN1 (Dykstra et al. (1991) Molec. Cell. Biol. 11:2583), STPα/DST1 (Clark et al. (1991) Molec. Cell. Biol. 11: 2576), HPP-1 (Moore et al. (1991) Proc. Natl. Acad. Sci. U.S.A. 88: 9067), and uvsX. The recA-803 protein is a high-activity mutant of recA. In addition, functionally active fragments of these proteins can be used.

A substantially pure preparation of the polypeptide can be prepared, e.g., by standard biochemical purification from *E. coli* cells overexpressing the recombinase using methods known in the art, see for example McEntee et al. (1980) Proc Natl Acad Sci USA 77:857-861. Alternatively, recA protein can also be purchased, e.g., from Pharmacia (Piscataway, NJ). The nucleic acid can be incubated with the purified polypeptide, e.g., in a buffer solution at room temperature, for a sufficient time for the polypeptide to adhere to or coat the nucleic acid. One method for coating the nucleic acid with recA is as follows. Briefly, the nucleic acid is denatured in an aqueous solution at 95°C for five minutes, immediately cooled to 4°C for one minute, and then rapidly centrifuged for 20 seconds at 0°C. The denatured nucleic acid is then mixed with a reaction buffer containing a nonhydrolyzable nucleotide, for example ATPγS. The reaction buffer can also include 0.5 to 2 mM Mg²⁺Cl₂, 50 mM Tris·HCl pH 7.5, and 0.5 mM dithiothreitol. Coating can be initiated by the addition of purified recA at 37°C for 10 minutes. The concentration of recA can be vary from in ratio of 1:3 to 3:1 relative to the concentration of individual bases in the denatured nucleic acid.

The coated nucleic acid can be isolated, e.g., by gel filtration, e.g., using a spin column. The extent of coating can be evaluated by electrophoresis under native conditions.

**Endonucleases.** Endonucleases that cleave a nucleic acid within the phosphate backbone can be used to generate an end that is a substrate for recombination. The end can be rendered at least partially single-stranded, e.g., by an endogenous exonuclease.

In *Saccharomyces cerevisiae,* the HO endonuclease cleaves only three sites in the genome. The cleavage event digests a single site, the *HO* cleavage site at the *MAT* locus. The other two sites reside in the silent mating cassettes *HMR**a*** and *HML*α and are resistant to cleavage. Cleavage of the *MAT* locus triggers mating type switching, which is a recombination event between the *MAT* locus and one of the two silent mating cassettes. Yeast strains can be engineered that express *HO*, but do not undergo mating type switching by deleting the *MAT* locus. The *HO* endonuclease can be used to simulate recombination between a display library vector in a yeast cell and a donor nucleic acid.

In one embodiment, the display library vector is targeted with a selectable marker cassette that includes an *HO* cleavage site. The selectable marker, e.g., *URA3,* can be both selectable and counter selectable. In combination with *HO* endonuclease mediated cleavage, the a donor nucleic acid or a pool of donor nucleic acids can be introduced into the cells. The donor nucleic acids include ends that are homologous to the.

In another embodiment, an intron, e.g., the yeast *MAT**a*** intron is inserted within the sequence that encodes the displayed ligand. With respect to the example of immunoglobulin display, the intron can be positioned within a framework coding region between two CDR coding regions. The intron includes the HO endonuclease site. The site does not disrupt the translation of the immunoglobulin domain since the intronic sequence is spliced out. Recombination in the immunoglobulin coding sequence is induced by the expression of HO endonuclease. The *HO* gene can be controlled, e.g., by the inducible GAL promoter. See, e.g., Herskowitz et al. (1991) Methods Enzymol. 194:132-46. The endonuclease can be induced, e.g., during or after transformation with a diverse nucleic acid pool or during or after mating to a cell that includes a homologous sequence.

Of course, any endonuclease can be expressed ectopically in a cell. Rare cutters, for example, may be used without deleterious effect.

### Constructing Diverse Nucleic Acids

In many implementations, the recombination methods use a pool of diverse nucleic acids as donor sequences for incorporation into one or more different acceptor sequences. Such a diverse pool of nucleic acids can be obtained from a variety of sources.

**Initial Design.** The construction of the diverse pool is based in part on the intended usage, e.g., the acceptor sequence and the cell type in which recombination occurs. One exemplary design includes regions of high (or perfect) homology at the 3' and 5' termini of the diverse nucleic acids, and a central segment. In this case, the diverse oligonucleotides vary to the greatest extent in the central segment, and to a much lesser extent in the homology regions.

The homologous regions can be designed to be sufficiently homologous to an intended template nucleic acid or a consensus sequence so that recombination ensues.

**PCR Amplification.** PCR can be used to amplify diverse nucleic acids. Forward and reverse primers are designed to anneal to conserved regions that flank a region of diversity, e.g., the homology regions. Any mixture that includes potential diversity can be a template for amplification. For example, the mixture can be an environmental sample that includes different microorganisms, or a heterogeneous cell population (e.g., a spleen).

**Oligonucleotide-Directed Cleavage.** Diverse nucleic acids can be obtained by oligonucleotide-directed cleavage. This method results in the precise excision of single stranded nucleic acid. Generally, a synthetic oligonucleotide directs cleavage of a single-stranded target nucleic acid by the formation of a duplex (e.g., a homo- or hetero-duplex) between a single stranded region of the oligonucleotide and a region of a nucleic acid that is a source of diversity.

Some exemplary methods for such cleavage are described in USSN 09/837,306, filed 17 April 2001. In one particular embodiment, the method is used to cleave homo- or heteroduplexes formed by a plurality of diverse single-stranded nucleic acids and one or more cleavage-directing oligonucleotides. Thus, the method enables natural sources of diversity to be readily accessed. For example, a population of diverse single-stranded segments can be excised from the sources and used for recombination with an acceptor nucleic acid. An exemplary application of the method to nucleic acids encoding immunoglobulin domains is described in USSN 60/343,954 and WO 03/035,842.

**Automated Oligonucleotide Synthesis.** The diverse nucleic acids can be synthesized, e.g., using automated oligonucleotide synthesizers. The synthesizers can be programmed to produce oligonucleotides that include at particular positions: a particular nucleotide, a mixture of nucleotides, a mixture of trinucleotides (or other oligomers), or an artificial nucleotide. The synthesizers typically use 3' phosphoramidite-activated and 5'-protected nucleotides to sequentially add nucleotides (or oligomers) to a solid support. Oligonucleotides can also be synthesized on a planar solid support, e.g., using photolithography (see, e.g., U.S. Patent No. 5,143,854, Fodor et al. (1991) Science 251:767-773; Fodor et al. (1993) Nature 364:555-556) or ink-jet printing (see, e.g., U.S. Patent No. 5,474,796). These oligonucleotide synthetic methods can be programmed to produce a large number of diverse individual oligonucleotides. After synthesis, the oligonucleotides are released from the array, e.g., using a chemical treatment or an enzyme. The released oligonucleotides are pooled for the diversification method described herein.

### Sources of Diverse Nucleic Acids

Display libraries include variation at one or more positions in the displayed polypeptide. The variation at a given position can be synthetic or natural. For some libraries, both synthetic and natural diversity are included.

**Synthetic Diversity.** Libraries can include regions of diverse nucleic acid sequence that originate from artificially synthesized sequences. Typically, these are formed from degenerate oligonucleotide populations that include a distribution of nucleotides at each given position. The inclusion of a given sequence is random with respect to the distribution. One example of a degenerate source of synthetic diversity is an oligonucleotide that includes NNN wherein N is any of the four nucleotides in equal proportion.

Synthetic diversity can also be more constrained, e.g., to limit the number of codons in a nucleic acid sequence at a given trinucleotide to a distribution that is smaller than NNN. For example, such a distribution can be constructed using less than four nucleotides at some positions of the codon. In addition, trinucleotide addition technology can be used to further constrain the distribution.

So-called "trinucleotide addition technology" is described, e.g., in Virnekas et al. (1994) Nucleic Acids Res. 22(25):5600-7. Oligonucleotides are synthesized on a solid phase support, one codon (i.e., trinucleotide) at a time. The support includes many functional groups for synthesis such that many oligonucleotides are synthesized in parallel. The support is first exposed to a solution containing a mixture of the set of codons for the first position. The unit is protected so additional units are not added. The solution containing the first mixture is washed away and the solid support is deprotected so a second mixture containing a set of codons for a second position can be added to the attached first unit. The process is iterated to sequentially assemble multiple codons. Trinucleotide addition technology enables the synthesis of a nucleic acid that at a given position can encode a number of amino acids. The frequency of these amino acids can be regulated by the proportion of codons in the mixture. Further the choice of amino acids at the given position is not restricted to quadrants of the codon table as is the case if mixtures of single nucleotides are added during the synthesis.

**Natural Diversity.** Libraries can include regions of diverse nucleic acid sequence that originate (or are synthesized based on) from different naturally-occurring sequences. An example of natural diversity that can be included in a display library is the sequence diversity present in immune cells (see also below). Nucleic acids are prepared from these immune cells and are manipulated into a format for polypeptide display. Another example of naturally diversity is the diversity of sequences among different species of organisms. For example, diverse nucleic acid sequences can be amplified from environmental samples, such as soil, and used to construct a display library.

### Antibody Display Libraries

In one embodiment, the display library presents a diverse pool of polypeptides, each of which includes an immunoglobulin domain, e.g., an immunoglobulin variable domain. Display libraries are particular useful, for example for identifying human or "humanized" antibodies that recognize human antigens. Such antibodies can be used as therapeutics to treat human disorders such as cancer. Since the constant and framework regions of the antibody are human, these therapeutic antibodies may avoid themselves being recognized and targeted as antigens. The constant regions are also optimized to recruit effector functions of the human immune system. The *in vitro* display selection process surmounts the inability of a normal human immune system to generate antibodies against self-antigens.

A typical antibody display library displays a protein that includes a VH domain and a VL domain. The display library can display the antibody as a Fab fragment (e.g., using two polypeptide chains) or a single chain Fv (e.g., using a single polypeptide chain). Other formats can also be used.

As in the case of the Fab and other formats, the displayed antibody can include a constant region as part of a light or heavy chain. In one embodiment, each chain includes one constant region, e.g., as in the case of a Fab. In other embodiments, additional constant regions are displayed.

Within the library, recombination can be used to generate variation, e.g., in a single immunoglobulin domain (e.g., VH or VL) or in multiple immunoglobulin domains (e.g., VH and VL). The variation can be introduced into an immunoglobulin variable domain, e.g., in the region of one or more of CDR1, CDR2, CDR3, FR1, FR2, FR3, and FR4, referring to such regions of either and both of heavy and light chain variable domains. In one embodiment, variation is introduced into all three CDRs of a given variable domain. In another preferred embodiment, the variation is introduced into CDR1 and CDR2, e.g., of a heavy chain variable domain. Any combination is feasible.

Antibody libraries for cell-based display can be constructed by a number of processes (see, e.g. WO 03/029,456).

The recombination methods described herein can be used to incorporate diversity from a variety of sources, including from synthetic nucleic acid, naïve nucleic acids, patients (e.g., immunized or diseased human subjects), and animals (e.g., immunized animals).

**Natural Immune Sources.** In one embodiment, immune cells can be used as a natural source of diversity for the variation of antibodies, MHC-complexes and T cell receptors. Some examples of immune cells are B cells and T cells. The immune cells can be obtained from, e.g., a human, a primate, mouse, rabbit, camel, or rodent. The cells can be selected for a particular property. For example, T cells that are CD4⁺ and CD8⁻ can be selected. B cells at various stages of maturity can be selected.

In another embodiment, fluorescent-activated cell sorting is used to sort B cells that express surface-bound IgM, IgD, or IgG molecules. Further B cells expressing different isotypes of IgG can be isolated. In another embodiment, the B or T cell is cultured *in vitro.* The cells can be stimulated *in vitro,* e.g., by culturing with feeder cells or by adding mitogens or other modulatory reagents, such as antibodies to CD40, CD40 ligand or CD20, phorbol myristate acetate, bacterial lipopolysaccharide, concanavalin A, phytohemagglutinin or pokeweed mitogen.

In still another embodiment, the cells are isolated from a subject that has an immunological disorder, e.g., systemic lupus erythematosus (SLE), rheumatoid arthritis, vasculitis, Sjögren's syndrome, systemic sclerosis, or anti-phospholipid syndrome. The subject can be a human, or an animal, e.g., an animal model for the human disease, or an animal having an analogous disorder. In still another embodiment, the cells are isolated from a transgenic non-human animal that includes a human immunoglobulin locus.

In one embodiment, the cells have activated a program of somatic hypermutation. Cells can be stimulated to undergo somatic mutagenesis of immunoglobulin genes, for example, by treatment with anti-immunoglobulin, anti-CD40, and anti-CD38 antibodies (see, e.g., Bergthorsdottir et al. (2001) J Immunol. 166:2228). In another embodiment, the cells are naïve.

Naturally diverse sequences can be obtained as cDNA produced from mRNAs isolated from cell and samples described herein. Full length (i.e., capped) mRNAs are separated (e.g. by degrading uncapped RNAs with calf intestinal phosphatase). The cap is then removed with tobacco acid pyrophosphatase and reverse transcription is used to produce the cDNAs. The reverse transcription of the first (antisense) strand can be done in any manner with any suitable primer. See, e.g., de Haard et al. (1999) J. Biol. Chem 274:18218-30. The primer binding region can be constant among different immunoglobulins, e.g., in order to reverse transcribe different isotypes of immunoglobulin. The primer binding region can also be specific to a particular isotype of immunoglobulin. Typically, the primer is specific for a region that is 3' to a sequence encoding at least one CDR. Poly-dT primers(e.g., for the heavy-chain genes) or synthetic primers that hybridize to a synthetic sequence ligated to the mRNA strand may also be used.

cDNA can be amplified, modified, fragmented, or cloned into a vector to form an antibody library. See, e.g., WO 00/70023 and de Haard *et al.* (1999) *supra* and WO 03/035,842 which describe a method of cleaving cDNA using oligonucleotide-directed cleavage and incorporating immunological diversity into a template immunoglobulin sequence.

**Murine-Derived Human Immunoglobulins.** A pool of diverse nucleic acids can be obtained from the immune cells of an immunize animal. In one embodiment, the immunized animal is a transgenic animal (e.g., a mouse) that has human immunoglobulin genes. See, e.g., U.S. Patent No. 6,150,584; Fishwild et al. (1996) Nature Biotechnol. 14:845-85; Mendez et al. (1997) Nature Genet. 15:146-156; Nicholson et al. (1999) J. Immunol. 163:6898. One such transgenic mouse can be constructed as described in WO 94/02602 using a YAC for the human heavy chain locus, e.g., yHlC (1020 kb), and human light chain locus YAC, e.g., yK2 (880 kb). yH1C includes 870 kb of the human variable region, the entire D and JH region, human µ, δ, γ2 constant regions and the mouse 3' enhancer. yK2 includes 650 kb of the human kappa chain proximal variable region (Vκ), the entire Jκ region, and Cκ with its flanking sequences. Administration of an antigen to such mice elicits the generation of human antibodies against the antigen. The spleens of such mice are isolated. mRNA encoding the human antibody genes is extracted and used to produce a nucleic acid library encoding antibodies against the antigen. In some implementations, the library is mutagenized, e.g., affinity matured, *in vitro* prior to selection and screening.

### Screening Cell-Display Libraries

Cell-display technology can be used to obtain ligands that bind to a target. As illustrated, the display technology can be used at a number of points in the recombination process. In one example, a cell display library is screened to identify sequences which are then varied by recombination. In another example, a cell-display library is constructed by recombination, and then screened. In still another example, a cell-display library is screened, cells which encode binding ligands are varied by recombination to produce a secondary cell-display library, and then the secondary library is screened.

Generally, ligands can be identified from a cell-display library by one or more cycles of selection. Some exemplary selection processes are as follows.

**Panning.** The target molecule is immobilized to a solid support such as a surface of a microtitre well, matrix, particle, or bead. The display library is contacted to the support. Library members that have affinity for the target are allowed to bind. Non-specifically or weakly bound members are washed from the support. Then the bound library members are recovered (e.g., by elution) from the support. Recovered library members are collected for further analysis (e.g., screening) or pooled for an additional round of selection..

**Magnetic Particle Processor.** One example of an automated selection uses magnetic particles and a magnetic particle processor. In this case, the target is immobilized on the magnetic particles, e.g., as described below. The KingFisher^{™} system, a magnetic particle processor from Thermo LabSystems (Helsinki, Finland), is used to select display library members against the target. The display library is contacted to the magnetic particles in a tube. The beads and library are mixed. Then a magnetic pin, covered by a disposable sheath, retrieves the magnetic particles and transfers them to another tube that includes a wash solution. The particles are mixed with the wash solution. In this manner, the magnetic particle processor can be used to serially transfer the magnetic particles to multiple tubes to wash non-specifically or weakly bound library members from the particles. After washing, the particles are transferred to a tube that includes an elution buffer to release specifically and/or strongly bound library members from the particles. These eluted library members can be individually isolated for analysis (e.g., screening) or pooled for an additional round of selection.

**FACS.** It is also possible to use fluorescent cell sorting to sort cells from a cell display library. The cells can be contacted with a target that is fluorescently labeled. Cells that interact with the target can be detected by the FACS sorter and deflected into a container. Further, the cells can be contacted with an unlabeled target to form cell-target complexes. The cell-target complexes can then be labeled, e.g., using a fluorescent reagent that is specific for the target.

**Capillary Device for Washing Magnetic Beads.** WO 03049530 describes an apparatus and methods that can, in one implementation, be used to wash magnetic particles in a capillary tube. On exemplary apparatus features a capillary that houses magnetic particles. The chamber is located between a first magnet and a second magnet. The magnets and are attached to a frame that can be actuated from a first position to a second position. When the frame is actuated, the magnetic particles in the capillary are agitated.

To use the apparatus for display library screening, library members are contacted to magnetic particles that have an attached target. The particles are disposed in the capillary (before, during, or after the contacting). Then, the particles are washed in the capillary with cycles of agitation and liquid flow to remove non-specifically or weakly bound library members. After washing, bound library members can be eluted or dissociated from the particles and recovered. Cells can also be grown in the device, e.g., during a selection, to amplify bound cells.

A screen for binding ligands can include measures to identify ligands that bind to a particular epitope of a target or that have a particular specificity. This can be done, for example, by using competing non-target molecules that lack the particular epitope or are mutated within the epitope, e.g., with alanine. Such non-target molecules can be used in a negative selection procedure as described below, as competing molecules when binding a display library to the target, or as a pre-elution agent, e.g., to capture in a wash solution dissociating display library members that are not specific to the target.

**Iterative Selection.** In one embodiment, display library technology is used in an iterative mode. A first display library is used to identify one or more ligands for a target. These identified ligands are then varied by recombination to form a second display library. Higher affinity ligands are then selected from the second library, e.g., by using higher stringency or more competitive binding and washing conditions.

As discussed, the recombination can be targeted to regions known or likely to be at the binding interface. If, for example, the identified ligands are antibodies, then recombination can be directed to the CDR regions of the heavy or light chains as described herein. Likewise, if the identified ligands are enzymes, recombination can be directed to the active site and vicinity.

In one example of iterative selection, the methods described herein are used to first identify a protein ligand from a display library that binds a target with at least a minimal binding specificity for a target or a minimal activity, e.g., an equilibrium dissociation constant for binding of greater than 1 nM, 10 nM, or 100 nM. The nucleic acid sequence encoding the initial identified protein ligand are used as a template nucleic acid for the introduction of variations, e.g., to identify a second protein ligand that has enhanced properties (e.g., binding affinity, kinetics, or stability) relative to the initial protein ligand.

**Off-Rate Selection.** Since a slow dissociation rate can be predictive of high affinity, particularly with respect to interactions between polypeptides and their targets, the methods described herein can be used to isolate ligands with a desired kinetic dissociation rate (i.e. reduced) for a binding interaction to a target.

To select for slow dissociating ligands from a display library, the library is contacted to an immobilized target. The immobilized target is then washed with a first solution that removes non-specifically or weakly bound biomolecules. Then the immobilized target is eluted with a second solution that includes a saturation amount of free target, i.e., replicates of the target that are not attached to the particle. The free target binds to biomolecules that dissociate from the target. Rebinding is effectively prevented by the saturating amount of free target relative to the much lower concentration of immobilized target.

The second solution can have solution conditions that are substantially physiological or that are stringent. Typically, the solution conditions of the second solution are identical to the solution conditions of the first solution. Fractions of the second solution are collected in temporal order to distinguish early from late fractions. Later fractions include biomolecules that dissociate at a slower rate from the target than biomolecules in the early fractions.

Further, it is also possible to recover display library members that remain bound to the target even after extended incubation. These can dissociated, e.g., by cleaving the target from the support, or by incubating the target under conditions which support cell division so that bound cells divide and outnumber available binding sites on the solid support..

**Selecting or Screening for Specificity.** The display library screening methods described herein can include a selection or screening process that discards display library members that bind to a non-target molecule. In one implementation, a so-called "negative selection" step is used to discriminate between the target and related non-target molecule and a related, but distinct non-target molecule. The display library or a pool thereof is contacted to the non-target molecule. Members of the sample that do not bind the non-target are collected and used in subsequent selections for binding to the target molecule or even for subsequent negative selections. The negative selection step can be prior to or after selecting library members that bind to the target molecule.

**Screening Individual Library Members.** After selecting candidate display library members that bind to a target, each candidate display library member can be further analyzed, e.g., to further characterize its binding properties for the target. Each candidate display library member can be subjected to one or more secondary screening assays. The assay can be for a binding property, a catalytic property, a physiological property (e.g., cytotoxicity, renal clearance, immunogenicity), a structural property (e.g., stability, conformation, oligomerization state) or another functional property. The same assay can be used repeatedly, but with varying conditions, e.g., to determine pH, ionic, or thermal sensitivities.

As appropriate, the assays can use the display library member directly, a recombinant polypeptide produced from the nucleic acid encoding a displayed polypeptide, or a synthetic peptide synthesized based on the sequence of a displayed peptide. Exemplary assays for binding properties include the following.

**ELISA.** Polypeptides encoded by a display library can also be screened for a binding property using an ELISA assay. For example, each polypeptide is contacted to a microtitre plate whose bottom surface has been coated with the target, e.g., a limiting amount of the target. The plate is washed with buffer to remove non-specifically bound polypeptides. Then the amount of the polypeptide bound to the plate is determined by probing the plate with an antibody that can recognize the polypeptide, e.g., a tag or constant portion of the polypeptide. The antibody is linked to an enzyme such as alkaline phosphatase, which produces a colorimetric product when appropriate substrates are provided. The polypeptide can be purified from cells or assayed in a display library format, e.g., as a cell surface protein. In another version of the ELISA assay, each polypeptide of a diversity strand library is used to coat a different well of a microtitre plate. The ELISA then proceeds using a constant target molecule to query each well.

**Homogeneous Binding Assays.** The binding interaction of candidate polypeptide with a target can be analyzed using a homogenous assay, i.e., after all components of the assay are added, additional fluid manipulations are not required. For example, fluorescence resonance energy transfer (FRET) can be used as a homogenous assay (see, for example, Lakowicz et al., U.S. Patent No. 5,631,169; Stavrianopoulos, et al., U.S. Patent No. 4,868,103). A fluorophore label on the first molecule (e.g., the molecule identified in the fraction) is selected such that its emitted fluorescent energy can be absorbed by a fluorescent label on a second molecule (e.g., the target) if the second molecule is in proximity to the first molecule. The fluorescent label on the second molecule fluoresces when it absorbs to the transferred energy. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. A binding event that is configured for monitoring by FRET can be conveniently measured through standard fluorometric detection means well known in the art (e.g., using a fluorimeter). By titrating the amount of the first or second binding molecule, a binding curve can be generated to estimate the equilibrium binding constant.

Another example of a homogenous assay is Alpha Screen (Packard Bioscience, Meriden CT). Alpha Screen uses two labeled beads. One bead generates singlet oxygen when excited by a laser. The other bead generates a light signal when singlet oxygen diffuses from the first bead and collides with it. The signal is only generated when the two beads are in proximity. One bead can be attached to the display library member, the other to the target. Signals are measured to determine the extent of binding.

The homogenous assays can be performed while the candidate polypeptide is attached to the cell surface of the displaying cell.

**Cell Arrays.** In still another embodiment, a cell display library generated by recombination is formatted as a cellular array. Individual cells of the library or small pools are manipulated onto a grid and cultivated. A labeled target can be contacted to the grid to identify library members that bind the target. The cellular array can likewise be screened for any appropriate detectable activity.

U.S.S.N. 10/309,391 describes, among other things, methods of automation which can be used in conjunction with a method described herein.

### Exemplary Eukaryotic Display Vectors

A genetic vector useful for display of a multi-chain polypeptide on the surface of a eukaryotic cell can be as described below. The multi-chain polypeptide may be encoded in a single vector, or individual chains of the multi-chain polypeptide may be encoded in separate vectors. In one example, the vector may exist as a vector set, wherein each chain of a multi-chain polypeptide is encoded on one of a matched pair of vectors such that when the vector pair is present in a single eukaryotic cell, the chains of the multi-chain polypeptide associate and the biological activity of the multi-chain polypeptide is exhibited at the surface of the eukaryotic cell. In another example, the display vector may be a dual display vector, wherein the vector is capable of (i) expressing in a eukaryotic cell and displaying on the surface of a eukaryotic cell a biologically active multi-chain polypeptide, and (ii) expressing in a prokaryotic cell and displaying on the surface of a bacteriophage (or the prokaryotic cell) the biologically active multi-chain polypeptide.

The multi-chain polypeptide may be a polypeptide that includes two or more discrete polypeptide elements, referred to as chains of the multi-chain polypeptide, which chains are covalently or non-covalently linked (other than by peptide bonding) to form a biologically active polypeptide. For example, the multi-chain polypeptide encoded by the multi-chain display vector(s) can form a protein that includes two-, three-, or four polypeptide chains. The chains of the polypeptide may be the same (e. g., a homodimer, homotrimer, or homotetramer) or different (e. g., a heterodimer, heterotrimer, or heterotetramer). In one embodiment, the multi-chain polypeptide is a two-chain or four-chain polypeptide comprised of two different chains. For example, the multi-chain polypeptide is selected from a group of multi-chain polypeptides consisting of T cell receptors, MHC class I molecules, MHC class II molecules, immunoglobulins and biologically active immunoglobulin fragments (e. g., Fabs). The multi-chain polypeptide can be an IA, IgD, IgE, IgG, IgM, or biologically active fragment thereof. The multi-chain polypeptide can be a Fab fragment of an Ig, wherein the first polynucleotide of the multi-chain display vector comprises a segment that encodes the VH and CH domains of an Ig heavy chain, and a second polynucleotide comprises a segment that encodes an Ig light chain (i. e., VL and CL domains).

The chains of the multi-chain polypeptide (e. g., first chain, second chain, third chain, etc.) can be encoded as polynucleotides (e. g., first polynucleotide, second polynucleotide, third polynucleotide, etc. respectively) in the expression vector. As stated earlier, the polynucleotide sequences encoding the chains do not necessarily have to be inserted into the identical plasmid, or under the same gene expression control, in order to produce a functional multi-chain polypeptide. For example, the polynucleotide encoding the light chain and heavy chain of an Ig Fab may be located on separate plasmids and transformed as such into an identical host cell for co-expression and co-processing into a functional multi-chain polypeptide.

The sequences of the polynucleotides that encode the chains of a multi-chain polypeptide need not originate from the same source. For instance, an Ig molecule may be produced having variable domains (VH and VL) the same as those from a monoclonal antibody having a desired specificity, and constant domains (CH and CL) from a different monoclonal antibody having desired properties (e. g., to provide human compatibility or to provide a particular complement binding site). Moreover, the heterologous polynucleotide encoding the chains of a multi-chain polypeptide (e.g., Ig domains) may be variegated, to produce a family of polynucleotide homologues, encoding polypeptide chains that vary slightly in amino acid sequence from one another while having the same overall structure. In this way, when the homologues are incorporated into different host cells and expressed, a multiplicity of multi-chain polypeptides of varied (chain) sequence are displayed, providing a display library suitable for screening, e.g., to discover homologous multi-chain polypeptides having enhanced biological activity. Such alterations in amino acid sequence (or variegation) may be achieved by suitable mutation or partial synthesis and replacement or partial or complete substitution of appropriate regions of the corresponding polynucleotide coding sequences. Substitute constant domain portions may be obtained from compatible recombinant DNA sequences. Given proper selection of expression vector components and compatible host cells, the chains of the multi-chain polypeptide will be displayed on the surface of a eukaryotic host cell. This may be achieved, e.g., using any of a number of variable expression vector constructs. The display vector itself may be constructed or modified from any of a number of genetic vectors and genetic control elements known in the art and commercially available (e. g., from Invitrogen (Carlsbad, CA); Stratagene (La Jolla, CA); American Type Culture Collection (Manassas, VA)).

The vector construct typically is designed to express the polypeptide chains for effective transport and anchor of a fully assembled multi-chain polypeptide on the surface of a eukaryotic cell transformed with the vector(s) such that the biological activity of the multi-chain polypeptide is exhibited at the surface of the host cell.

To achieve effective cellular expression of the multi-chain polypeptide, the polynucleotides encoding each of the chains of the multi-chain polypeptide are, e.g., operatively linked to a transcriptional promoter to regulate expression of the polypeptide chains. The effective promoter must be functional in a eukaryotic system, and optionally (particularly in the case of a dual display vector) also effective as a prokaryotic promoter as well. In a dual display vector, the eukaryotic promoter(s) and the prokaryotic promoter(s) selected for regulating expression of the heterologous polypeptide chains of a multi-chain polypeptide may be the same or different promoters (so long as they are appropriately functional in the intended host organisms) or may be independently selected for the expression of each chain in a particular host. The eukaryotic promoter may be a constitutive promoter or an inducible promoter. A vector construct that utilizes the same promoter for each chain is preferred, in order to achieve balanced expression and to ensure simultaneous induction of expression. A number of eukaryotic promoters can be used. Exemplary eukaryotic promoters include yeast promoters, such as galactose inducible promoters, pGAL1, pGALI-10, pGa14, and pGal10; phosphoglycerate kinase promoter, pPGK, cytochrome c promoter, pCYC1; and alcohol dehydrogenase I promoter, pADH1. The T7 promoter can also be used in both prokaryotes and eukaryotes if the T7 RNA polymerase is expressed in the cell.

Each of the polynucleotides encoding a chain of a multi-chain polypeptide can also be also linked to a signal sequence (or a leader peptide sequence). The signal sequence operates to direct transport (sometimes referred to as secretion) of a nascent polypeptide into or across a cellular membrane. Chains of a multi-chain polypeptide expressed in a eukaryotic cell from a vector can be transported to the endoplasmic reticulum (ER) for assembly and transport to the cell surface for extracellular display. An effective signal sequence is functional in a eukaryotic system. Optionally (e.g., in the case of a dual display vector) the signal sequence is effective in a prokaryotic system as well. Polynucleotides encoding the chains of a multi-chain polypeptide are typically directly linked, in frame (either immediately adjacent to the polynucleotide or optionally linked via a linker or spacer sequence), to a signal sequence, thus generating a polypeptide chain signal peptide fusion protein. Preferably, each chain of a multi-chain polypeptide is fused to a separate signal peptide. The signal sequence encoding the signal peptide may be the same or different for each chain of the multi-chain polypeptide. The signal sequence may be native to the host or heterologous, so long as it is operable to effect extracellular transport of the polypeptide to which it is fused. Several signal sequences are known to persons skilled in the art (e. g., Mfα1 prepro, Mfα1 pre, acid phosphatase Pho5, Invertase SUC2 signal sequences operable in yeast; pIII, PelB, OmpA, PhoA signal sequences operable in *E. coli*; gp64 leader operable in insect cells; Igκ leader, honeybee melittin secretion signal sequences operable in mammalian cells). The signal sequences can be derived from native secretory proteins of the host cell. Other exemplary eukaryotic signal sequences include those of α-mating factor of yeast (e.g., of *S*. *cerevisiae*), α-agglutinin of yeast (e.g., of *S. cerevisiae*), invertase of yeast (e.g., of *S. cerevisiae*), inulinase of Kluyveromyces, and the signal peptide of the Aga2p subunit of a-agglutinin (e.g., of *S. cerevisiae*) (especially in embodiments where the anchoring polypeptide to be used is the Aga2p polypeptide).

For example, when the multi-chain polypeptide is a Fab, the first polynucleotide can include an Aga2p signal sequence in frame with a segment that encodes the VH and CH regions of an Ig heavy chain, and the second polynucleotide comprises an Aga2p signal sequence in frame with a segment that encodes an Ig light chain.

The multi-chain eukaryotic display vector can operate in a eukaryotic host cell such that the multi-chain polypeptide encoded by the vector(s) is displayed on the surface of the host cell. Immobilization ("tethering" or "display") on the surface of the host cell can be achieved, e.g., by linking at least one chain of the multi-chain polypeptide to a molecular moiety immobilized on the host cell wall or the host cell plasma membrane. The linkage can be covalent or non-covalent. Typically the linkage is covalent (e.g., a peptide, disulfide, amide, or other bond). More than one chain of a multi-chain polypeptide may be linked to an anchor. Generally, the fully assembled multi-chain polypeptide has only one point of attachment to the host cell surface. Although more than one attachment can be used, only one chain of the multi-chain polypeptide needs to be attached to the cell. Display on the surface of the cell can be achieved by linking at least one of the polypeptide chains to an anchor protein or functional fragment thereof. The effective anchor must be functional in a eukaryotic system, and optionally (particularly in the case of a dual display vector) the anchor needs to be effective as an anchor on the surface of a bacteriophage as well. The anchor can be a surface-expressed protein native to the host cell, e. g., either a transmembrane protein or a protein linked to the cell surface via a glycan bridge. Several operable anchor proteins can be used (e. g., ~111, pVI, pVII1, LamB, PhoE, Lpp-OmpA, Flagellin (FliC), or at least the transmembrane portions thereof, operable in prokaryotes/phage; platelet-derived growth factor receptor (PDGFR) transmembrane domain, glycosylphosphatidylinositol (GPI) anchors, operable in mammalian cells; gp64 anchor in insect cells, and the like). Where yeast is the host, the anchor protein can be α-agglutinin, a-agglutinin (having subcomponents Aga1p andAga2p), or FLO, which naturally form a linkage to the yeast cell surface.

Linkage of a polypeptide chain to an anchor may be achieved, directly or indirectly, by a variety of molecular biology techniques and also by the engineering of other indirect linkages, e.g., a disulfide bond, or a non-covalent binding interaction.

One exemplary method of chain-anchor linkage is through the construction of a chain:anchor fusion protein. A polynucleotide encoding a chain of a multi-chain polypeptide is directly linked, in frame (either immediately adjacent to the polynucleotide or optionally linked via a linker or spacer sequence) to an anchor, thus generating a polypeptide that includes a signal peptide and a chain:anchor fusion protein. Alternative modes of peptide-peptide linkage are know and available to achieve effective chain-anchor linkage. For example, a chain of the multi-chain polypeptide may be indirectly linked to an anchor via an intermediate association such as the high affinity interaction of the Jun and Fos leucine zippers c-jun/fos linkage) to covalently link a polypeptide chain to an anchor of a phage or host cell (e. g., see Crameri & Suter, 1993;Crameri & Blaser, 1996).

A multi-chain display vector can include cloning sites that facilitate transfer of the polynucleotide sequences that encode the chains of a multi-chain polypeptide. Such vector cloning sites can include at least one restriction endonuclease recognition site positioned to facilitate excision and insertion, in reading frame, of polynucleotides segments. Any of the restriction sites known in the art may be utilized in the vector construct; most commercially available vectors already contain multiple cloning sites or polylinkers. In addition, genetic engineering techniques useful to incorporate new and unique restriction sites into a vector are known and routinely practiced by persons of ordinary skill in the art.

A cloning site may involve as few as one restriction endonuclease recognition site to allow for the insertion or excision of a single polynucleotide fragment. More typically, two or more restriction sites are employed to provide greater control of, for example, insertion (e.g., direction of insert), and greater flexibility of operation (e.g., the directed transfer of more than one polynucleotide fragment).

In one exemplary vector construct disclosed in WO 03/029456, the multi-chain polypeptide is a Fab, the first polynucleotide comprises an Aga2p signal sequence in frame with a segment that encodes an Aga2p anchor, and in frame with a segment that encodes the VH and CH regions of an Ig heavy chain, wherein the Ig heavy chain region is bordered by unique restriction sites (e. g., *Sfi*I and *Not*I); and the second polynucleotide comprises an Aga2p signal sequence in frame with a segment that encodes an Ig light chain, wherein the Ig light chain region is bordered by unique restriction sites (e.g., *ApaL*I*,* and *Asc*I)*.*

In an exemplary multi-chain eukaryotic display vector, one or more of the chains of the multi-chain polypeptide expressed by the vector(s) in a host cell is linked to a molecular tag or reporter gene. The linkage can be achieved via a fusion protein that includes the polypeptide tag and the chain of the multi-chain polypeptide. Exemplary tags include epitope tags (e.g., tags generated by fusing peptide sequence that is recognized as an antigenic determinant to the polypeptide of interest) and tags that chelate a metal (e.g., polyHis tags). Examples of epitope tags include the HA tag and myc 12CA5 tag.

As described above, an exemplary vector construct for encoding a multi-chain polypeptide such as a Fab fragment of an immunoglobulin is exemplified as follows: the first polynucleotide comprises an Aga2p signal sequence in frame with a segment that encodes an Aga2p anchor, in frame with a segment that encodes the VH and CH regions of an Ig heavy chain, and in frame with a segment that encodes a myc tag, wherein the Ig heavy chain region is bordered by unique restriction sites (e. g., *Sfi*I and *Not*I); and the second polynucleotide comprises an Aga2p signal sequence in frame with a segment that encodes a HA tag, and in frame with a segment that encodes an Ig light chain, wherein the Ig light chain region is bordered by unique restriction sites (e. g., *ApaL*I, and *Asc*I).

### Example

The following is an example of a method for affinity maturing Fab antibodies. The method uses iterative combinatorial mating of LC and HC repertoires. These repertoires are diversified by *in vivo* recombination with diverse DNA fragments that include CDR coding sequences.

### Construction of a LC expression cassette in the chromosome of a Mat α haploid cell

Referring to FIG. 5, a LC expression cassette is constructed in the yeast chromosome of a haploid MAT α *S*. *cerevisiae* cell. A LC of a target specific lead antibody is cloned into the yeast display vector pTQ6 as a *Apa*L1/*Asc*1 fragment resulting is a LC driven by the expression of a inducible GAL1 promotor, fused at its N-terminus to a Aga2p signal sequence and appended with a HA epitope tag. The polarity of the expression cassette is: GAL1:Aga2p signal sequence (SS):LC:HA epitope tag:CYCtt transcriptional terminator sequence. A PCR fragment corresponding to this expression cassette is amplified using primers specific to vector sequences 5' adjacent to the GAL1 promoter and 3' adjacent to the CYCtt terminator sequence.

A *LEU2* marker gene is amplified by PCR from a host plasmid (for example pESC) and appended with a sequence at the 5' end which is homologous to the 3' end sequence adjacent to the CYCtt terminator sequence. The PCR product GAL1:SS: LC:HA: CYCtt and the LEU2 PCR product are mixed together and assembled using pull through primers specific to the 5' sequence adjacent to the GAL1 promoter and 3' adjacent to the Leu marker to give a PCR product of GAL1:SS:LC:HA:CYCtt: LEU2

Referring also to FIG. 5, the sequences appended to either end of the PCR product GAl1:SS:LC:HA: CYCtt: LEU2 contain two incompatible unique restriction sites for cloning into an appropriate yIP plasmid. The PCR product is digested with the appropriate restriction enzymes and cloned into a yIP plasmid. The yeast strain BJ5457 (Mat α) is then transformed with the yIP vector containing the LC expression construct and selection for integration into the yeast chromosome and the Leu+ phenotype. This procedure produces the haploid yeast strain BJ5457-LC.

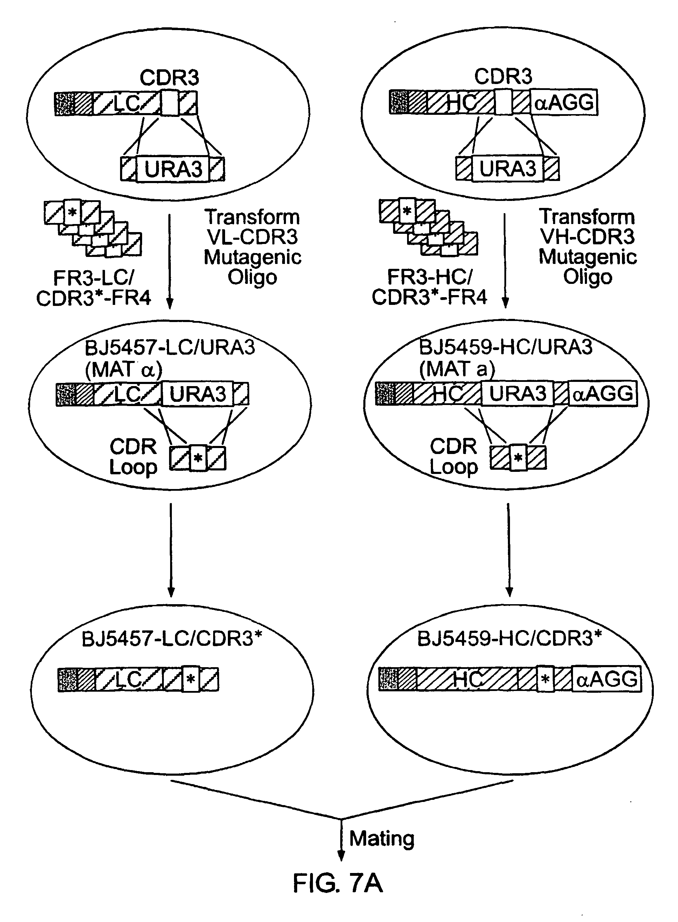

After successful construction of this strain, it is possible to replace the LC with a counter selectable marker such as URA3 to create a strain for direct cloning of antibody LC using *in vivo* recombination (see, top left, FIG. 7A). A LC expression strain can be constructed by amplification of the target LC with primers carrying a 5' adjacent sequence homologous to the SS:HA sequence and a 3' adjacent sequence to the CYCtt gene.

### Construction of a HC expression cassette in the chromosome of a MAT a haploid cell

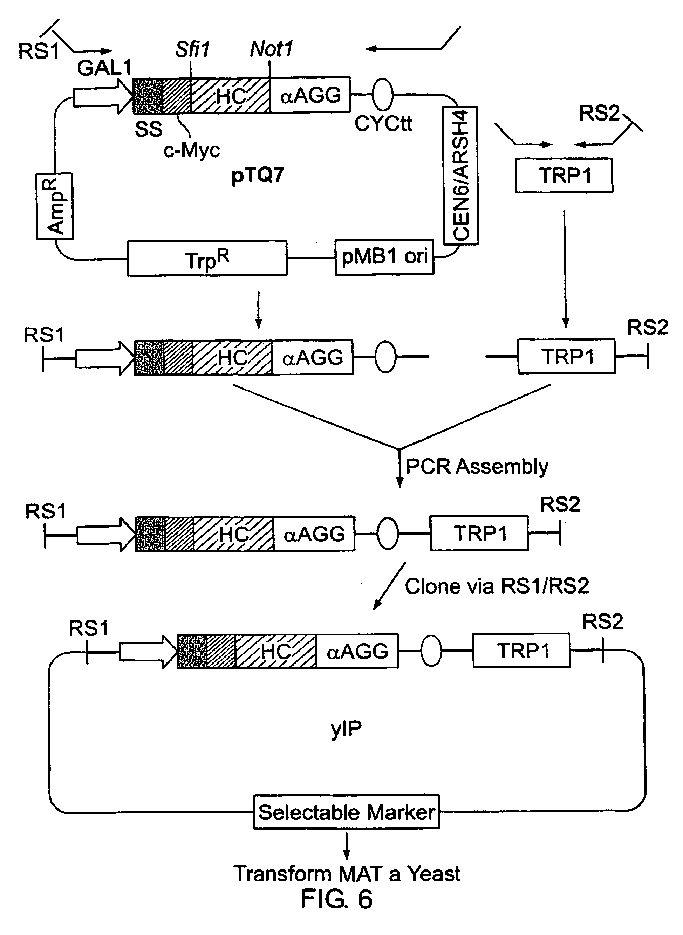

Referring also to FIG. 6, a HC expression cassette is constructed in the yeast chromosome of a haploid Mat a cell of *S. cerevisiae.* A HC of a target specific lead antibody is cloned into the yeast display vector pTQ7 as a *Sfi*1/*Not*1 fragment resulting is a HC driven by the expression of a inducible GAL1 promotor, fused at its N-terminus to the signal sequence alpha agglutinin yeast cell surface anchor protein (SS) and at its C- terminus to the alpha agglutinin yeast cell surface anchor gene (α AGG). The construct is further appended with a c-Myc epitope tag and a CYCtt transcriptional terminator sequence. The polarity of the expression cassette was GAL1:SS:HC:c-Myc:α AGG: CYCtt. A PCR fragment corresponding to this expression cassette was amplified using primers specific to vector sequences 5' adjacent to the GAL1 promotor and 3' adjacent to the CYCtt terminator sequence.

A *TRP1* marker gene is amplified by PCR from a host plasmid (for example pYD1) and appended with a sequence at the 5' end which is homologous to the 3' end sequence adjascent to the CYCtt terminator sequence. The PCR product GAL1:SS : HC: c-Myc:α AGG: CYCtt and the *TRP1* PCR product are mixed together and assembled using pull through primers specific to the 5' sequence adjacent to the GAL1 promoter and 3' adjacent to the *TRP1* marker to give a PCR product of GAL1 :SS:HC:c-Myc:α AGG:CYCtt : Trp

Sequences are appended to either end of the PCR product GAL1 promoter and 3' adjacent to the *TRP1* marker to produce a PCR product that is:
GAL1:SS:HC:c-Myc: α AGG: CYCtt :*TRP1*

This product contains two unique restriction sites for directional cloning into an appropriate yIP plasmid. The PCR product is digested with the appropriate restriction enzymes and cloned into a yIP plasmid. The yeast strain BJ5459 (Mat a) is then transformed with the yIP vector containing the HC expression construct and selection for integration into the yeast chromosome and the Trp + phenotype. This produced produces the haploid yeast strain BJ5459-HC-AGG (Fig 6).

After successful construction of this strain, it is possible to replace the HC with a counter selectable marker such as URA3 to create a strain for direct cloning of antibody HC using *in vivo* recombination. A HC expression strain can be constructed by amplification of the target HC with primers carrying a 5' adjacent sequence homologous to the SS:cMyc sequence and a 3' adjacent sequence to the αAGG gene.

### Targetted disruption of an antibody LC and HC with a counter-selectable marker

Haploid yeast are transformed with diversified oligonucleotides to diversify the CDR3 loop of the HC and the CDR3 loop of the LC. These oligonucleotides include the region of diversity (encoding CDR3) bracketed by sequences homologous to the framework region 3 (FR3) and framework region 4 (FR4) of the target LC and HC. These homologous sequences enable recombination into the chromosomal LC and HC expression cassettes. The counter-selectable markers URA3 or LYS2 are used to select for recombinants. They are introduced by targeted deletion of the VH-CDR3 or VL-CDR3 sequence. Subsequent replacement of the URA3 marker with recombined oligonucleotides is selected for by growth on 5-FOA (Fig 3). This counter selection reduces or eliminates the presence of wild-type antibody after recombination.

In order to create a targeted deletion of the LC-CDR3 sequence a URA3 cassette carrying the open reading frame of the URA3 gene is constructed bracketed by homologous sequences at its 5' end to the framework region 3 (FR3) and at its 3' end homologous to framework region 4 (FR4) of the antigen specific LC. The FR3-URA3-FR4 PCR product is constructed using PCR and oligonucleotides carrying homology to the *URA3* gene and appended with the FR3 and FR4 homologous sequences. The yeast strain harboring the LC expression cassette (BJ5457 - LC) is transformed with the FR3-URA3-FR4 PCR product and targeted deletion of the LC-CDR3 is selected for on Ura3- selective plates to give the yeast strain BJ5457-LC/URA3. To determine if recombination has occurred at the correct position in the yeast chromosome diagnostic PCR with primers specific for the *URA3* gene and the LC are used to confirm position of insertion.

FIG. 7A includes an illustration of a method for creating a targeted deletion of the HC-CDR3 sequence. A *URA3* cassette carrying the open reading frame of the *URA3* gene is constructed bracketed by homologous sequences at its 5' end to the framework region 3 (FR3) and at its 3' end homologous to framework region 4 (FR4) of the antigen specific HC. The FR3-URA3-FR4 PCR product is constructed using PCR and oligonucleotides carrying homology to the URA3 gene and appended with the FR3 and FR4 homologous sequences. The yeast strain harboring the HC expression cassette (BJ5459 - HC-αAGG) is transformed with the FR3-URA3-FR4 PCR product and targeted deletion of the HC-CDR3 is selected for on Ura3- selective plates to give the yeast strain BJ5459-HC/*URA3*. To determine if recombination has occurred at the correct position in the yeast chromosome diagnostic PCR with primers specific for the *URA3* gene and the LC are used to confirm position of insertion.

A representative number of clones are sequenced to determine the library size and the mutation frequency in the HC-CDR3 sequence.

### Construction of haploid LC-CDR3 and haploid HC-CDR3 repertoires diversified by in vivo recombination

A pool of oligonucleotides corresponding to the LC-CDR3 sequence of the target LC is constructed. The synthesis of the oligonucleotides is spiked so over the length of the CDR3 it is synthesized with a ratio of 90% wild-type nucleotide and 2.5% of each of A, C, G and T to give an oligonucleotide pool of FR3-LC/CDR3*-FR4. The region of diversity is bracketed by 20 bp sequences homologous to the FR3 and FR4 of the target LC. The yeast strain BJ5457-LC/URA3 is transformed with FR3-LC/CDR3*-FR4 and homologous recombination is selected for through the loss of the URA3 gene and replacement with a mutated FR3-LC/CDR3*-FR4 oligonucleotide. This creates a haploid Mat α repertoire BJ5457-LC/CDR3* in which the LC is diversified at the CDR3 loop using *in vivo* recombined mutagenic oligonucleotides. This repertoire can be about 10⁶ in size, or larger or smaller.

A pool of oligonucleotides corresponding to the HC-CDR3 sequence of the target HC is constructed. The synthesis of the oligonucleotides is spiked so over the length of the CDR3 it is synthesized with a ratio of 90% wild-type nucleotide and 2.5% of each of A, C, G and T to give an oligonucleotide pool of FR3-HC/CDR3*-FR4. The region of diversity is bracketed by 15 bp sequences homologous to the FR3 and FR4 of the target LC. The yeast strain BJ5457-LC/URA3 is transformed with FR3-HC/CDR3*-FR4 and homologous recombination is selected for on 5-FOA (5'fluoro-orotic acid; growth on 5-FOA requires loss of the *URA3* gene) and replacement with a mutated FR3-HC/CDR3*-FR4 oligonucleotide. This procedure creates a haploid Mat a repertoire BJ5457-HC/CDR3* in which the HC is diversified at the CDR3 loop using *in vivo* recombined mutagenic oligonucleotides. This repertoire can be about 10⁶ in size, or larger or smaller.

A representative number of clones are sequenced to determine the library size and the mutation frequency in the HC-CDR3 sequence.

### Combinatorial mating of BJ545 7-LC/CDR3 * and BJ545 7-HC/CDR3 * repertoires to create a Fab repertoire comprising antibodies with mutated LC-CDR3 and HC CDR3.

To produce a novel Fab (diploid) yeast display library two (haploid) host cell populations: a first population containing a repertoire of LC fragments diversified in the CDR3 loop and a second population containing a repertoire of HC fragments diversified in the CDR loop, are co-cultured under conditions sufficient to permit cellular fusion and the resulting diploid population grown under conditions sufficient to permit expression and display of the Fab (LC-CDR3*/HC-CDR3*) library.

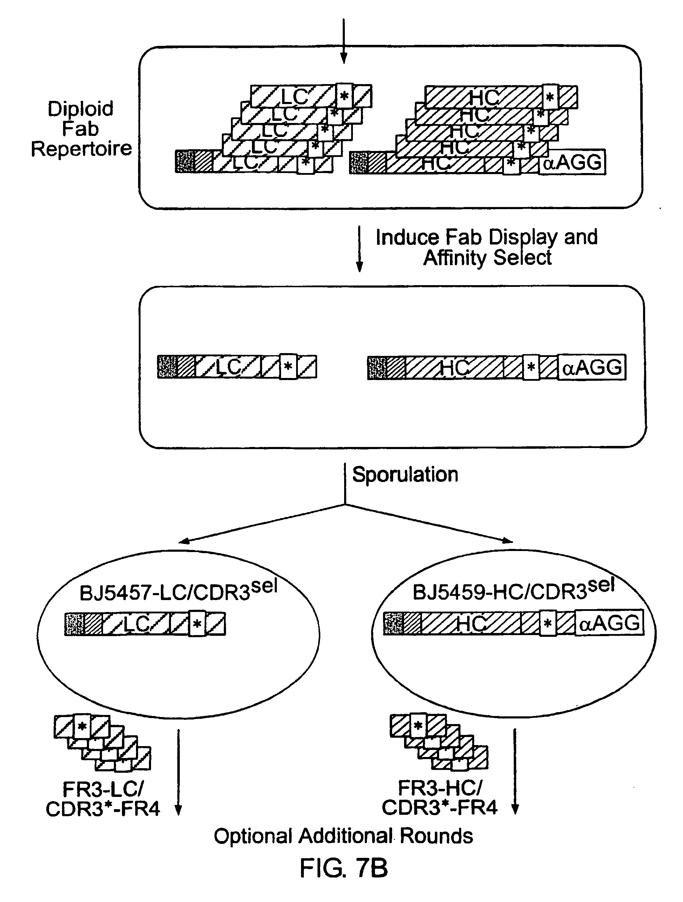

Approximately 10¹⁰ BJ5457-LC/CDR3* cells are mated with approximately 10¹⁰ BJ5457-HC/CDR3* yeast cells. 10% mating efficiency results in approximately 10⁹ diploid repertoire (thus capturing, for example, 10⁹ LC/HC combinations of 10¹² possible combinatorial LC/HC diversity, given the maximum starting diversity of the individual component LC and HC repertoires in the haploid parents) (Fig 7).

### Affinity selection of combinatorial [BJ5457-LC/CDR3*][BJ5457-HC/CDR3*] Fab displayed repertoire

The diploid repertoire is cultured and expression of LC and HC is induced. The diploid culture is then incubated with antigen and selected using a combination of magnetic bead based selection methods (e.g., MACS) for the first round of selection followed by flow cytometric sorting. The first round of MACS selection is performed with a excess of antigen to ensure all functional clones are enriched and this is followed by affinity driven selection at a concentration approximately 5 fold lower than the starting Kd of the target antibody to be affinity matured.

The affinity variants are screened for off rate using, for example, FACS to quantitate the affinity improvement after one or more rounds of combinatorial yeast mating and *in vivo* recombination.

### Resolution of selected diploid Fab into LC and HC haploid yeast cells and iterated gene diversification by in vivo recombination

Selected diploid cells containing affinity improved Fab antibodies are induced to sporulate by culturing the isolates under conditions of nitrogen starvation (Guthrie and Fink, 1991). Sporulated diploids are harvested, treated with zymolase, sonicated and plated on rich plates. Haploid colonies are subsequently transferred to selective plates to select either haploid HC cells (-Trp selection) or haploid LC cells (-Leu selection). This results in cells BJ5457-HC/CDR3^{sel} and BJ5457-LC/CDR3^{sel} which contain a HC and LC expression cassette with a recombined optimized CDR3 respectively. The haploid colonies harboring an improved LC-CDR3* or a improved HC-CDR3* are then subjected to a second round of diversification by *in vivo* recombination by one of several strategies:
(i) Transformation of haploid cells BJ5457-HC/CDR3^{sel} and BJ5457-LC/CDR3^{sel} with mutagenic oligonucleotides corresponding to FR3-HC/CDR3*-FR4 for HC diversification or FR3-LC/CDR3*-FR4 for LC diversification.
(ii) Targetted removal of LC-CDR1 loop and HC-CDR1 loop using a URA3 expression cassette bracketed by sequences homologous to the FR1 and FR2 of the target LC and HC respectively. This is followed by transformation with mutagenic oligos diversified in the CDR1 loop and bracketed by sequences homologous to the FR1 and FR2 of the LC or HC
(iii) A repeat targeted removal of LC-CDR3 loop and HC-CDR3 loop using a URA3 expression cassette bracketed by sequences homologous to the FR1 and FR2 of the target LC and HC respectively. This is followed by transformation with mutagenic oligos diversified in the CDR3 loop and bracketed by sequences homologous to the FR1 and FR2 of the LC or HC.

A second round of combinatorial yeast mating and affinity selection is then performed.

## Claims

1. A method of altering the sequence of an antibody protein, the method comprising:
providing yeast cells that each comprise a heterologous nucleic acid comprising a promoter and an operably linked coding region, wherein the coding region comprises nucleic acid homologous to a immunoglobulin variable domain encoding nucleic acid, wherein the coding region is a nonfunctional coding region prior to recombination;
introducing one or more Ig segment-coding nucleic acids into the yeast cells, wherein each of the one or more Ig segment-coding nucleic acids comprises a sequence encoding a segment of an immunoglobulin variable domain or a complement thereof;
maintaining the yeast cells under conditions that allow the introduced Ig segment-coding nucleic acids to recombine with the coding region, thereby producing a recombined nucleic acid in which the coding region encodes a polypeptide that includes an immunoglobulin variable domain, the variable domain being encoded, at least in part, by a sequence introduced by an Ig segment-coding nucleic acid; and
expressing the recombined nucleic acid such that the polypeptide that includes the immunoglobulin variable domain is displayed on a cell surface and is accessible to a probe.

2. The method of claim 1
(a) wherein the polypeptide expressed from the recombined nucleic acid comprises a sequence which enables an interaction with the cell such that recovery of the polypeptide results in recovery of the cell containing nucleic acid encoding the polypeptide, particularly wherein the sequence that enables interaction with the cell encodes an anchor domain that comprises a transmembrane domain or a domain that becomes GPI-linked to the yeast cell surface, wherein GPI stands for glycosylphosphatidylinositol;
(b) wherein the method further comprises fusing the yeast cells that include the recombined nucleic acid to other yeast cells that include a nucleic acid that encodes an immunoglobulin variable domain, compatible to the immunoglobulin variable domain encoded by the recombined nucleic acid so that the fused cells include a nucleic acid that encodes a polypeptide that includes an Ig LC and a polypeptide that includes an Ig HC, particularly wherein the fusing comprises yeast mating;
(c) wherein providing the yeast cells comprises introducing the one or more Ig segment-coding nucleic acids into the yeast cells, which include the heterologous nucleic acid;
(d) wherein providing the yeast cells comprises concurrent introducing, into the cells, the one or more Ig segment-coding nucleic acids and one or more nucleic acids homologous to regions of a immunoglobulin variable domain of the heterologous nucleic acid into the yeast cells, particularly wherein the antibody-coding nucleic acid sequences comprises a single-chain coding sequence that encodes a polypeptide that comprises a LC variable immunoglobulin domain and a HC variable immunoglobulin domain, or particularly wherein the antibody protein is a Fab;
(e) wherein the Ig segment-coding nucleic acids each comprise a sequence encoding a CDR of an immunoglobulin variable domain or a complement thereof;
(f) wherein the Ig segment-coding nucleic acids each contain a sequence encoding a single CDR of an immunoglobulin variable domain or a complement thereof, particularly wherein the one or more of the Ig segment-coding nucleic acids comprise nucleic acids encoding different variants of CDR1, CDR2 and CDR3 of a immunoglobulin light or heavy chain variable domain;
(g) wherein the one or more of the Ig segment-coding nucleic acids are obtained from human nucleic acids;
(h) wherein the one or more of the Ig segment-coding nucleic acids comprise a plurality of nucleic acids that encode different variants of the same CDR;
(i) wherein the one or more of the Ig segment-coding nucleic acids comprise a plurality of nucleic acids that encode different variants for a plurality of different CDRs;
(j) wherein each Ig segment-coding nucleic acid is less than 300 nucleotides in length;
(k) wherein introducing comprises contacting at least 10³ different Ig segment-coding nucleic acids to one or more of the yeast cells;
(l) wherein the one or more of the Ig segment-coding nucleic acids are single stranded;
(m) wherein the method further comprises, prior to the introducing, inserting a counter-selectable marker into the nucleic acid members of the cells of the subset, and, after the introducing, selecting cells in which the counter-selectable marker is replaced by an Ig segment coding nucleic acid;
(n) wherein the method further comprises, prior to the introducing, inserting a mutation into the coding region, wherein the mutation prevents expression of a functional immunoglobulin variable domain, particularly wherein the mutation comprises a stop codon, a marker gene, a frameshift, or a site of site-specific endonuclease; or
(o) wherein the method further comprises selecting one or more of the recombined nucleic acids for a criterion and repeating the method by providing further yeast cells, wherein the coding region of the further yeast cells is prepared from the one or more selected recombined nucleic acids.

3. A method of altering the sequence of an antibody protein, the method comprising:
providing a plurality of yeast cells, each cell of the plurality comprising antibody coding nucleic acid sequences that encode a unique antibody protein, wherein the antibody protein comprises a light chain variable immunoglobulin domain and a heavy chain variable immunoglobulin domain;
introducing one or more Ig segment-coding nucleic acids into one or more cells from the plurality, wherein each of the one or more Ig segment-coding nucleic acids comprises a sequence encoding a segment of an immunoglobulin variable domain or a complement thereof; and
maintaining the cell or cells from the plurality under conditions that allow the introduced Ig segment-coding nucleic acids to recombine with antibody-coding nucleic acid sequences of the cells of the subset, thereby producing one or a plurality of cells that can express on the cell surface an antibody protein having an altered immunoglobulin variable domain.

4. The method of claim 3
(a) wherein the plurality of the Ig segment-coding nucleic acids are introduced into cells of the plurality in parallel;
(b) wherein the plurality of the Ig segment-coding nucleic acids are introduced into cells of the plurality in the same reaction mixture;
(c) wherein the antibody-coding nucleic acid sequences comprises a LC coding sequence that encodes a polypeptide that comprises a LC variable immunoglobulin domain and a HC-coding sequence that encodes a polypeptide that comprises a HC variable immunoglobulin domain, particularly wherein the LC and HC coding sequences are integrated into different yeast chromosomes, or particularly wherein the yeast cell is a diploid cell, at least at the time of the selecting, and the LC coding sequence and the HC-coding sequence are integrated into loci on homologous chromosomes such that the LC coding sequence and the HC-coding sequence segregate into different spores when the diploid cell is sporulated, more particularly wherein the loci are linked to the MAT loci; or
(d) wherein the antibody-coding nucleic acid sequences are integrated into a yeast chromosome.

5. A method of selecting a cell that displays an antibody protein on the cell surface, the method comprising:
providing a plurality of yeast cells, the plurality comprising cells that each include antibody coding sequences that can encode an antibody protein that comprises a light chain variable immunoglobulin domain and a heavy chain variable immunoglobulin domain or that can recombine with Ig-segment encoding nucleic acids to form functional coding sequences that encode the antibody protein;
performing one or more cycles of:
(i) introducing nucleic acids that each comprise a segment encoding a CDR of an immunoglobulin variable domain or complement thereof into cells that include at least a part of the antibody coding sequences from cells of the subset;
(ii) maintaining the cells that contact the nucleic acid in (i) under conditions that allow the introduced nucleic acids to recombine with antibody coding sequences of the cells, thereby producing modified cells that include altered antibody coding sequences that have one or more altered immunoglobulin variable domains;
(iii) expressing the altered antibody coding sequences in the modified cells;
(iv) contacting the modified cells to the target; and
(v) selecting a further subset of cells from the modified cells, the further subset comprising one or more yeast cells that interact with the target.

6. The method of claim 5
(a) wherein at least two cycles are performed, particularly wherein the step (iii) of contacting comprises contacting the cells to the target under different conditions during different cycles, or particularly wherein the step (v) of selecting comprises requiring improved binding to the target relative to a previous selecting step;
(b) wherein, prior to the step (i) of introducing the nucleic acid, a marker sequence is inserted into the antibody-coding sequences;
(c) wherein the method further comprises recovering an antibody coding sequence from a cell of the further subset from one or more of the cycles;
(d) wherein the method further comprises sequencing at least a CDR-coding region of an antibody coding sequence in cell of the further subset from one or more of the cycles;
(e) wherein the nucleic acids are introduced into cells of the subset;
(f) wherein the method further comprises, in one or more of the cycles, sporulating cells of the subset prior to (i), and mating cells into which nucleic acids have been introduced after (ii);
(g) wherein the providing of a plurality of yeast cells comprises amplifying an original cell such that yeast cells of the plurality are substantially identical;
(h) wherein the providing of a plurality of yeast cells comprises amplifying a plurality of original cells wherein the original cells comprise antibody coding sequences for different antibodies that interact with the same target; or
(i) wherein the providing of a plurality of yeast cells comprises selecting one or more nucleic acids from a phage display library and reformatting one or more nucleic acids from a phage display system into a yeast expression system.

7. A method of varying subunits of an antibody protein displayed on a yeast cell surface, the method comprising:
providing a first haploid yeast cell that includes a first nucleic acid member encoding a first subunit of an antibody protein, the first subunit comprising a immunoglobulin variable domain;
introducing one or a plurality of nucleic acids that each comprise a segment encoding a CDR of the immunoglobulin variable domain or a complement thereof into the first haploid cell or clones thereof such that the introduced nucleic acid recombines with the first nucleic acid member in the first haploid cell or clones thereof, thereby producing one or more modified first haploid cells; and
mating the one or more modified first haploid cells to one or more second haploid cells to provide one or more diploid cells, wherein a second haploid yeast cell includes a second nucleic acid member encoding a second subunit of the antibody protein, the second subunit comprising a immunoglobulin variable domain complementary to the immunoglobulin variable domain of the first subunit, and each diploid cell can express, on its cell surface, an antibody protein comprising the first and second subunit,
particularly wherein the method further comprises, prior to the introducing, inserting a non-immunoglobulin sequence into the first nucleic acid member, thereby disrupting the coding of the first subunit, more particularly wherein the non-immunoglobulin coding sequence comprises a counter-selectable marker.

8. A method of varying subunits of an antibody protein displayed on a yeast cell surface, the method comprising:
providing a first haploid yeast cell that includes a first nucleic acid member that can encode a polypeptide comprising a first subunit of an antibody protein, the first subunit comprising a immunoglobulin variable domain, wherein the region of the nucleic acid member that encodes the immunoglobulin variable domain is disrupted;
introducing one or a plurality of nucleic acids that each comprise a segment encoding a CDR of the immunoglobulin variable domain or a complement thereof into the first haploid cell or clones thereof such that the introduced nucleic acid recombines with the first nucleic acid member in the first haploid cell or clones thereof, thereby producing one or more modified, first haploid cells; and
mating the one or more modified, first haploid cells to one or more second haploid cells to provide one or more diploid cells, wherein a second haploid yeast cell includes a second nucleic acid member encoding a polypeptide comprising a second subunit of the antibody protein, the second subunit comprising a immunoglobulin variable domain complementary to the immunoglobulin variable domain of the first subunit, and each diploid cell can express, on its cell surface, an antibody protein comprising the first and second subunit,
particularly wherein the method further comprises, prior to the mating, amplifying one or more of the modified, first haploid cells by one or more rounds of cell division.

9. A method of varying subunits of an antibody protein displayed on a yeast cell surface, the method comprising:
providing a first haploid yeast cell that includes a first nucleic acid member encoding a polypeptide comprising a first subunit of an antibody protein, the first subunit comprising a immunoglobulin light chain variable domain and a second haploid yeast cell that includes a second nucleic acid member encoding a polypeptide comprising a second subunit of the antibody protein, the second subunit comprising a immunoglobulin heavy chain variable domain;
introducing one or a plurality of nucleic acids that each comprise a segment encoding a CDR of an immunoglobulin variable light chain domain or a complement thereof into the first haploid cell or clones thereof such that the introduced nucleic acid recombines with the first nucleic acid member in the first haploid cell or clones thereof, thereby producing one or more modified first haploid cells;
introducing one or a plurality of nucleic acids that each comprise a segment encoding a CDR of an immunoglobulin variable heavy chain domain or a complement thereof into the second haploid cell or clones thereof such that the introduced nucleic acid recombines with the second nucleic acid member in the second haploid cell or clones thereof, thereby producing one or more modified second haploid cells; and
mating the one or more modified first haploid cells to the one or more modified second haploid cells to provide one or more diploid cells that each can express, on a cell surface, an antibody protein with a varied immunoglobulin light chain variable domain and a varied immunoglobulin heavy chain variable domain.

10. The method of claim 9 wherein the steps of providing the first and second haploid cell comprises:
providing a diploid yeast cell that includes (i) a first nucleic acid member encoding a first subunit of an antibody protein, the first subunit comprising a immunoglobulin light chain variable domain and (ii) a second nucleic acid member, encoding a second subunit of the heteroligomeric protein, the second subunit comprising a immunoglobulin heavy chain variable domain; and
sporulating the diploid yeast cell to provide the first haploid cell that contains the first nucleic acid member, but not the second nucleic acid member and the second haploid cell that contains the second nucleic acid member, but not the first nucleic acid member.

11. The method of claim 9 wherein the method further comprises
(a) prior to the mating, amplifying the one or more modified first or second haploid cells by one or more rounds of cell division,
(b) sporulating one or more of the diploid cells formed by the mating, or
(c) selecting a subset of the one or more of the diploid cells by contacting the one or more diploid cells, or clones thereof, to a target,
particularly
(i) wherein the subset comprises one or more of the cells that interact with the target,
(ii) wherein the subset comprises one or more of the cells that do not interact with the target, or
(iii) wherein the method further comprises sporulating cells of the subset.

12. A method of selecting a cell that displays a binding protein, the method comprising:
providing a plurality of diverse nucleic acids that include a protein coding sequence or a complement thereof;
providing a plurality of yeast cells, each cell of the plurality comprising a heterologous nucleic acid that comprises acceptor sequences that can recombine with homologous sequences present in one or more of the diverse nucleic acids, the heterologous nucleic acid comprising a nonfuctional immunoglobulin domain coding sequence that provides the acceptor sequences for recombination,
wherein recombination of the acceptor sequences and a diverse nucleic acid produces a recombined nucleic acid that encodes a polypeptide that includes a segment encoded by the diverse nucleic acid or complement thereof,
wherein the recombined nucleic acid includes a sequence encoding an immunoglobulin light chain variable domain or heavy chain variable domain;
introducing nucleic acids from the plurality of diverse nucleic acids into cells of the plurality of cells;
recombining one or more of the introduced nucleic acids with the heterologous nucleic acid in each cell, thereby producing recombined nucleic acids that each encodes a polypeptide that comprises a segment that is encoded by one or more of the introduced nucleic acids or a complement thereof;
expressing the recombined nucleic acids in the cells such that the polypeptides encoded by the recombined nucleic acids are associated with a surface of the cells;
contacting the cells that express the recombined nucleic acids to a target; and selecting one or more cells as a function of their interaction with the target.

13. The method of claim 12,
(a) wherein the nonfunctional immunoglobulin domain coding sequence comprises a stop codon prior to the 3' end of the immunoglobulin domain coding sequence, more particularly wherein the stop codon is in a region encoding a CDR,
(b) wherein the nonfunctional immunoglobulin domain coding sequence comprises a marker gene in a region encoding a CDR and sequences encoding FR regions of the immunoglobulin domain are intact, more particularly wherein the marker gene comprises a counter-selectable marker, or
(c) wherein the recombined nucleic acids encoded an antibody light chain, and the expressing further comprises expressing a nucleic acid encoding an antibody heavy chain that comprises a VH domain and CH1 domain and an anchor domain such that the antibody heavy and light chain associate and the anchor domain anchors the antibody heavy chain to the cell surface.

14. The method of claim 12 or 13
(a) wherein the heterologous nucleic acid comprises a segment encoding an anchor domain, particularly wherein the anchor domain comprises a transmembrane domain, or particularly wherein the anchor domain mediates a GPI-linkage;
(b) wherein the target is a protein;
(c) wherein the target is a mammalian cell; or
(d) wherein the recombining is enhanced by cleaving the heterologous nucleic acid in each cell, particularly wherein the cleaving creates a double-stranded break in the heterologous nucleic acid, or particularly wherein, prior to the recombining, a site recognized by a site-specific endonuclease that can be expressed in yeast cells is located between the acceptor sequences of the heterologous nucleic acid, and the recombining is enhanced by providing the site-specific endonuclease in each of the cells, more particularly wherein, the site-specific endonuclease is HO endonuclease and the providing comprises transcribing a gene encoding the HO endonuclease.

## Patentansprüche

1. Verfahren zum Ändern der Sequenz eines Antikörperproteins, wobei das Verfahren umfasst:
Bereitstellen von Hefezellen, von den jede eine heterologe Nukleinsäure umfasst, welche einen Promotor und eine funktionsfähig verbundene kodierende Region umfasst, wobei die kodierende Region Nukleinsäure umfasst, die homolog zu einer Nukleinsäure ist, die eine variable Domäne eines Immunglobulins kodiert, wobei die kodierende Region vor der Rekombination eine nicht funktionstüchtige kodierende Region ist;
Einführen von einer oder mehreren Ig-Segment-kodierenden Nukleinsäuren in die Hefenzellen, wobei jede der einen oder mehreren Ig-Segment-kodierenden Nukleinsäuren eine Sequenz umfasst, die ein Segment einer variablen Domäne eines Immunglobulins oder ein Komplement davon kodiert;
Halten der Hefezellen unter Bedingungen, die eine Rekombination der eingeführten Ig-Segment-kodierenden Nukleinsäuren mit der kodierenden Region erlauben, wodurch eine rekombinierte Nukleinsäure hergestellt wird, in der die kodierende Region ein Polypeptid kodiert, das eine variable Domäne eines Immunglobulins einschließt, wobei die variable Domäne zumindest zu einem Teil durch eine Sequenz kodiert wird, die durch eine Ig-Segment-kodierende Nukleinsäure eingeführt wurde; und
Exprimieren der rekombinierten Nukleinsäure in einer Weise, dass das Polypeptid, welches die variable Domäne eines Immunglobulins einschließt, auf einer Zelloberfläche präsentiert wird und einer Sonde zugänglich ist.

2. Verfahren nach Anspruch 1
(a) wobei das Polypeptid, das von der rekombinierten Nukleinsäure exprimiert wird, eine Sequenz umfasst, welche eine Wechselwirkung mit der Zelle ermöglicht, so dass die Gewinnung des Polypeptids in der Gewinnung der Zelle, die die das Polypeptid kodierende Nukleinsäure enthält, resultiert, insbesondere wobei die Sequenz, die eine Interaktion mit der Zelle möglich macht, eine Ankerdomäne kodiert, die eine Transmembrandomäne oder eine Domäne, die durch GPI mit der Hefezelloberfläche verbunden wird, umfasst, wobei GPI für Glycosylphosphatidylinositol steht;
(b) wobei das Verfahren weiterhin umfasst ein Verschmelzen der Hefezellen, die die rekombinierte Nukleinsäure einschließen, mit anderen Hefezellen, die eine Nukleinsäure einschließen, die eine variable Domäne eines Immunglobulins kodiert, die kompatibel ist zur variablen Domäne eines Immunglobulins, die durch die rekombinierte Nukleinsäure kodiert wird, so dass die verschmolzenen Zellen eine Nukleinsäure einschließen, die ein Polypeptid kodiert, das eine Ig-LC und ein Polypeptid einschließt, das eine Ig-HC einschließt, insbesondere wobei das Verschmelzen ein Verpaaren der Hefe umfasst;
(c) wobei das Bereitstellen der Hefezellen das Einführen der einen oder mehreren Ig-Segment-kodierenden Nukleinsäuren in die Hefezellen, die die heterologe Nukleinsäure einschließen, umfasst;
(d) wobei das Bereitstellen der Hefezellen gleichzeitig umfasst ein Einführen, in die Zellen, von einer oder mehreren Ig-Segment-kodierenden Nukleinsäuren und einer oder mehreren Nukleinsäuren, die homolog zu Regionen einer variablen Domäne eines Immunglobulins der heterologen Nukleinsäure ist, in die Hefezellen, insbesondere wobei die Antikörper-kodierenden Nukleinsäuresequenzen eine einzelkettige kodierende Sequenz umfassen, die ein Polypeptid kodiert, das eine LC- variable Immunglobulindomäne und eine HCvariable Immunglobulindomäne umfasst, oder insbesondere wobei das Antikörperprotein ein Fab ist;
(e) wobei die Ig-Segment-kodierenden Nukleinsäuren jeweils eine Sequenz umfassen, die eine CDR einer variablen Domäne eines Immunglobulins oder eines Komplements davon kodieren;
(f) wobei die Ig-Segment-kodierenden Nukleinsäuren jeweils eine Sequenz enthalten, die eine einzelne CDR einer variablen Domäne eines Immunglobulins oder eines Komplements davon kodiert, insbesondere wobei die eine oder mehrere der Ig-Segment-kodierenden Nukleinsäuren solche Nukleinsäuren umfassen, die verschieden Varianten von CDR1, CDR2 und CDR3 einer variablen Domäne einer leichten oder schweren Kette eines Immunglobulins kodieren;
(g) wobei die eine oder mehreren der Ig-Segment-kodierenden Nukleinsäuren aus menschlichen Nukleinsäuren erhalten werden;
(h) wobei die eine oder mehreren der Ig-Segment-kodierenden Nukleinsäuren eine Vielzahl von Nukleinsäuren umfassen, die verschiedene Varianten der gleichen CDR kodieren;
(i) wobei die eine oder mehreren der Ig-Segment-kodierenden Nukleinsäuren eine Vielzahl von Nukleinsäuren umfassen, die verschiedene Varianten einer Vielzahl von verschiedenen CDRs kodieren;
(j) wobei jede Ig-Segment-kodierende Nukleinsäure weniger als 300 Nukleotide lang ist;
(k) wobei das Einführen ein Inkontaktbringen von mindestens 10³ verschiedener Ig-Segment-kodierender Nukleinsäuren mit einer oder mehreren der Hefezellen umfasst;
(l) wobei die eine oder mehreren der Ig-Segment-kodierenden Nukleinsäuren einzelsträngig ist;
(m) wobei das Verfahren weiterhin vor dem Einführen das Insertieren eines gegenselektierbaren Markers in die Nukleinsäuremitglieder der Zellen der Untergruppe, und nach dem Einführen ein Selektionieren von Zellen umfasst, in denen der gegenselektierbare Marker durch eine Ig-Segment-kodierende Nukleinsäure ersetzt wurde;
(n) wobei das Verfahren weiterhin vor dem Einführen das Insertieren einer Mutation in die kodierende Region umfasst, wobei die Mutation die Expression einer funktionstüchtigen variablen Domäne eines Immunglobulins verhindert, insbesondere wobei die Mutation ein Stoppkodon, ein Markergen, eine Verschiebung des Leserahmens, oder eine Schnittstelle einer ortsspezifischen Endonuklease umfasst; oder
(o) wobei das Verfahren weiterhin das Selektionieren einer oder mehrerer der rekombinierten Nukleinsäuren nach einem Kriterium und ein Wiederholen des Verfahrens durch Bereitstellen weiterer Hefezellen umfasst, wobei die kodierende Region der weiteren Hefezellen aus der einen oder den mehreren selektionierten rekombinierten Nukleinsäuren hergestellt wird.

3. Verfahren zum Verändern der Sequenz eines Antikörperproteins, wobei das Verfahren umfasst:
Bereitstellen einer Vielzahl von Hefezellen, wobei jede Zelle der Vielzahl Antikörper-kodierende Nukleinsäuresequenzen umfasst, die ein einzigartiges Antikörperprotein kodieren, wobei das Antikörperprotein eine variable Immunglobulindomäne einer leichten Kette und eine variable Immunglobulindomäne einer schweren Kette umfasst;
Einführen von einer oder mehreren Ig-Segment-kodierenden Nukleinsäuren in eine oder mehrere Zellen der Vielzahl, wobei jede der einen oder mehreren Ig-Segment-kodierenden Nukleinsäuren eine Sequenz umfasst, die ein Segment einer variablen Domäne eines Immunglobulins oder eines Komplements davon kodiert; und
Halten der Zelle oder Zellen der Vielzahl unter Bedingungen, die ein Rekombinieren der eingeführten Ig-Segment-kodierenden Nukleinsäuren mit Antikörper-kodierenden Nukleinsäuresequenzen der Zellen der Untergruppe erlauben, wodurch eine oder eine Vielzahl von Zellen hergestellt wird, die auf der Zelloberfläche ein Antikörperprotein exprimieren können, das eine veränderte variable Domäne eines Immunglobulins besitzt.

4. Verfahren nach Anspruch 3
(a) wobei die Vielzahl der Ig-Segment-kodierenden Nukleinsäuren in Zellen der Vielzahl in einer parallelen Weise eingeführt wird;
(b) wobei die Vielzahl der Ig-Segment-kodierenden Nukleinsäuren in Zellen der Vielzahl in der gleichen Reaktionsmischung eingeführt wird;
(c) wobei die Antikörper-kodierenden Nukleinsäuresequenzen eine LC- e) kodierende Sequenz umfassen, die ein Polypeptid kodiert, das eine variable LC-Immunglobulindomäne und eine HC- kodierende Sequenz umfasst, die ein Polypeptid kodiert, das eine variable HC-Immunglobulindomäne umfasst, insbesondere wobei die LC- und HC- kodierenden Sequenzen in verschiedene Hefechromosomen integriert sind, oder insbesondere wobei die Hefezelle eine diploide Zelle ist, zumindest zur Zeit des Selektionierens, und die LC-kodierende Sequenz und die HC-kodierende Sequenz in einer Weise in verschiedene Loci auf homologen Chromosomen integriert sind, so dass die LC-kodierende Sequenz und die HC-kodierende Sequenz sich in verschiedene Sporen auftrennen, wenn die diploide Zelle sporuliert wird, weiter insbesondere wobei die Loci mit den MAT-Loci verbunden sind; oder
(d) wobei die Antikörper-kodierenden Nukleinsäuresequenzen in ein Hefechromosom integriert sind.

5. Verfahren zum Selektionieren einer Zelle, die ein Antikörperprotein auf der Zelloberfläche präsentiert, wobei das Verfahren umfasst:
Bereitstellen einer Vielzahl von Hefezellen, wobei die Vielzahl Zellen umfasst, die jeweils Antikörper-kodierende Sequenzen einschließen, die ein Antikörperprotein kodieren können, das eine variable Immunglobulindomäne einer leichten Kette und eine variable Immunglobulindomäne einer schweren Kette umfasst, oder die mit Ig-Segment-kodierenden Nukleinsäuren rekombinieren können, um funktionstüchtige kodierende Sequenzen zu bilden, die das Antikörperprotein kodieren;
Durchführen von einem oder mehreren Zyklen von:
(i) Einführen von Nukleinsäuren, die jeweils ein Segment umfassen, das eine CDR einer variablen Domäne eines Immunglobulins oder eines Komplements davon kodiert, in Zellen, die mindestens einen Teil der Antikörper-kodierenden Sequenzen von Zellen der Untergruppe einschließen;
(ii) Halten der Zellen, die die Nukleinsäure in (i) in Kontakt halten, unter Bedingungen, die ein Rekombinieren der eingeführten Nukleinsäuren mit Antikörper-kodierenden Sequenzen der Zellen erlauben, wodurch veränderte Zellen hergestellt werden, die veränderte Antikörper-kodierende Sequenzen einschließen, die eine oder mehrere veränderte variable Domänen eines Immunglobulins besitzen;
(iii) Exprimieren der veränderten Antikörper-kodierenden Sequenzen in den veränderten Zellen;
(iv) Inkontaktbringen der veränderten Zellen mit dem Ziel; und
(v) Selektionieren einer weiteren Untergruppe von Zellen aus den veränderten Zellen, wobei die weitere Untergruppe eine oder mehrere Hefezellen umfasst, die mit dem Ziel wechselwirken.

6. Verfahren nach Anspruch 5
(a) wobei mindestens zwei Zyklen durchgeführt werden, insbesondere wobei der Schritt (iii) des Inkontaktbringens ein Inkontaktbringen der Zellen mit dem Ziel unter verschiedenen Bedingungen während verschiedener Zyklen umfasst, oder insbesondere wobei der Schritt (v) des Selektionierens umfasst, dass eine verbesserte Bindung an das Ziel verglichen mit einem vorherigen Selektionsschritt verlangt wird;
(b) wobei vor dem Schritt (i) des Einführens der Nukleinsäure eine Markersequenz in die Antikörper-kodierenden Sequenzen insertiert wird;
(c) wobei das Verfahren weiterhin das Gewinnen einer Antikörper-kodierenden Sequenz aus einer Zelle der weiteren Untergruppe aus einem oder mehreren der Zyklen umfasst;
(d) wobei das Verfahren weiterhin das Sequenzieren von mindestens einer CDRkodierenden Region einer antikörperkodierenden Sequenz in einer Zelle der weiteren Untergruppe aus einem oder mehreren der Zyklen umfasst;
(e) wobei die Nukleinsäuren in Zellen der Untergruppe eingeführt werden;
(f) wobei das Verfahren weiterhin in einem oder mehreren der Zyklen ein Sporulieren der Zellen der Untergruppe vor (i) umfasst, und ein Verpaaren der Zellen, in welche Nukleinsäuren nach (ii) eingeführt wurden;
(g) wobei das Bereitstellen einer Vielzahl von Hefezellen das Amplifizieren einer ursprünglichen Zelle umfasst, so dass die Hefezellen der Vielzahl im Wesentlichen identisch sind;
(h) wobei das Bereitstellen einer Vielzahl von Hefezellen ein Amplifizieren einer Vielzahl von ursprünglichen Zellen umfasst, wobei die ursprünglichen Zellen Antikörper-kodierende Sequenzen für verschiedene Antikörper umfassen, die mit dem gleichen Ziel wechselwirken; oder
(i) wobei das Bereitstellen einer Vielzahl von Hefezellen das Selektionieren von einer oder mehreren Nukleinsäuren aus einer Phagenpräsentationsbibliothek umfasst, und das Umbilden von einer oder mehreren Nukleinsäuren aus einem Phagenpräsentationssystem in ein Hefeexpressionssystem.

7. Verfahren zum Verändern von Untereinheiten eines Antikörperproteins, das auf einer Hefezelloberfläche präsentiert wird, wobei das Verfahren umfasst:
Bereitstellen einer ersten haploiden Hefezelle, die ein erstes Nukleinsäuremitglied umfasst, die eine erste Untereinheit eines Antikörperproteins kodiert, wobei die erste Untereinheit eine variable Domäne eines Immunglobulins umfasst;
Einführen von einer oder einer Vielzahl von Nukleinsäuren, die jeweils ein Segment umfassen, das eine CDR der variablen Domäne eines Immunglobulins oder eines Komplements davon kodiert, in die erste haploide Zelle oder Klone von ihr, so dass die eingeführte Nukleinsäure mit dem ersten Nukleinsäuremitglied in der ersten haploiden Zelle oder Klonen von ihr rekombiniert, wodurch eine oder mehrere veränderte erste haploide Zellen hergestellt werden; und
Verpaaren der einen oder mehreren veränderten ersten haploiden Zellen mit einer oder mehreren zweiten haploiden Zellen, um eine oder mehrere diploide Zellen bereitzustellen, wobei eine zweite haploide Hefezelle ein zweites Nukleinsäuremitglied einschließt, das eine zweite Untereinheit des Antikörperproteins kodiert, wobei die zweite Untereinheit eine variable Domäne eines Immunglobulins umfasst, die komplementär zur variablen Domäne eines Immunglobulins der ersten Untereinheit ist, und wobei jede diploide Zelle auf ihrer Zelloberfläche ein Antikörperprotein exprimieren kann, das die erste und zweite Untereinheit umfasst,
insbesondere wobei das Verfahren weiterhin vor dem Einführen das Insertieren einer nicht-Immunglobulinsequenz in das erste Nukleinsäuremitglied umfasst, wodurch das Kodieren der ersten Untereinheit unterbrochen wird, weiter insbesondere wobei die nicht-Immunglobulin kodierende Sequenz einen gegenselektierbaren Marker umfasst.

8. Verfahren zum Variieren von Untereinheiten eines Antikörperproteins, das auf einer Hefezelloberfläche präsentiert wird, wobei das Verfahren umfasst:
Bereitstellen einer ersten haploiden Hefezelle, die ein erstes Nukleinsäuremitglied einschließt, das ein Polypeptid kodieren kann, das eine erste Untereinheit eines Antikörperproteins umfasst, wobei die erste Untereinheit eine variable Domäne eines Immunglobulins umfasst, wobei die Region des Nukleinsäuremitglieds, die die variable Domäne eines Immunglobulins kodiert, unterbrochen ist;
Einführen einer oder einer Vielzahl von Nukleinsäuren, die jede ein Segment umfasst, das eine CDR einer variablen Domäne eines Immunglobulins oder ein Komplement davon kodiert, in die erste haploide Zelle oder Klone von ihr, so dass die eingeführte Nukleinsäure mit dem ersten Nukleinsäuremitglied in der ersten haploiden Zelle oder Klonen von ihr rekombiniert, wodurch eine oder mehrere veränderte erste haploide Zellen hergestellt werden; und
Verpaaren der einen oder mehreren veränderten ersten haploiden Zellen mit einer oder mehreren zweiten haploiden Zellen, um eine oder mehrere diploide Zellen bereitzustellen, wobei eine zweite haploide Hefezelle ein zweites Nukleinsäuremitglied einschließt, das ein Polypeptid kodiert, das eine zweite Untereinheit des Antikörperproteins umfasst, wobei die zweite Untereinheit eine variable Domäne eines Immunglobulins umfasst, die komplementär zur variablen Domäne eines Immunglobulins der ersten Untereinheit ist, und wobei jede diploide Zelle auf ihrer Zelloberfläche ein Antikörperprotein exprimieren kann, das die erste und zweite Untereinheit umfasst,
insbesondere wobei das Verfahren weiterhin vor dem Verpaaren das Amplifizieren einer oder mehrerer der veränderten ersten haploiden Zellen durch eine oder mehrere Runden von Zellteilung umfasst.

9. Verfahren zum Variieren von Untereinheiten eines Antikörperproteins, das auf einer Hefezelloberfläche präsentiert wird, wobei das Verfahren umfasst:
Bereitstellen einer ersten haploiden Hefezelle, die ein erstes Nukleinsäuremitglied einschließt, das ein Polypeptid kodiert, das eine erste Untereinheit eines Antikörperproteins umfasst, wobei die erste Untereinheit eine variable Domäne einer leichten Kette eines Immunglobulins umfasst und eine zweite haploide Hefezelle, die ein zweites Nukleinsäuremitglied einschließt, welches ein Polypeptid kodiert, das eine zweite Untereinheit des Antikörperproteins umfasst, wobei die zweite Untereinheit eine variable Domäne einer schweren Kette eines Immunglobulins umfasst;
Einführen von einer oder einer Vielzahl von Nukleinsäuren, die jeweils eine Segment umfassen, welches eine CDR einer variablen Domäne einer leichten Kette eines Immunglobulins oder eines Komplements davon kodiert, in die erste haploide Zelle oder Klone von ihr, so dass die eingeführte Nukleinsäure mit dem ersten Nukleinsäuremitglied in der ersten haploiden Zelle oder Klonen von ihr rekombiniert, und **dadurch** eine oder mehrere modifizierte erste haploide Zellen hergestellt werden;
Einführen von einer oder einer Vielzahl von Nukleinsäuren, die jeweils eine Segment umfassen, welches eine CDR einer variablen Domäne einer schweren Kette eines Immunglobulins oder eines Komplements davon kodiert, in die zweite haploide Zelle oder Klone von ihr, so dass die eingeführte Nukleinsäure mit dem zweiten Nukleinsäuremitglied in der zweiten haploiden Zelle oder Klonen von ihr rekombiniert, wodurch eine oder mehrere modifizierte zweite haploide Zellen hergestellt werden; und
Verpaaren der einen oder mehreren modifizierten ersten haploiden Zellen mit der einen oder mehreren modifizierten zweiten haploiden Zellen, um eine oder mehrere diploide Zellen bereitzustellen, von denen jede auf einer Zelloberfläche ein Antikörperprotein mit einer variierten variablen Domäne einer leichten Kette eines Immunglobulins und einer variierten variablen Domäne einer schweren Kette eines Immunglobulins exprimieren kann.

10. Verfahren nach Anspruch 9, wobei die Schritte des Bereitstellens der ersten und zweiten haploiden Zelle umfassen:
Bereitstellen einer diploiden Hefezelle, die einschließt (i) ein erstes Nukleinsäuremitglied, das eine erste Untereinheit eines Antikörperproteins kodiert, wobei die erste Untereinheit eine variable Domäne einer leichten Kette eines Immunglobulins umfasst, und (ii) ein zweites Nukleinsäuremitglied, das eine zweite Untereinheit des heterooligomeren Proteins kodiert, wobei die zweite Untereinheit eine variable Domäne einer schweren Kette eines Immunglobulins umfasst; und
Sporulieren der diploiden Hefezelle, um die erste haploide Zelle bereitzustellen, die das erste Nukleinsäuremitglied enthält, aber nicht das zweite Nukleinsäuremitglied, und die zweite haploide Zelle, die das zweite Nukleinsäuremitglied, aber nicht das erste Nukleinsäuremitglied enthält.

11. Verfahren nach Anspruch 9, wobei das Verfahren weiterhin umfasst
(a) vor dem Verpaaren, Amplifizieren der einen oder mehreren modifizierten ersten oder zweiten haploiden Zellen durch eine oder mehrere Runden von Zellteilung,
(b) Sporulieren von einer oder mehreren der diploiden Zellen, die durch das Verpaaren gebildet werden, oder
(c) Selektionieren einer Untergruppe der einen oder mehreren der diploiden Zellen durch Inkontaktbringen der einen oder mehreren diploiden Zellen oder Klonen von ihr mit einem Ziel,
insbesondere
(i) wobei die Untergruppe eine oder mehrere der Zellen umfasst, die mit dem Ziel wechselwirken,
(ii) wobei die Untergruppe eine oder mehrere der Zellen umfasst, die nicht mit dem Ziel wechselwirken, oder
(iii) wobei das Verfahren weiterhin das Sporulieren von Zellen der Untergruppe umfasst.

12. Verfahren zum Selektionieren einer Zelle, die ein Bindeprotein präsentiert, wobei das Verfahren umfasst:
Bereitstellen einer Vielzahl von verschiedenen Nukleinsäuren, die eine Proteinkodierende Sequenz oder ein Komplement davon einschließen;
Bereitstellen einer Vielzahl von Hefezellen, wobei jede Zelle der Vielzahl eine heterologe Nukleinsäure umfasst, die Akzeptorsequenzen umfasst, die mit homologen Sequenzen rekombinieren können, die in einer oder mehrerer der verschiedenen Nukleinsäuren vorhanden sind, wobei die heterologe Nukleinsäure eine nicht funktionstüchtige kodierende Sequenz einer Immunglobulindomäne umfasst, die die Akzeptorsequenzen für die Rekombination bereitstellt,
wobei eine Rekombination der Akzeptorsequenzen und einer verschiedenen Nukleinsäure eine rekombinierte Nukleinsäure herstellt, die ein Polypeptid kodiert, das ein Segment einschließt, das durch die verschiedene Nukleinsäure oder einem Komplement davon kodiert wird,
wobei die rekombinierte Nukleinsäure eine Sequenz einschließt, die eine variable Domäne einer leichten Kette oder eine variable Domäne einer schwere Kette eines Immunglobulins kodiert;
Einführen von Nukleinsäuren aus der Vielzahl der verschiedenen Nukleinsäuren in Zellen der Vielzahl von Zellen;
Rekombinieren von einer oder mehreren der eingeführten Nukleinsäuren mit der heterologen Nukleinsäure in jeder Zelle, wodurch rekombinierte Nukleinsäuren hergestellt werden, die jeweils ein Polypeptid kodieren, das ein Segment umfasst, das durch eine oder mehrere der eingeführten Nukleinsäuren oder einem Komplement davon kodiert wird;
Exprimieren der rekombinierten Nukleinsäuren in den Zellen, so dass die Polypeptide, die durch die rekombinierten Nukleinsäuren kodiert werden, mit einer Oberfläche der Zellen assoziiert sind;
Inkontaktbringen der Zellen, die die rekombinierten Nukleinsäuren exprimieren, mit einem Ziel; und
Selektionieren einer oder mehrerer Zellen als eine Funktion ihrer Wechselwirkung mit dem Ziel.

13. Verfahren nach Anspruch 12,
(a) wobei die nicht funktionstüchtige kodierende Sequenz einer Immunglobulindomäne ein Stoppkodon vor dem 3'-Ende der kodierenden Sequenz einer Immunglobulindomäne umfasst, weiter insbesondere wobei das Stoppkodon in einer Region liegt, die eine CDR kodiert,
(b) wobei die nicht funktionstüchtige kodierende Sequenz einer Immunglobulindomäne ein Markergen in einer Region, die eine CDR kodiert, umfasst, und die Sequenzen, die FR-Regionen der Immunglobulindomäne kodieren, intakt sind, weiter insbesondere wobei das Markergen einen gegenselektierbaren Marker umfasst, oder
(c) wobei die rekombinierten Nukleinsäuren eine leichte Kette eines Antikörpers kodieren, und weiterhin das Exprimieren einer Nukleinsäure umfasst, welche eine schwere Kette eines Antikörpers kodiert, die eine VH-Domäne und eine CH1-Domäne und eine Ankerdomäne umfasst, so dass die schwere und leichte Kette des Antikörpers assoziieren, und die Ankerdomäne die schwere Kette des Antikörpers auf die Zelloberfläche verankert.

14. Verfahren nach Anspruch 12 oder 13
(a) wobei die heterologe Nukleinsäure ein Segment umfasst, das eine Ankerdomäne kodiert, insbesondere wobei die Ankerdomäne eine Transmembrandomäne umfasst, oder insbesondere wobei die Ankerdomäne eine GPI-Verknüpfung vermittelt;
(b) wobei das Ziel ein Protein ist;
(c) wobei das Ziel eine Säugerzelle ist; oder
(d) wobei das Rekombinieren durch Spalten der heterologen Nukleinsäure in jeder Zelle verstärkt wird, insbesondere wobei das Spalten einen doppelsträngigen Bruch in der heterologen Nukleinsäure erschafft, oder insbesondere wobei vor dem Rekombinieren eine Schnittstelle, der durch eine ortspezifische Endonuklease erkannt wird, die in Hefezellen exprimiert werden kann, sich zwischen den Akzeptorsequenzen der heterologen Nukleinsäure befindet, und das Rekombinieren durch Bereitstellen der ortsspezifischen Endonuklease in jeder der Zellen verstärkt wird, weiter insbesondere wobei die ortsspezifische Endonuklease HO-Endonuklease ist, und das Bereitstellen das Transkribieren eines Gens, welches die HO-Endonuklease kodiert, umfasst.

## Revendications

1. Procédé de modification de la séquence d'une protéine d'anticorps, le procédé comprenant :
l'apport de cellules de levure qui comprennent chacune un acide nucléique hétérologue comprenant un promoteur et une région codante liée de façon opérationnelle, où la région codante comprend un acide nucléique homologue à un acide nucléique codant pour un domaine variable d'immunoglobuline, où la région codante est une région codante non fonctionnelle avant la recombinaison ;
l'introduction d'un ou plusieurs acides nucléiques codant pour un ou des segment(s) d'Ig dans les cellules de levure, où l'acide nucléique ou chacun des acides nucléiques codant pour un ou des segment(s) d'Ig comprend une séquence codant pour un segment d'un domaine variable d'immunoglobuline ou un complément de celle-ci ;
le maintien des cellules de levure dans des conditions qui permettent aux acides nucléiques introduits codant pour un ou des segment(s) d'Ig de se recombiner avec la région codante, produisant ainsi un acide nucléique recombiné dans lequel la région codante code pour un polypeptide qui inclut un domaine variable d'immunoglobuline, le domaine variable étant codé, au moins en partie, par une séquence introduite par un acide nucléique codant pour un ou des segment(s) d'Ig; et
l'expression de l'acide nucléique recombiné de façon telle que le polypeptide qui inclut le domaine variable d'immunoglobuline est présenté sur une surface cellulaire et est accessible à une sonde.

2. Procédé selon la revendication 1
(a) dans lequel le polypeptide exprimé à partir de l'acide nucléique recombiné comprend une séquence qui permet une interaction avec la cellule de façon telle que la récupération du polypeptide résulte en la récupération de la cellule contenant l'acide nucléique codant pour le polypeptide, en particulier dans lequel la séquence qui permet l'interaction avec la cellule code pour un domaine d'ancrage qui comprend un domaine transmembranaire ou un domaine qui devient lié par le GPI à la surface d'une cellule de levure, où GPI signifie glycosylphosphatidylinositol ;
(b) dans lequel le procédé comprend en outre la fusion des cellules de levure qui incluent l'acide nucléique recombiné à d'autres cellules de levure qui incluent un acide nucléique qui code pour un domaine variable d'immunoglobuline, compatible avec le domaine variable d'immunoglobuline codé par l'acide nucléique recombiné de sorte que les cellules fusionnées incluent un acide nucléique qui code pour un polypeptide qui inclut une Ig LC et un polypeptide qui inclut une Ig HC, en particulier dans lequel la fusion comprend une conjugaison chez la levure ;
(c) dans lequel l'apport des cellules de levure comprend l'introduction du ou des acides nucléiques codant pour un ou des segment(s) d'Ig dans les cellules de levure, qui incluent l'acide nucléique hétérologue ;
(d) dans lequel l'apport des cellules de levure comprend l'introduction concomitante, dans les cellules, du ou des acides nucléiques codant pour un ou des segment(s) d'Ig et du ou des acides nucléiques homologues aux régions d'un domaine variable d'immunoglobuline de l'acide nucléique hétérologue dans les cellules de levure, en particulier dans lequel les séquences d'acide nucléique codant pour un ou des anticorps comprennent une séquence codante à chaîne unique qui code pour un polypeptide qui comprend un domaine variable LC d'immunoglobuline et un domaine variable HC d'immunoglobuline, ou en particulier dans lequel la protéine d'anticorps est un Fab ;
(e) dans lequel les acides nucléiques codant pour un ou des segment(s) d'Ig comprennent chacun une séquence codant pour un CDR d'un domaine variable d'immunoglobuline ou un complément de celle-ci ;
(f) dans lequel les acides nucléiques codant pour un ou des segment(s) d'Ig contiennent chacun une séquence codant pour un CDR unique d'un domaine variable d'immunoglobuline ou un complément de celle-ci, en particulier dans lequel le ou les acides nucléiques codant pour un ou des segment(s) d'Ig comprennent des acides nucléiques codant pour des variants différents de CDR1, CDR2 et CDR3 d'un domaine variable de chaîne lourde ou légère d'immunoglobuline ;
(g) dans lequel le ou les acides nucléiques codant pour un ou des segment(s) d'Ig sont obtenus à partir d'acides nucléiques humains ;
(h) dans lequel le ou les acides nucléiques codant pour un ou des segment(s) d'Ig comprennent une pluralité d'acides nucléiques qui codent pour différents variants du même CDR ;
(i) dans lequel le ou les acides nucléiques codant pour un ou des segment(s) d'Ig comprennent une pluralité d'acides nucléiques qui codent pour différents variants d'une pluralité de CDR différents ;
(j) dans lequel chaque acide nucléique codant pour un ou des segment(s) d'Ig est d'une longueur inférieure à 300 nucléotides ;
(k) dans lequel l'introduction comprend la mise en contact d'au moins 10³ acides nucléiques différents codant pour un ou des segment(s) d'Ig avec une ou plusieurs des cellules de levure ;
(l) dans lequel le ou les acides nucléiques codant pour un ou des segment(s) d'Ig sont monocaténaires ;
(m) dans lequel le procédé comprend en outre, avant l'introduction, l'insertion d'un marqueur contre-sélectionnable dans les éléments d'acide nucléique des cellules du sous-ensemble, et, après l'introduction, la sélection de cellules dans lesquelles le marqueur contre-sélectionnable est remplacé par un acide nucléique codant pour un ou des segment(s) d'Ig ;
(n) dans lequel le procédé comprend en outre, avant l'introduction, l'insertion d'une mutation dans la région codante, laquelle mutation empêche l'expression d'un domaine variable d'immunoglobuline fonctionnel, en particulier laquelle mutation comprend un codon de terminaison, un gène marqueur, un décalage du cadre de lecture, ou un site d'endonucléase site-spécifique ; ou
(o) dans lequel le procédé comprend en outre la sélection d'un ou plusieurs des acides nucléiques recombinés pour un critère et la répétition du procédé en apportant des cellules de levure supplémentaires, dans lequel la région codante des cellules de levure supplémentaires est préparée à partir d'un ou plusieurs acides nucléiques recombinés sélectionnés.

3. Procédé de modification de la séquence d'une protéine d'anticorps, le procédé comprenant :
l'apport d'une pluralité de cellules de levure, chaque cellule de la pluralité comprenant des séquences d'acide nucléique codant pour un ou des anticorps qui codent pour une protéine d'anticorps unique, dans lequel la protéine d'anticorps comprend un domaine variable de chaîne légère d'immunoglobuline et un domaine variable de chaîne lourde d'immunoglobuline ;
l'introduction d'un ou plusieurs acides nucléiques codant pour un ou des segment (s) d'Ig dans une ou plusieurs cellules provenant de la pluralité, où l'acide nucléique ou chacun des acides nucléiques codant pour un ou des segment(s) d'Ig comprend une séquence codant pour un segment d'un domaine variable d'immunoglobuline ou un complément de celle-ci ; et
le maintien de la cellule ou des cellules provenant de la pluralité dans des conditions qui permettent aux acides nucléiques introduits codant pour un ou des segment(s) d'Ig de se recombiner avec des séquences d'acide nucléique codant pour un ou des anticorps des cellules du sous-ensemble, produisant ainsi une ou une pluralité de cellules qui peuvent exprimer sur la surface cellulaire une protéine d'anticorps ayant un domaine variable d'immunoglobuline modifié.

4. Procédé selon la revendication 3
(a) dans lequel la pluralité des acides nucléiques codant pour un ou des segment(s) d'Ig est introduite dans des cellules de la pluralité en parallèle ;
(b) dans lequel la pluralité des acides nucléiques codant pour un ou des segment(s) d'Ig est introduite dans des cellules de la pluralité dans le même mélange réactionnel ;
(c) dans lequel les séquences d'acide nucléique codant pour un ou des anticorps comprennent une séquence codante LC qui code pour un polypeptide qui comprend un domaine variable LC d'immunoglobuline et une séquence codante HC qui code pour un polypeptide qui comprend un domaine variable HC d'immunoglobuline, en particulier dans lequel les séquences codantes LC et HC sont intégrées dans des chromosomes de levure différents, ou en particulier dans lequel la cellule de levure est une cellule diploïde, au moins au moment de la sélection, et la séquence codante LC et la séquence codante HC sont intégrées dans des loci sur des chromosomes homologues de façon telle que la séquence codante LC et la séquence codante HC se séparent en spores différents quand la cellule diploïde est sporulée, plus particulièrement dans lequel les loci sont liés aux loci MAT ; ou
(d) dans lequel les séquences d'acide nucléique codant pour un ou des anticorps sont intégrées dans un chromosome de levure.

5. Procédé de sélection d'une cellule qui présente une protéine d'anticorps sur la surface cellulaire, le procédé comprenant :
l'apport d'une pluralité de cellules de levure, la pluralité comprenant des cellules qui incluent chacune des séquences codant pour un ou des anticorps qui peuvent coder pour une protéine d'anticorps qui comprend un domaine variable de chaîne légère d'immunoglobuline et un domaine variable de chaîne lourde d'immunoglobuline ou qui peuvent se recombiner avec des acides nucléiques codant pour un ou des segment(s) d'Ig afin de former des séquences codantes fonctionnelles qui codent pour la protéine d'anticorps ;
la réalisation d'un ou plusieurs cycles de :
(i) introduction d'acides nucléiques qui comprennent chacun un segment codant pour un CDR d'un domaine variable d'immunoglobuline ou un complément de celui-ci dans des cellules qui incluent au moins une partie des séquences codant pour un ou des anticorps provenant des cellules du sous-ensemble ;
(ii) maintien des cellules qui sont mises en contact avec l'acide nucléique dans (i) dans des conditions qui permettent aux acides nucléiques introduits de se recombiner avec les séquences codant pour un ou des anticorps des cellules, produisant ainsi des cellules modifiées qui incluent des séquences codant pour un ou des anticorps modifiées qui ont un ou plusieurs domaines variables d'immunoglobuline modifiés ;
(iii) expression des séquences codant pour un ou des anticorps modifiées dans les cellules modifiées ;
(iv) mise en contact des cellules modifiées avec la cible ; et
(v) sélection d'un sous-ensemble supplémentaire de cellules parmi les cellules modifiées, le sous-ensemble supplémentaire comprenant une ou plusieurs cellules de levure qui interagissent avec la cible.

6. Procédé selon la revendication 5
(a) dans lequel au moins deux cycles sont effectués, en particulier dans lequel l'étape (iii) de mise en contact comprend la mise en contact des cellules avec la cible dans des conditions différentes pendant des cycles différents, ou en particulier dans lequel l'étape (v) de sélection comprend l'exigence d'une liaison améliorée à la cible par rapport à une étape de sélection précédente ;
(b) dans lequel, avant l'étape (i) d'introduction de l'acide nucléique, une séquence marqueur est insérée dans les séquences codant pour un ou des anticorps;
(c) dans lequel le procédé comprend en outre la récupération d'une séquence codant pour un ou des anticorps à partir d'une cellule du sous-ensemble supplémentaire provenant d'un ou plusieurs des cycles ;
(d) dans lequel le procédé comprend en outre le séquençage d'au moins une région codant pour un ou des CDR d'une séquence codant pour un ou des anticorps dans une cellule du sous-ensemble supplémentaire provenant d'un ou plusieurs des cycles ;
(e) dans lequel les acides nucléiques sont introduits dans des cellules du sous-ensemble ;
(f) dans lequel le procédé comprend en outre, dans un ou plusieurs des cycles, la sporulation de cellules du sous-ensemble avant (i), et la conjugaison entre des cellules dans lesquelles des acides nucléiques ont été introduits après (ii) ;
(g) dans lequel l'apport d'une pluralité de cellules de levure comprend l'amplification d'une cellule originale de façon telle que les cellules de levure de la pluralité sont essentiellement identiques ;
(h) dans lequel l'apport d'une pluralité de cellules de levure comprend l'amplification d'une pluralité de cellules originales, les cellules originales comprenant des séquences codant pour un ou des anticorps pour des anticorps différents qui interagissent avec la même cible ; ou
(i) dans lequel l'apport d'une pluralité de cellules de levure comprend la sélection d'un ou plusieurs acides nucléiques à partir d'une bibliothèque de présentation de phage et le reformatage d'un ou plusieurs acides nucléiques à partir d'un système de présentation de phage dans un système d'expression de levure.

7. Procédé de variation de sous-unités d'une protéine d'anticorps présentée sur une surface cellulaire de levure, le procédé comprenant :
l'apport d'une première cellule de levure haploïde qui inclut un premier élément d'acide nucléique codant pour une première sous-unité d'une protéine d'anticorps, la première sous-unité comprenant un domaine variable d'immunoglobuline ;
l'introduction d'un acide nucléique ou d'une pluralité d'acides nucléiques qui comprennent chacun un segment codant pour un CDR du domaine variable d'immunoglobuline ou un complément de celui-ci dans la première cellule haploïde ou dans des clones de celle-ci de façon telle que l'acide nucléique introduit se recombine avec le premier élément d'acide nucléique dans la première cellule haploïde ou les clones de celle-ci, produisant ainsi une ou plusieurs premières cellules haploïdes modifiées ; et
la conjugaison entre la ou les premières cellules haploïdes modifiées à une ou plusieurs secondes cellules haploïdes afin de fournir une ou plusieurs cellules diploïdes, dans lequel une seconde cellule de levure haploïde inclut un second élément d'acide nucléique codant pour une seconde sous-unité de la protéine d'anticorps, la seconde sous-unité comprenant une domaine variable d'immunoglobuline complémentaire au domaine variable d'immunoglobuline de la première sous-unité, et chaque cellule diploïde peut exprimer, sur sa surface cellulaire, une protéine d'anticorps comprenant les première et seconde sous-unités,
en particulier dans lequel le procédé comprend en outre, avant l'introduction, l'insertion d'une séquence qui n'est pas une immunoglobuline dans le premier élément d'acide nucléique, interrompant ainsi le codage de la première sous-unité, plus particulièrement dans lequel la séquence codante qui ne code pas pour une immunoglobuline comprend un marqueur contre-sélectionnable.

8. Procédé de variation de sous-unités d'une protéine d'anticorps présentée sur une surface cellulaire de levure, le procédé comprenant :
l'apport d'une première cellule de levure haploïde qui inclut un premier élément d'acide nucléique qui peut coder pour un polypeptide comprenant une première sous-unité d'une protéine d'anticorps, la première sous-unité comprenant un domaine variable d'immunoglobuline, dans lequel la région de l'élément d'acide nucléique qui code pour le domaine variable d'immunoglobuline est interrompue ;
l'introduction d'un acide nucléique ou une pluralité d'acides nucléiques qui comprennent chacun un segment codant pour un CDR du domaine variable d'immunoglobuline ou un complément de celui-ci dans la première cellule haploïde ou dans des clones de celle-ci de façon telle que l'acide nucléique introduit se recombine avec le premier élément d'acide nucléique dans la première cellule haploïde ou les clones de celle-ci, produisant ainsi une ou plusieurs première cellules haploïdes, modifiées ; et
la conjugaison de la ou des premières cellules haploïdes, modifiées, à une ou plusieurs secondes cellules haploïdes afin de fournir une ou plusieurs cellules diploïdes, dans lequel une seconde cellule de levure haploïde inclut un second élément d'acide nucléique codant pour un polypeptide comprenant une seconde sous-unité de la protéine d'anticorps, la seconde sous-unité comprenant un domaine variable d'immunoglobuline complémentaire au domaine variable d'immunoglobuline de la première sous-unité, et chaque cellule diploïde peut exprimer, sur sa surface cellulaire, une protéine d'anticorps comprenant les première et seconde sous-unités,
en particulier dans lequel le procédé comprend en outre, avant la conjugaison, l'amplification d'une ou plusieurs des premières cellules haploïdes, modifiées, par un ou plusieurs cycles de division cellulaire.

9. Procédé de variation de sous-unités d'une protéine d'anticorps présentée sur une surface cellulaire de levure, le procédé comprenant :
l'apport d'une première cellule de levure haploïde qui inclut un premier élément d'acide nucléique codant pour un polypeptide comprenant une première sous-unité d'une protéine d'anticorps, la première sous-unité comprenant un domaine variable de chaîne légère d'immunoglobuline et une seconde cellule de levure haploïde qui inclut un second élément d'acide nucléique codant pour un polypeptide comprenant une seconde sous-unité de la protéine d'anticorps, la seconde sous-unité comprenant un domaine variable de chaîne lourde d'immunoglobuline ;
l'introduction d'un acide nucléique ou d'une pluralité d'acides nucléiques qui comprennent chacun un segment codant pour un CDR d'un domaine variable de chaîne légère d'immunoglobuline ou un complément de celui-ci dans la première cellule haploïde ou dans des clones de celle-ci de façon telle que l'acide nucléique introduit se recombine avec le premier élément d'acide nucléique dans la première cellule haploïde ou les clones de celle-ci, produisant ainsi une ou plusieurs premières cellules haploïdes modifiées ;
l'introduction d'un acide nucléique ou d'une pluralité d'acides nucléiques qui comprennent chacun un segment codant pour un CDR d'un domaine variable de chaîne lourde d'immunoglobuline ou un complément de celui-ci dans la seconde cellule haploïde ou des clones de celle-ci de façon telle que l'acide nucléique introduit se recombine avec le second élément d'acide nucléique dans la seconde cellule haploïde ou les clones de celle-ci, produisant ainsi une ou plusieurs secondes cellules haploïdes modifiées ; et
la conjugaison de la ou des premières cellules haploïdes modifiées à la ou les secondes cellules haploïdes modifiées afin de fournir une ou plusieurs cellules diploïdes qui peuvent chacune exprimer, sur une surface cellulaire, une protéine d'anticorps avec un domaine variable de chaîne légère d'immunoglobuline modifié et un domaine variable de chaîne lourde d'immunoglobuline modifié.

10. Procédé selon la revendication 9, dans lequel les étapes d'apport des première et seconde cellules haploïdes comprennent :
l'apport d'une cellule de levure diploïde qui inclut (i) un premier élément d'acide nucléique codant pour une première sous-unité d'une protéine d'anticorps, la première sous-unité comprenant un domaine variable de chaîne légère d'immunoglobuline et (ii) un second élément d'acide nucléique, codant pour une seconde sous-unité de la protéine hétéroligomère, la seconde sous-unité comprenant un domaine variable de chaîne lourde d'immunoglobuline ; et
la sporulation de la cellule de levure diploïde afin de fournir la première cellule haploïde qui contient le premier élément d'acide nucléique, mais pas le second élément d'acide nucléique, et la seconde cellule haploïde qui contient le second élément d'acide nucléique, mais pas le premier élément d'acide nucléique.

11. Procédé selon la revendication 9, dans lequel le procédé comprend en outre
(a) avant la conjugaison, l'amplification de la ou des premières ou secondes cellules haploïdes modifiées par un ou plusieurs cycles de division cellulaire,
(b) la sporulation d'une ou plusieurs des cellules diploïdes formées par la conjugaison, ou
(c) la sélection d'un sous-ensemble de la ou des cellules diploïdes en mettant en contact la ou les cellules diploïdes, ou des clones de celles-ci, avec une cible, en particulier
(i) dans lequel le sous-ensemble comprend une ou plusieurs des cellules qui interagissent avec la cible,
(ii) dans lequel le sous-ensemble comprend une ou plusieurs des cellules qui n'interagissent pas avec la cible, ou
(iii) dans lequel le procédé comprend en outre la sporulation de cellules du sous-ensemble.

12. Procédé de sélection d'une cellule qui présente une protéine de liaison, le procédé comprenant :
l'apport d'une pluralité d'acides nucléiques divers qui incluent une séquence codant pour une protéine ou un complément de celle-ci ;
l'apport d'une pluralité de cellules de levure, chaque cellule de la pluralité comprenant un acide nucléique hétérologue qui comprend des séquences acceptrices qui peuvent se recombiner avec des séquences homologues présentes dans un ou plusieurs des acides nucléiques divers, l'acide nucléique hétérologue comprenant une séquence codant pour un ou des domaine(s) d'immunoglobuline non fonctionnel(s) qui fournit (fournissent) les séquences acceptrices pour une recombinaison,
dans lequel la recombinaison des séquences acceptrices et d'un acide nucléique divers produit un acide nucléique recombiné qui code pour un polypeptide qui inclut un segment codé par l'acide nucléique divers ou le complément de celui-ci ;
dans lequel l'acide nucléique recombiné inclut une séquence codant pour un domaine variable de chaîne lourde ou un domaine variable de chaîne légère d'immunoglobuline ;
l'introduction d'acides nucléiques provenant de la pluralité d'acides nucléiques divers dans des cellules de la pluralité de cellules ;
la recombinaison d'un ou plusieurs des acides nucléiques introduits avec l'acide nucléique hétérologue dans chaque cellule, produisant ainsi des acides nucléiques recombinés qui codent chacun pour un polypeptide qui comprend un segment qui est codé par un ou plusieurs des acides nucléiques introduits ou un complément de ceux-ci ;
l'expression des acides nucléiques recombinés dans les cellules de façon telle que les polypeptides codés par les acides nucléiques recombinés sont associés à une surface des cellules ;
la mise en contact des cellules qui expriment les acides nucléiques recombinés avec une cible ; et
la sélection d'une ou plusieurs cellules en fonction de leur interaction avec la cible.

13. Procédé selon la revendication 12,
(a) dans lequel la séquence codant pour un ou des domaine(s) d'immunoglobuline non fonctionnel(s) comprend un codon de terminaison avant l'extrémité 3' de la séquence codant pour un ou des domaine(s) d'immunoglobuline, plus particulièrement dans lequel le codon de terminaison est dans une région codant pour un CDR,
(b) dans lequel la séquence codant pour un ou des domaine(s) d'immunoglobuline non fonctionnel(s) comprend un gène marqueur dans une région codant pour un CDR et les séquences codant pour des régions FR du domaine d'immunoglobuline sont intactes, plus particulièrement dans lequel le gène marqueur comprend un marqueur contre-sélectionnable, ou
(c) dans lequel les acides nucléiques recombinés codent pour une chaîne légère d'anticorps, et l'expression comprend en outre l'expression d'un acide nucléique codant pour une chaîne lourde d'anticorps qui comprend un domaine VH et un domaine CH1 et un domaine d'ancrage de façon telle que les chaînes lourde et légère d'anticorps s'associent et que le domaine d'ancrage fixe la chaîne lourde d'anticorps sur la surface cellulaire.

14. Procédé selon la revendication 12 ou 13
(a) dans lequel l'acide nucléique hétérologue comprend un segment codant pour un domaine d'ancrage, en particulier dans lequel le domaine d'ancrage comprend un domaine transmembranaire, ou en particulier dans lequel le domaine d'ancrage aide une liaison par le GPI ;
(b) dans lequel la cible est une protéine ;
(c) dans lequel la cible est une cellule de mammifère ; ou
(d) dans lequel la recombinaison est accrue en clivant l'acide nucléique hétérologue dans chaque cellule, en particulier dans lequel le clivage crée une rupture bicaténaire dans l'acide nucléique hétérologue, ou en particulier dans lequel, avant la recombinaison, un site reconnu par une endonucléase site-spécifique qui peut être exprimée dans des cellules de levure est situé entre les séquences acceptrices de l'acide nucléique hétérologue, et la recombinaison est accrue en fournissant l'endonucléase site-spécifique dans chacune des cellules, plus particulièrement dans lequel l'endonucléase site-spécifique est l'endonucléase HO et la fourniture de l'endonucléase site-spécifique comprend la transcription d'un gène codant pour l'endonucléase HO.
